# EUROPEAN PATENT SPECIFICATION

(11) **EP 0 558 673 B1**
(45) Date of publication and mention of the grant of the patent: **17.04.1996**
(21) Application number: 92902324.0
(22) Date of filing: 18.11.1991
(51) Int. Cl.: C07D 215/48, C07D 295/13, C07C 311/46, C07C 311/47, C07C 317/50, C07C 323/60, C07C 275/14

(54) **RETROVIRAL PROTEASE INHIBITORS**
RETROVIRALE PROTEASEINHIBITOREN
INHIBITEURS DE PROTEASES RETROVIRALES

(30) Priority: 19.11.1990 US 615210; 14.11.1991 US 789645
(43) Date of publication of application: 08.09.1993
(73) Proprietor: MONSANTO COMPANY, St. Louis Missouri 63167 (US)
(72) Inventor: REED, Kathryn, Lea, Richmond Heights, MO 63117 (US); TALLEY, John, Jeffrey, Chesterfield, MO 63017 (US)
(74) Representative: Ernst, Hubert
(86) International application number: US9108593
(87) International publication number: WO9208699

(56) References cited:
- EP-A- 0 264 795
- US-A- 4 599 198

## Description

This application is a continuation-in-part of U. S. Patent Application Serial No. 07/615,210, filed November 19, 1990.

### BACKGROUND OF THE INVENTION

### 1. Field of the Invention

The present invention relates to retroviral protease inhibitors and, more particularly, relates to novel compounds and a composition and method for inhibiting retroviral proteases. This invention, in particular, relates to urea-containing hydroxyethylamine protease inhibitor compounds, a composition and method for inhibiting retroviral proteases such as human immunodeficiency virus (HIV) protease and for treating a retroviral infection, e.g., an HIV infection. The subject invention also relates to processes for making such compounds as well as to intermediates useful in such processes.

### 2. Related Art

During the replication cycle of retroviruses, gag and gag-pol gene products are translated as proteins. These proteins are subsequently processed by a virally encoded protease (or proteinase) to yield viral enzymes and structural proteins of the virus core. Most commonly, the gag precursor proteins are processed into the core proteins and the pol precursor proteins are processed into the viral enzymes, e.g., reverse transcriptase and retroviral protease. It has been shown that correct processing of the precursor proteins by the retroviral protease is necessary for assembly of infectious virons. For example, it has been shown that frameshift mutations in the protease region of the pol gene of HIV prevents processing of the gag precursor protein. Thus, attempts have been made to inhibit viral replication by inhibiting the action of retroviral proteases.

Retroviral protease inhibition typically involves a transition-state mimetic whereby the retroviral protease is exposed to a mimetic compound which binds (typically in a reversible manner) to the enzyme in competition with the gag and gag-pol proteins to thereby inhibit replication of structural proteins and, more importantly, the retroviral protease itself. In this manner, retroviral proteases can be effectively inhibited.

Several classes of mimetic compounds have been proposed, particularly for inhibition of proteases, such as for inhibition of HIV protease. Such mimetics include hydroxyethylamine isosteres and reduced amide isosteres. See, for example, EP 0 346 847; EP 0 342,541; Roberts et al, "Rational Design of Peptide-Based Proteinase Inhibitors, "Science, 248, 358 (1990); and Erickson et al, "Design Activity, and 2.8A Crystal Structure of a C₂ Symmetric Inhibitor Complexed to HIV-1 Protease," Science, 249, 527 (1990).

Several classes of mimetic compounds are known to be useful as inhibitors of the proteolytic enzyme renin. See, for example, U.S. No. 4,599,198; U.K. 2,184,730; G.B. 2,209,752; EP 0 264 795; G.B. 2,200,115 and U.S. SIR H725. Of these, G.B. 2,200,115, GB 2,209,752, EP 0 264,795, U.S. SIR H725 and U.S. 4,599,198 disclose urea-containing hydroxyethylamine renin inhibitors. However, it is known that, although renin and HIV proteases are both classified as aspartyl proteases, compounds which are effective renin inhibitors generally cannot be predicted to be effective HIV protease inhibitors.

### BRIEF DESCRIPTION OF THE INVENTION

The present invention is directed to virus inhibiting compounds and compositions. More particularly, the present invention is directed to retroviral protease inhibiting compounds and compositions, to a method of inhibiting retroviral proteases, to processes for preparing the compounds and to intermediates useful in such processes. The subject compounds are characterized as urea-containing hydroxyethylamine inhibitor compounds.

### DETAILED DESCRIPTION OF THE INVENTION

In accordance with the present invention, there is provided a retroviral protease inhibiting compound of the formula: 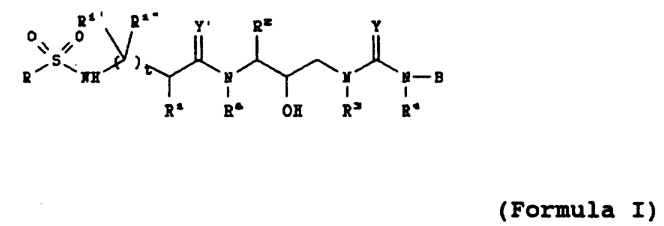 or a pharmaceutically acceptable salt, prodrug or ester thereof wherein:
- R: represents alkyl, alkenyl, hydroxyalkyl, cycloalkyl, cycoalkylalkyl, heterocycloalkyl, heterocycloalkylalkyl, aryl, aralkyl and heteroaryl radicals;
- t: represents either 0 or 1;
- R¹: represents hydrogen, -CH₂SO₂NH₂, alkyl, alkenyl, alkynyl and cycloalkyl radicals and amino acid side chains selected from asparagine, S-methyl cysteine and the corresponding sulfoxide and sulfone derivatives thereof, glycine, leucine, isoleucine, allo-isoleucine, tert-leucine, phenylalanine, ornithine,alanine, histidine, norleucine, glutamine, valine, threonine, serine, aspartic acid, beta-cyano alanine and allo-threonine side chains;
- R^{1'} and R^{1"}: independently represent hydrogen and radicals as defined for R¹;
- R: represents alkyl, aryl, cycloalkyl, cycloalkylalkyl and aralkyl radicals, which radicals are optionally substituted with a group selected from -OR⁹, -SR⁹, and halogen radicals, wherein R⁹ represents hydrogen and alkyl radicals;
- R³: represents alkyl, alkenyl, hydroxyalkyl, cycloalkyl, cycloalkylalkyl, heterocycloalkyl, heteroaryl, heterocycloalkylalkyl, aryl, aralkyl, and heteroaralkyl radicals;
- Y and Y': independently represent O,S and NR¹⁵ wherein R¹⁵ represents radicals as defined for R³;
- B: represents R5 and radicals represented by the formula: 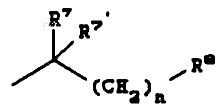
wherein
- n: represents an integer of from 0 to 6, R⁷ and R^{7'} independently represent radicals as defined for R³ and amino acid side chains selected from the group consisting of valine, isoleucine, glycine, alanine, allo-isoleucine, asparagine, leucine, glutamine, and t-butylglycine or R⁷ and R^{7'} together with the carbon atom to which they are attached form a cycloalkyl radical; and R⁸ represents cyano, hydroxyl, alkyl, alkoxy, cycloalkyl, aryl, aralkyl, heterocycloalkyl and heteroaryl radicals and radicals represented by the formulas C(O)R¹⁶, CO₂R¹⁶, SO₂R¹⁶, SR¹⁶, CON¹⁶R¹⁷, OR¹⁶, CF₃ and NR¹⁶R¹⁷ wherein R¹⁶ and R¹⁷ independently represent hydrogen and radicals as defined for R³ or R¹⁶ and R¹⁷ together with a nitrogen to which they are attached represent heterocycloalkyl and heteroaryl radicals.
- R⁴ and R⁵: independently represent hydrogen and radicals as defined by R³, or together with the nitrogen atom to which they are bonded represent heterocycloalkyl and heteroaryl radicals; and
- R⁶: represents hydrogen and radicals as defined for R³.

A preferred class of retroviral inhibitor compounds of the present invention are those represented by the formula: 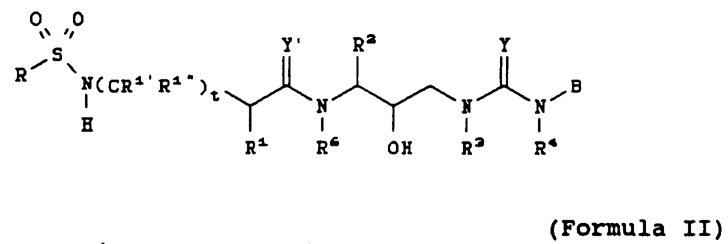 or a pharmaceutically acceptable salt, prodrug or ester thereof, preferably wherein the stereochemistry about the hydroxy group is designated as (R);
R represents alkyl, alkenyl, hydroxyalkyl, cycloalkyl, cycloalkylalkyl, heterocycloalkyl, heterocycloalkylalkyl, aryl, aralkyl and heteroaryl radicals;
R¹ represents hydrogen, -CH₂SO₂NH₂, alkyl, alkenyl, alkynyl, and cycloalkyl radicals, and amino acid side chains selected from asparagine, S-methyl cysteine and the sulfoxide (SO) and sulfone (SO₂) derivatives thereof isoleucine, allo-isoleucine, alanine, leucine, tertleucine, phenylalanine, arnithine, histidine, norleucine glutamine, threonine, glycine, allo-threonine, serine, aspartic acid, beta-cyano alanine and valine side chains;
   - R^{1'} and R^{1"}: independently represent hydrogen and radicals as defined for R¹;
   - R: represents alkyl, aryl, cycloalkyl, cycloalkylalkyl, and aralkyl radicals, which radicals are optionally substituted with a group selected from alkyl radicals, OR⁹ and SR⁹ wherein R⁹ represents hydrogen and alkyl radicals, and halogen radicals;
   - R³: represents alkyl, cycloalkyl, cycloalkylalkyl, heterocycloalkyl, heterocycloalkylalkyl, aryl, heteroaryl, aralkyl and heteroaralkyl radicals; and
   - R⁴: represents hydrogen and radicals as defined for R³;
   - B: represents radicals represented by the formula: 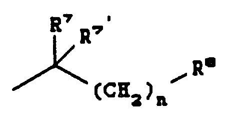
wherein n represents an integer of from 0 to 6, R⁷ and
- R^{7'}: independently represent radicals as defined for R³ and amino acid side chains selected from the group consisting of valine, isoleucine, glycine, alanine, allo-isoleucine, asparagine, leucine, glutamine, and t-butylglycine or R⁷ and R^{7'} together with the carbon atom to which they are attached form a cycloalkyl radical; and
- R⁸: represents cyano, hydroxyl, alkyl, cycloalkyl, aryl, aralkyl, heterocycloalkyl and heteroaryl radicals and radicals represented by the formulas C(O)R¹⁶, CO₂R¹⁶, SO₂R¹⁶, SR¹⁶, CON¹⁶R¹⁷ and NR¹⁶R¹⁷, CF₃ wherein R¹⁶ and R¹⁷ independently represent hydrogen and radicals as defined for R³ or R¹⁶ and R¹⁷ together with a nitrogen to which they are attached represent heterocycloalkyl and heteroaryl radicals.
- t: represents 0 or 1;
- Y and Y': independently represent 0, S, and NR¹⁵ wherein
- R¹⁵: represents radicals as defined for R³. Preferably, Y represents 0.

Preferably, R³ represents radicals as defined above which contain no α-branching, e.g., as in an isopropyl radical or a t-butyl radical. The preferred radicals are those which contain a -CH₂- moiety between the nitrogen of the urea and the remaining portion of the radical. Such preferred groups include, but are not limited to, benzyl, isoamyl, cyclohexylmethyl, 4-pyridylmethyl and the like.

Another preferred class of compounds are those represented by the formula: 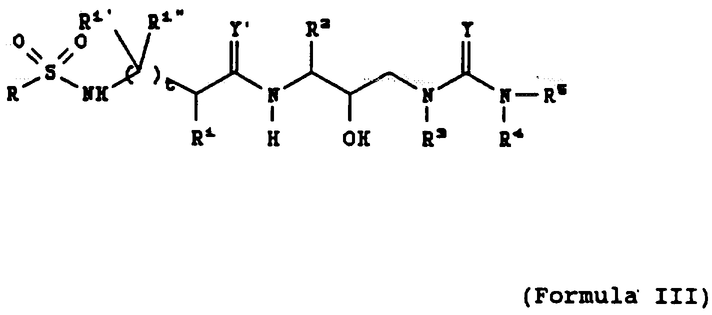 or a pharmaceutically acceptable salt, prodrug or ester thereof wherein Y, Y', R, R¹, R^{1'}, R^{1"},R, R³, R⁴, and R⁵ are as defined above.

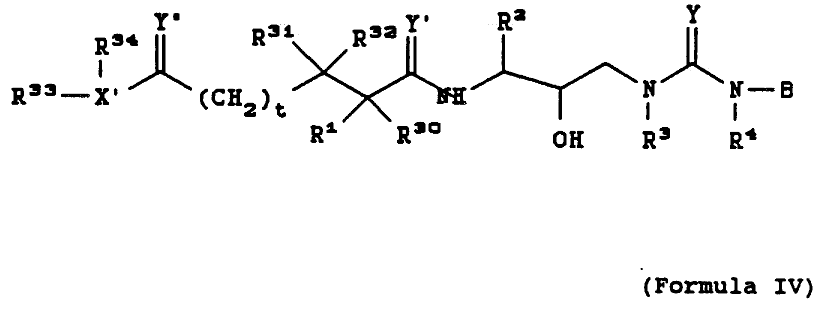

As utilized herein, the term "alkyl", alone or in combination, means a straight-chain or branched-chain alkyl radical containing from 1 to about 10, preferably from 1 to about 8, carbon atoms. Examples of such radicals include methyl, ethyl, n-propyl, isopropyl, n-butyl, isobutyl, sec-butyl, tert-butyl, pentyl, isoamyl, hexyl, octyl and the like. The term "alkoxy", alone or in combination, means an alkyl ether radical wherein the term alkyl is as defined above. Examples of suitable alkyl ether radicals include methoxy, ethoxy, n-propoxy, isopropoxy, n-butoxy, iso-butoxy, sec-butoxy, tert-butoxy and the like. The term "cycloalkyl" means an alkyl radical which contains from 3 to 8 carbon atoms and is cyclic. The term "cycloalkylalkyl" means an alkyl radical as defined above which is substituted by a cycloalkyl radical containing from 3 to 8, preferably from 3 to 6, carbon atoms. Examples of such cycloalkyl radicals include cyclopropyl, cyclobutyl, cyclopentyl, cyclohexyl and the like. The term "aryl", alone or in combination, means a phenyl or naphthyl radical which optionally carries one or more substituents selected from alkyl, alkoxy, halogen, hydroxy, amino and the like, such as phenyl, p-tolyl, 4-methoxyphenyl, 4-(tert-butoxy)phenyl, 4-fluorophenyl, 4-chlorophenyl, 4-hydroxyphenyl, 1-naphthyl, 2-naphthyl, and the like. The term "aralkyl", alone or in combination, means an alkyl radical as defined above in which one hydrogen atom is replaced by an aryl radical as defined above, such as benzyl, 2-phenylethyl and the like. The term "aralkoxy carbonyl", alone or in combination, means a radical of the formula -C(O)-O-aralkyl in which the term "aralkyl" has the significance given above. An example of an aralkoxycarbonyl radical is benzyloxycarbonyl. The term "aryloxy" means a radical of the formula aryl-o- in which the term aryl has the significance given above. The term "alkanoyl", alone or in combination, means an acyl radical derived from an alkanecarboxylic acid, examples of which include acetyl, propionyl, butyryl, valeryl, 4-methylvaleryl, and the like. The term "cycloalkylcarbonyl" means an acyl group derived from a monocyclic or bridged cycloalkanecarboxylic acid such as cyclopropanecarbonyl, cyclohexanecarbonyl, adamantanecarbonyl, and the like, or from a benz-fused monocyclic cycloalkanecarboxylic acid which is optionally substituted by, for example, alkanoylamino, such as 1,2,3,4-tetrahydro-2-naphthoyl,2-acetamido-1,2,3,4-tetrahydro-2-naphthoyl. The term "aralkanoyl" means an acyl radical derived from an aryl-substituted alkanecarboxylic acid such as phenylacetyl, 3-phenylpropionyl (hydrocinnamoyl), 4-phenylbutyryl, (2-naphthyl)acetyl, 4-chlorohydrocinnamoyl, 4-aminohydroinnamoyl,4-methoxyhydrocinnamoyl, and the like. The term "aroyl" means an acyl radical derived from an aromatic carboxylic acid. Examples of such radicals include aromatic carboxylic acids, an optionally substituted benzoic or naphthoic acid such as benzoyl, 4-chlorobenzoyl, 4-carboxybenzoyl, 4-(benzyloxycarbonyl)benzoyl, 1-naphthoyl, 2-naphthoyl, 6-carboxy-2 naphthoyl, 6-(benzyloxycarbonyl)-2-naphthoyl, 3-benzyloxy-2-naphthoyl, 3-hydroxy-2-naphthoyl, 3-(benzyloxyformamido)-2-naphthoyl, and the like. The heterocyclyl or heterocycloalkyl portion of a heterocyclylcarbonyl, heterocyclyloxycarbonyl, heterocyclylalkoxycarbonyl, or heterocyclyalkyl group or the like is a saturated or partially unsaturated monocyclic, bicyclic or tricyclic heterocycle which contains one or more hetero atoms selected from nitrogen, oxygen and sulphur, which is optionally substituted on one or more carbon atoms by halogen, alkyl, alkoxy, oxo, and the like, and/or on a secondary nitrogen atom (i.e., -NH-) by alkyl, aralkoxycarbonyl, alkanoyl, phenyl or phenylalkyl or on a tertiary nitrogen atom (i.e. = N-) by oxido and which is attached via a carbon atom. The heteroaryl portion of a heteroaroyl, heteroaryloxycarbonyl, or a heteroaralkoxy carbonyl group or the like is an aromatic monocyclic, bicyclic, or tricyclic heterocycle which contains the hetero atoms and is optionally substituted as defined above with respect to the definition of heterocyclyl. Examples of such heterocyclyl and heteroaryl groups are pyrrolidinyl, piperidinyl, piperazinyl, morpholinyl, thiamorpholinyl, pyrrolyl, imidazolyl (e.g., imidazol 4-yl, 1-benzyloxycarbonylimidazol-4-yl, etc.), pyrazolyl, pyridyl, pyrazinyl, pyrimidinyl, furyl, thienyl, triazolyl, oxazolyl, thiazolyl, indolyl (e.g., 2-indolyl, etc.), quinolyl (e.g., 2-quinolyl, 3-quinolyl, l-oxido-2-quinolyl, etc.), isoquinolyl (e.g., 1-isoquinolyl, 3-isoquinolyl, etc.), tetrahydroquinolyl (e.g., 1,2,3,4-tetrahydro-2-quinolyl, etc.), 1,2,3,4-tetrahydroisoquinolyl (e.g., 1,2,3,4-tetrahydro-1-oxoisoquinolyl, etc.), quinoxalinyl, β-carbolinyl, 2-benzofurancarbonyl, benzimidazolyl, and the like. The term "cycloalkylalkoxycarbonyl" means an acyl group derived from a cycloalkylalkoxycarboxylic acid of the formula cycloalkylalkyl-O-COOH wherein cycloalkylalkyl has the significance given above. The term "aryloxyalkanoyl" means an acyl radical of the formula aryl-O-alkanoyl wherein aryl and alkanoyl have the significance given above. The term "heterocyclyloxycarbonyl" means an acyl group derived from heterocyclyl-O-COOH wherein heterocyclyl is as defined above. The term "heterocyclylalkanoyl" is an acyl radical derived from a heterocyclyl-substituted alkane carboxylic acid wherein heterocyclyl has the significance given above. The term "heterocyclylalkoxycarbonyl" means an acyl radical derived from a heterocyclyl-substituted alkane-O-COOH wherein heterocyclyl has the significance given above. The term "heteroaryloxycarbonyl" means an acyl radical derived from a carboxylic acid represented by heteroaryl-O-COOH wherein heteroaryl has the significance given above. The term "aminoalkanoyl" means an acyl group derived from an amino-substituted alkanecarboxylic acid wherein the amino group can be a primary, secondary or tertiary amino group containing substituents selected from hydrogen, and alkyl, aryl, aralkyl, cycloalkyl, cycloalkylalkyl radicals and the like. The term "halogen" means fluorine, chlorine, bromine or iodine. The term "leaving group" generally refers to groups readily displaceable by a nucleophile, such as an amine or an alcohol nucleophile. Such leaving groups are well known and include carboxylates, N-hydroxysuccinimide, N-hydroxybenzotriazole, halides, triflates, tosylates -OR and -SR and the like. Preferred leaving groups are indicated herein where appropriate.

Procedures for preparing the compounds of Formula I are set forth below. It should be noted that the general procedure is shown as it relates to preparation of compounds having the specified stereochemistry, for example, wherein the stereochemistry about the hydroxyl group is designated as (R). However, such procedures are generally applicable, as illustrated in Example 45, to those compounds of opposite configuration, e.g., where the stereochemistry about the hydroxyl group is (S).

### Preparation of Compounds of Formula I

The compounds of the present invention represented by Formula I above can be prepared utilizing the following general procedure. An N-protected chloroketone derivative of an amino acid having the formula: 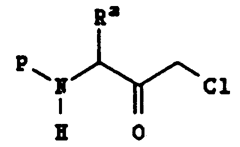 wherein P represents an amino protecting group, and R is as defined above, is reduced to the corresponding alcohol utilizing an appropriate reducing agent. Suitable amino protecting groups are well known in the art and include carbobenzoxy, butyryl, t-butoxycarbonyl, acetyl, benzoyl and the like. A preferred amino protecting group is carbobenzoxy. A preferred N-protected chloroketone is N-benzyloxycarbonyl-L-phenylalanine chloromethyl ketone. A preferred reducing agent is sodium borohydride. The reduction reaction is conducted at a temperature of from -10°C to about 25°C, preferably at about 0°C, in a suitable solvent system such as, for example, tetrahydrofuran, and the like. The N-protected chloroketones are commercially available from Bachem, Inc., Torrance, California. Alternatively, the chloroketones can be prepared by the procedure set forth in S. J. Fittkau, J. Prakt. Chem., 315, 1037 (1973), and subsequently N-protected utilizing procedures which are well known in the art.

The resulting alcohol is then reacted, preferably at room temperature, with a suitable base in a suitable solvent system to produce an N-protected amino epoxide of the formula: 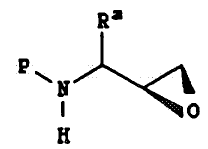 wherein P and R are as defined above. Suitable solvent systems for preparing the amino epoxide include ethanol, methanol, isopropanol, tetrahydrofuran, dioxane, and the like including mixtures thereof. Suitable bases for producing the epoxide from the reduced chloroketone include potassium hydroxide, sodium hydroxide, potassium t-butoxide, DBU and the like. A preferred base is potassium hydroxide.

The amino epoxide is then reacted, in a suitable solvent system, with an excess of a desired amine of the formula:

R³NH₂

wherein R³ is hydrogen or is as defined above. The reaction can be conducted over a wide range of temperatures, e.g., from about 10°C to about 100°C, but is preferably, but not necessarily, conducted at a temperature at which the solvent begins to reflux. Suitable solvent systems include those wherein the solvent is an alcohol, such as methanol, ethanol, isopropanol, and the like, ethers such as tetrahydrofuran, dioxane and the like, and toluene, N,N-dimethylformamide, dimethyl sulfoxide, and mixtures thereof. A preferred solvent is isopropanol. Exemplary amines corresponding to the formula R³NH₂ include benzyl amine, isobutylamine, n-butyl amine, isopentyl amine, isoamylamine, cyclohexanemethyl amine, naphthylene methyl amine and the like. The resulting product is a 3-(N-protected amino)-3-(R)-1-(NHR³)-propan-2-ol derivative (hereinafter referred to as an amino alcohol) is a novel intermediate and can be represented by the formula: 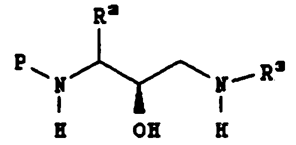 wherein P, R and R³ are as described above.

Where B represents R⁵, the salt of the resulting amino alcohol can be reacted with an isocyanate of the formula R⁴NCO where R⁵ is hydrogen, or a compound of the formula 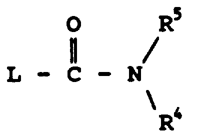 where R⁵ is other than hydrogen. In this formula R⁴ and R⁵ are as described above and L represents a leaving group such as a halide, e.g., chloride, an imidazole radical, the radical p-NO₂-(C₆H₄)-O, and the like. A preferred group of this formula is a carbamoyl chloride. The corresponding sulfur analogs can also be utilized where Y is S. These reactions are conducted in suitable solvent systems such as methylene chloride and tetrahydrofuran.

A salt of the resulting amino alcohol described above is then reacted, in a suitable solvent system, with carbonyldiimidazole and an amine salt to produce a urea derivative of the amino alcohol. This reaction can be represented as follows: 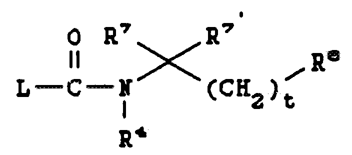 wherein R⁴ is as described above and L represents a leaving group such as a halide, e.g., chloride, imidazole radical, the radical p-NO₂-(C₆H₄)-O-, and the like is prepared by reacting a carbonyldiimidazole with an amine salt, e.g., the hydrochloride salt of a compound represented by the formula: 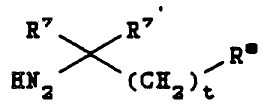 in a suitable solvent such as, for example, chloroform. The resulting product is then reacted with the salt, such as, for example, the hydrochloride salt, of the amino alcohol described above. The corresponding sulfur analogs can be utilized where Y of Formula II is S.

Alternatively, one can react the amino alcohol with an isocyanate of the formula: 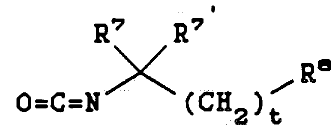 either in the presence or absence of a suitable base, such as triethylamine, diisopropylethylamine, and the like in a suitable solvent such as toluene, methylene chloride, chloroform or tetrahydrofuran. The isocyanate can be readily prepared and isolated, if desired, by standard methods such as the reaction of an amine of the formula: 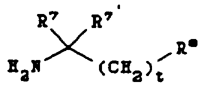 with phosgene or a phosgene equivalent, such as triphosgene, in the presence or absence of a suitable base, such as triethylamine, diisopropylamine and the like in a suitable solvent such as toluene, methylene chloride, chloroform or tetrahydrofuran. Alternatively, one can generate the isocyanate in situ by the Curtius rearrangement of a carboxylic acid of the formula: 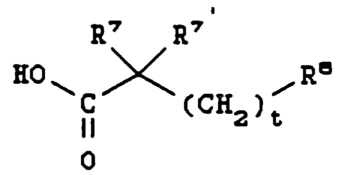 by an appropriate method. One such method is by the reaction of the carboxylic acid with diphenylphosphoryl azide in the presence of a suitable base, such as triethylamine or diisopropylethylamine, in a suitable solvent such as toluene, methylene chloride, chloroform and tetrahydrofuran and the like.

The carboxylic acids are either commercially available or can be prepared in a number of ways, which are known to those skilled in the art. For example, one can form the dianion of a carboxylic acid (or the monoanion of the corresponding ester) of the formula: 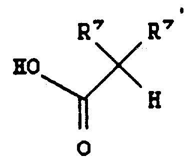 by deprotonation with a strong base, such as lithium diisopropyl amide or lithium hexamethyldisilazide, in a suitable solvent such as tetrahydrofuran and react the anion or dianion with an electrophilic reagent of the formula: 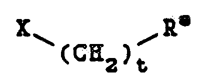 where X is an appropriate leaving group such as chloride, bromide, iodide, methanesulfonyl, p-toluenesulfonyl or trifluoromethanesulfonyl and the like.

Alternatively, one can alkylate a diester of malonic acid of the formula: 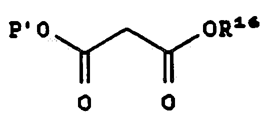 where P' is a suitable acid protecting groups such as methyl, ethyl, isopropyl, benzyl, tertiary-butyl, trimethylsilyl, t-butyldimethylsilyl, and the like, with appropriate electrophiles;

R⁷-X R^{7'}-X

where R⁷, R^{7'} and X are as defined above, in the presence of a suitable base such as sodium hydride, potassium hydride, sodium alkoxide or potassium alkoxide. Suitable alkoxides being methoxide, ethoxide, isopropoxide and tertiary-butoxide and the like. The reaction is carried out in a suitable solvent such as tetrahydrofuran, N,N-dimethylformamide or an alcohol solvent, such as methanol, ethanol, isopropanol or tertiary-butanol. The reaction with R⁷ and R^{7'} can be done sequentially if R⁷ and R^{7'} are different, or simultaneously if R⁷ and R^{7'} are identical or form a cyclic ring during the alkylation step. The resulting product is a mono- or di-substituted malonate diester of the formula:

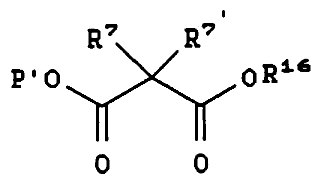

In order to generate the carboxylic acid required for the Curtius rearrangement, the acid protecting group P' is selectively removed. Suitable methods for removal are (1) hydrolysis with lithium hydroxide, sodium hydroxide, potassium hydroxide and the like, (2) acidolysis with an acid such as hydrochloric acid, hydrobromic acid, trifluoroacetic acid and the like, and (3) hydrogenolysis with hydrogen in the presence of a suitable catalyst such as palladium-on-carbon. The resulting carboxylic acid has the formula: 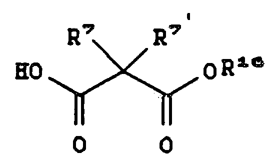 In the case where R⁸ is an amino group NR¹⁶R¹⁷, the amino group an be introduced either by displacement of an appropriate leaving group or reductive amination with an appropriate aldehyde. The displacement of the leaving group from an ester of the formula: 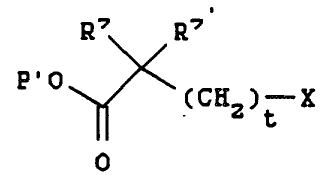 where P' and X are as defined above, can be readily accomplished by one skilled in the art. The protecting group P' is then removed by the methods discussed above to provide the required carboxylic acid of the formula: 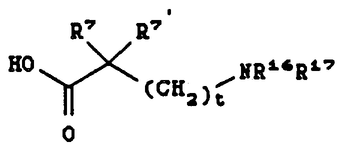 The reductive amination procedure is readily accomplished by the reaction of an aldehyde of the formula: 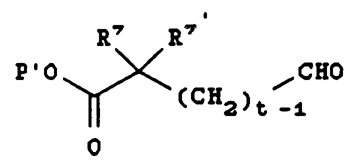 with the amine HNR¹⁶R¹⁷ in the presence of sodium cyanoborohydride or hydrogen and a suitable catalyst, such as palladium-on-carbon, and the acid protecting group P' is removed by the methods discussed above. The required aldehydes can be prepared by a number of methods well-known to those in the art. Such methods include reduction of an ester, oxidation of an alcohol or ozonolysis of an olefin.

In the case where R⁸ is a keto-group and t is O, one can mono- or dialkyate an ester of acetoacetic acid of the formula: 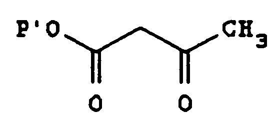 as described above for the malonate diesters to provide a compound of the formula: 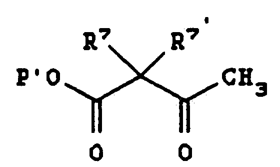 The acid protecting group can be removed to provide the desired carboxylic acid for the Curtius rearrangement, or the ketone can be converted to a ketal, such as the dimethyl ketal, diethyl ketal or ethylene glycol ketal, by reaction with the appropriate alcohol in the presence of a suitable acid, such as p-toluenesulfonic acid or the like, and a dehydrating agent such as trimethyl- or triethylorthoformate to provide, for example, a compound of the following formula: 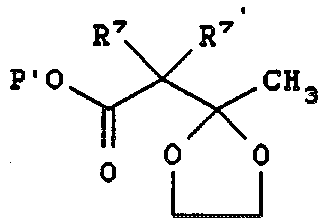 The protecting group P' can then be removed to provide a compound of the formula: 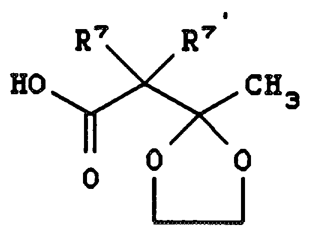 which is suitable for the Curtius rearrangement. If desired, the ketal group can be converted at any time during the subsequent synthesis to the corresponding ketone by hydrolysis in the presence of an acid such as aqueous hydrochloric acid.

The urea derivative of the amino alcohol, and the corresponding sulfur analog can be represented by the formula: 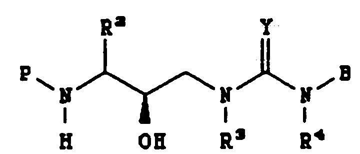 Following preparation of the urea derivative, or corresponding analogs wherein Y is S, the amino protecting group P is removed under conditions which will not affect the remaining portion of the molecule. These methods are well known in the art and include acid hydrolysis, hydrogenolysis and the like. A preferred method involves removal of the protecting group, e.g., removal of a carbobenzoxy group, by hydrogenolysis utilizing palladium on carbon in a suitable solvent system such as an alcohol, acetic acid, and the like or mixtures thereof. Where the protecting group is a t-butoxycarbonyl group, it can be removed utilizing an inorganic or organic acid, e.g., HCl or trifluoroacetic acid, in a suitable solvent system, e.g., dioxane or methylene chloride. The resulting product is the amine salt derivative. Following neutralization of the salt, the amine is then reacted with a substituted sulfonyl derivative of an amino acid or corresponding analog or derivative thereof represented by the formula (RSO₂N[CR^{1'}R^{1"}]ₜCH(R¹)COOH) wherein t, R¹, R^{1'} and R^{1"} are as defined above, to produce the antiviral compounds of the present invention having the formula: 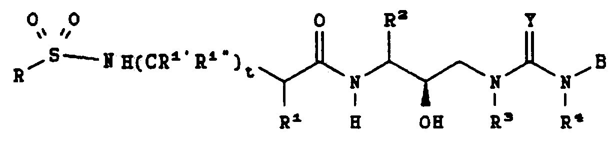 wherein t, B, R, R¹, R^{1'}, R¹", R, R³, R⁴, and Y are as defined above. The sulfonyl derivative of the amino acid or corresponding analog or derivative thereof is reacted with a substituted sulfonyl chloride (RSO₂CL) at a suitable pH value, e.g. pH9, to produce the corresponding sulfonamide. Alternatively, the amine salt can be reacted with a protected amino acid and the resulting compound deprotected and then reacted with a sulfonyl chloride. Where the amine is reacted with a substituted sulfonyl derivative of an amino acid, e.g., when t=1 and R^{1'} and R^{1"} are both H, so that the amino acid is a β-amino acid, such β-amino acids can be prepared according to the procedure set forth in a copending application, U. S. Serial No. 07/345,808. Where t is 1, one of R^{1'} and R^{1"} is H and R¹ is hydrogen so that the amino acid is a homo-β-amino acid, such homo-β-amino acids can be prepared by the same procedure. Where t is O and R¹ is alkyl, cycloalkyl,-CH₂SO₂NH₂ or an amino acid side chain, such materials are well known and many are commercially available from Sigma-Aldrich.

Alternatively, the protected amino alcohol from the epoxide opening can be further protected at the newly introduced amino group with a protecting group P' which is not removed when the first protecting P is removed. One skilled in the art can choose appropriate combinations of P and P'. One suitable choice is when P is Cbz and P' is Boc. The resulting compound represented by the formula: 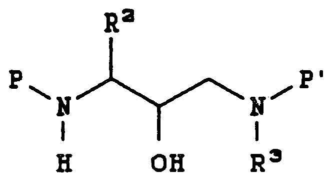 can be carried through the remainder of the synthesis to provide a compound of the formula: 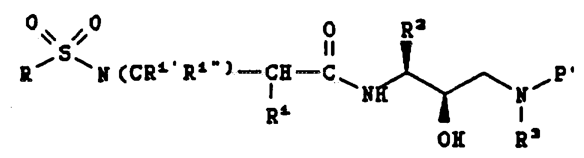 and the new protecting group P' is selectively removed, and following deprotection, the resulting amine reacted to form the urea derivative as described above. This selective deprotection and conversion to the urea can be accomplished at either the end of the synthesis or at any appropriate intermediate step if desired. This alternate procedure is also suitable for producing compounds of formula III.

It is contemplated that for preparing compounds of the Formulas having R⁶, the compounds can be prepared following the procedure set forth above and, prior to coupling the urea derivative or analog thereof to the amino acid PNH(CH₂)ₜCH(R¹)COOH, carried through a procedure referred to in the art as reductive amination. Thus, a sodium cyanoborohydride and an appropriate aldehyde R⁶C(O)H or ketone R⁶C(O)R⁶ can be reacted with the urea derivative compound or appropriate analog at room temperature in order to reductively aminate any of the compounds of Formulas I-VI. It is also contemplated that where R³ of the amino alcohol intermediate is hydrogen, the inhibitor compounds can be prepared through reductive amination of the final product of the reaction between the amino alcohol and the amine or at any other stage of the synthesis for preparing the inhibitor compounds.

Contemplated equivalents of the general formulas set forth above for the antiviral compounds and derivatives as well as the intermediates are compounds otherwise corresponding thereto and having the same general properties wherein one or more of the various R groups are simple variations of the substituents as defined therein, e.g., wherein R is a higher alkyl group than that indicated. In addition, where a substituent is designated as, or can be, a hydrogen, the exact chemical nature of a substituent which is other than hydrogen at that position, e.g., a hydrocarbyl radical or a halogen, hydroxy, amino and the like functional group, is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

All reagents were used as received without purification. All proton and carbon NMR spectra were obtained on either a Varian VXR-300 or VXR-400 nuclear magnetic resonance spectrometer.

### Example 1

This example illustrates preparation of compounds of the present invention.

### a) The procedure described below was used to prepare (2R,35)-3-[N-(n-butylsulfonyl)-L-tert-butylglycyl]amido-1-isoamyl-1-(tert-butylcarbamyl)amino-4-phenyl-2-butanol.

A solution of N-(n-butylsulfonyl)-L-tert-butylglycine (431.6 mg, 1.79 mmol), N-hydroxybenzotriazole (HOBT) (351.0 mg, 2.29 mmol), and 1-(3-dimethylaminopropyl)-3-ethylarbodiimide hydrochloride (EDC) (361.9 mg, 1.86 mmol) in 3 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (601.4 mg, 1.75 mmol) prepared as in Example 9, and stirred at room temperature for 16 h. The solution was poured into 50 mL of 60% saturated aqueous sodium bicarbonate solution. The aqueous solution was then extracted three times with dichloromethane, the combined organic extract was washed with 10% aqueous citric acid, brine, dried over anhydrous magnesium sulfate, filtered and concentrated to give 670.0, 66% of (2R,3S)-3-[N-(n-butylsulfonyl)-L-tert-butylglycyl]amido-1-isoamyl-1-(tert-butylcarbamoyl)amino-4-phenyl-2-butanol, mass spectrum (MH+) calc'd. for C₃₀H₅₅N₄O₅S: 583.3893. Found: 583.3893.

### b) The procedure described below was used to prepare (2R,3S)-3-[N-(E)-2-phenylethenesulfonyl-L-valyl]amido-1-isoamyl-1-(tert-butylcarbamoyl)amino-4-phenyl-2-butanol.

A solution of N-(E)-2-phenylethenesulfonyl-L-valine (386.9 mg, 1.54 mmol) N-hydroxybenzotriazole (HOBT) (317.8 mg, 2.08 mmol), and 1-(3-dimethylaminopropyl)-3-ethylcarbodiimide hydrochloride (EDC) (325 mg, 1.67 mmol) in 3 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (530.5 mg, 1.54 mmol), prepared as in Example 2, and stirred at room temperature for 16 h. The solution was poured into 50 mL of 60% saturated aqueous sodium bicarbonate solution. The aqueous solution was then decanted from the organic residue. the organic residue was taken up in dichloromethane and washed with 10% aqueous citric acid, brine, dried over anhydrous magnesium sulfate, filtered and concentrated to give 690 mg, 71% of (2R,3S)-3-[N-(E)-2-phenylethenesulfonyl-L-valyl]amido-1-isoamyl-1-(tert-butylcarbamoyl)amino-4-phenyl-2-butanol, mass spectrum (MH+) calc'd. for C₃₃H₅₀N₄O₅S: 615.3580. Found: 615.3580.

### c) The procedure described below was used to prepare (2R,3S)-3-[N-(E)-2-phenylethenesulfonyl-L-tert-butylglycyl]amido-1-isoamyl-1-(tert-butylarbamoyl)amino-4-phenyl-2-butanol.

A solution of N-(E)-2-phenylethenesulfonyl-L-tert-butylglycine (102. mg, 0.35 mmol), N-hydroxybenzotriazole (HOBT) (73.5 mg, 0.49 mmol), and 1-(3-dimethylaminopropyl)-3-ethylcarbodiimide hydrochloride (EDC) (69.8 mg. 0.36 mmol) in 2 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (120.8 mg, 0.35 mmol), prepared as in Example 2, and stirred at room temperature for 16 h. The solution was poured into 50 mL of 60% saturated aqueous sodium bicarbonate solution. The aqueous solution was then decanted from the organic residue. The organic residue was taken up in cichloromethane and washed with 10% aqueous citric acid, brine, dried over anhydrous magnesium sulfate, filtered and concentrated to give 100 mg, 45% of (2R,3S)-3-[N-(E)-2-phenylethenesulfonyl)-L-tert-butylglycyl]amido-1-isoamyl-1-(tert-butylcarbamoyl)amino-4-phenyl-2-butanol, mass spectrum (MH+) calc'd. for C₃₄H₅₂N₄O₅S: 629.3734. Found: 629.3734.

### d) The procedure described below was used to prepare (2R,3S)-3-[N-(E)-2-phenylethenesulfonyl-L-alanyl]amido-1-isoamyl-1-(tert-butylcarbamoyl)amino-4-phenyl-2-butanol.

A solution of N-(E)-2-phenylethenesulfonyl-L-alanine (165. mg, 0.65 mmol), N-hydroxybenzotriazole (HOBT) (160.8 mg, 1.06 mmol), and 1-(3-dimethylaminopropyl)-3-ethylcarbodiimide hydrochloride (EDC) (137.3 mg,0.70 mmol) in 7 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (224.4 mg, 0.65 mmol), prepared as in Example 2, and stirred at room temperature for 16 h. The solution was poured into 50 mL of 60% saturated aqueous sodium bicarbonate solution. the aqueous solution was then decanted from the organic residue. The organic residue was taken up in dichloromethane and washed with 10% aqueous citric acid, brine, dried over anhydrous magnesium sulfate, filtered and concentrated to give 110 mg, 29% of (2R,3S)-3-[N-(E)-2-phenylethenesulfonyl)-L-alanyl]amido-1-isoamyl-1-(tert-butylcarbomoyl) amino-4-phenyl-2-butanol, mass spectrum (MLi+) calc'd. for C₃₁H₄₆N₄O₅SLi: 593.3349. Found: 593.3315.

### e) The procedure described below was used to prepare (2R,3S)-3-[N-2-napthylsulfonyl-L-valyl]amido-1-isoamyl-1-(tert-butylcarbamoyl)amino-4-phenyl-2'-butanol.

A solution of N-(E)-2-napthylsulfonyl-L-valine (374.5 mg, 1.22 mmol) N-hydroxybenzotriazole (HOBT) (164.8 mg, 1.09 mmol), and 1-(3-dimethylaminopropyl)-3-ethylcarbodiimide hydrochloride (EDC) (233.5 mg, 1.20 mmol) in 2 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (415.8 mg, 1.21 mmol), prepared as in Example 2, and stirred at room temperature for 16 h. The solution was poured into 50 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 660 mg, 85% of (2R,3S)-3-[N-2-napthylsulfonyl-L-valyl]amido-1-isoamyl-1-(tert-butylcarbamoyl)amino-4-phenyl-2-butanol, mass spectrum (MH+) calc'd. for C₃₅H₅₀N₄O₅S: 639.3580. Found: 639.3580.

### f) The procedure described below was used to prepare (2R,3S)-3-[N-2-napthylsulfonyl-L-alanyl]amido-1-isoamyl-1-(tert-butylcarbamoyl)amino-4-phenyl-2-butanol.

A solution of N-(E)-2-napthylsulfonyl-L-alanine (638.1 mg, 2.28 mmol) N-hydroxybenzotriazole (HOBT) (466.1 mg, 3.09 mmol), and 1-(3-dimethylaminopropyl)-3-ethylcarbodiimide hydrochloride (EDC) (484.1 mg, 2.49 mmol) in 12 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (786.0 mg, 2.28 mmol), prepared as in Example 2, and stirred at room temperature for 16 h. The solution was poured into 50 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give a white solid which was isolated by filtration and air dried to give 410 mg, 30% of (2R,3S)-3-[N-2-napthylsulfonyl-L-alanyl]amido-1-isoamyl-1-(tert-butylcarbamoyl)amino-4-phenyl-2-butanol, mass spectrum (MH+) calc'd. for C₃₃H₄₆N₄O₅S: 611.3267. Found: 611.3267.

### g) The procedure described below was used to prepare (2R,35)-3-[N-2-napthylsulfonyl-L-tert-butylglycyl]amido-1-isoamyl-1-(tert-butylcarbamoyl)amino-4-phenyl-2-butanol.

A solution of N-(E)-2-napthylsulfonyl-L-tert-butylglycine (49.1 mg, 0.15 mmol) N-hydroxybenzotriazole (HOBT) (31.5 mg, 0.21 mmol), and 1-(3-dimethylaminopropyl) -3-ethylcarbodiimide hydrochloride (EDC) (32.1 mg, 0.16 mmol) in 1 mL of anhydrous dimethylformamide (DMF) cooled to 0°C and stirred under nitrogen for 0.5 h. This solution was then treated with (2R,3R)-3-amino-1-isoamyl-1-(tert-butylcarbamoyl)amino-4-phenyl-2-butanol (53.6 mg, 0.15 mmol), prepared as in Example 2, and stirred at room temperature for 16 h. The solution was poured into 5.0 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give a white solid which was isolated by filtration and air dried to give 74.1 mg, 30% of (2R,3S)-3-[(N-2-napthylsulfonyl-L-tert-butylglycyl]amido-1-isoamyl-1-(tert-butylarbamoyl)amino-4-phenyl-2-butanol, mass spectrum (MH+) calc'd. for C₃₂H₅₃N₄O₅S: 653.3736. Found: 653.3736.

### h) The procedure described below was used to prepare (2R,3S)-3-(N-methanesulfonyl-L-asparaginyl)amido-1-isoamyl-1-(tert-butylcarbamoyl)amino-4-phenyl-2-butanol.

A solution of (2R,3S)-3-(N-L-asparaginyl)amido-1-isoamyl-1-(tert-butylcarbamoyl)amino-4-phenyl-2-butanol (260 mg, 0.56 mmol), prepared as in Example 2, and triethylamine (2 equivalents) in 1 mL of tetrahydrofuran (THF) was treated with methanesulfonyl chloride (70 mg, 0.61 mmol). The solution was maintained at room temperture for 16 h and then concentrated *in vacuo.* The residue was taken up in dichloromethane, washed with saturated aqueous sodium bicarbonate, 1 N aqueous potassium bisulfate, saturated aqueous sodium chloride, dried over anhydrous magnesium sulfate, filtered and concentrated to give 180 mg, 59% of (2R,3S)-3-(N-methanesulfonyl-L-asparaginyl)amido-1-isoamyl-1-(tert-butylcarbamoyl)amino-4-phenyl-2-butanol, FAB mass spectrum (MH+) = 542.

### i) The procedure described below was used to prepare (2R,3S)-3-(N-methanesulfonyl-L-tert-butylglycyl)amido-1-isoamyl-1-(tert-butylcarbamoyl)amino-4-phenyl-2-butanol.

A solutio of N-methanesulfonyl-L-tert-butylglycine (237.7 mg, 1.14 mmol), N-hydroxybenzotriazole (HOBT) (262.4 mg, 1.74 mmol), and 1-(3-dimethylaminopropyl)-3-ethylcarbodiimide hydrochloride (EDC) (243.3 mg, 1.25 mmol) in 6 mL of anhydrous dimethylformamide (DMF) cooled to 0°C and stirred under nitrogen for 0.5 h. This solution was then treated with (2R,3R)-3-amino-1-awoamyl-1-(tert-butylcarbamoyl)amino-4-phenyl-2-butanol (390.8 mg, 1.13 mmol), prepared as in Example 2, and stirred at room temperatre for 16 h. The solution was poured into 30 mL of 60% saturated aqueous sodium bicarbonate solution. The aqueous solution was then decanted from the organic residue. The organic residue was taken up in dichloromethane and washed with 10%aqueous citric acid, brine, dried anhydrous magnesium sulfate, filtered and concentrated to give 435.8 mg, 72% of (2R,3S)-3-(N-methanesulfonyl-L-tert-butylglycyl)amido-1-isoamyl-1-(tert-butylcarbamoyl)amino-4-phenyl-2-butanol, mass spectrum (MH+) calc'd. for C₂₇H₄₈N₄O₅S: 541.3424. Found: 541.3424.

### j) The procedure described below was used to prepare (2R,3s)-3-(N-2-phenylethanesulfonyl-L-alanyl)amido-1-isoamyl-1-(tert-butylcarbamoyl)amino-4-phenyl-2-butanol.

A solution of (2R,3S)-3-[N-(E)-2-phenylethenesulfonyl)-L-alanyl]amido-1-isoamyl-1-(tert-butylcarbamoyl)amino-4-phenyl-2-butanol (250 mg, 0.43 mmol), prepared as in Example 2, in 20 mL of methanol was charged into a Fisher-Porter bottle along with 10% palladium on carbon atalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtered through a pad of celite to remove the catalyst and the filterate concentrated *in vacuo* to give 200 mg, 79% of (2R,3S)-3-(N-2-phenylethanesulfonyl-L-alanyl)amido-1-isoamyl-1-(tert-butylarbamoyl)amino-4-penyl-2-butanol, FAB mass spectrum (MLi+) = 595.

### k) The procedure described below was used to prepare (2R,3S)-3-(N-2-phenylethanesulfonyl-L-valyl)amido-1-isoamyl-1-(tert-butylcarbamoyl)amino-4-phenyl-2-butanol.

A solution of (2R,3S)-3-[N-(E)-2-phenylethenesulfonyl-L-valyl]amido-1-isoamyl-1-(tert-butylcarbamoyl)amino-4-phenyl-2-butanol. (300 mg, 0.49 mmol) in 20 mL of methanol was chrged into a Fisher-Porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtred through a pad of celite to remove the catalyst and the filterate concentrated *in vacuo* to give 280 mg, 93% of (2R,3S)-3-(N-2-phenylethanesulfonyl-L-valyl)amido-1-isoamyl-1-(tert-butylcarbamoyl)amino-4-phenyl-2-butanol, FAB mass spectrum (ML+) = 623.

### l) The procedure described below was used to prepare (2R,3S)-3-(N-2-phenylethanesulfonyl-L-tert-butylglyhcyl)amido-1-isoamyl-1-(tert-butylcarbamoyl) amino-4-phenyl-2-butanol.

A solution of (2R,3S)-3-[N-(E)-2-phenylethenesulfonyl-L-tert-butylglycyl]amido-1-isoamyl-1-(trt-butylcarbamoyl)amino-4-phenyl-2-butanol. (150 mg, 0.24 mmol) in 20 mL of methanol was charged into a Fisher-Porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtred through a pad of celite to remove the catalyst and the filterate concentrated *in vacuo* to give 140 mg, 92% of (2R,3S)-3-(N-2-phenylethanesulfonyl-L-tert-butylglycyl)amido-1-isoamyl-2-(tert-butylcarbamoyl)amino-4-phenyl-2-butanol, FAB mass spectrum (MLi+) = 637.

### m) The procedure described below was used to prepare (2R,3S)-3-(N-2-phenylethanesulfonyl-L-asparaginyl)amido-2-isoamyl-2-(tert-butylcarbamoyl)amino-4-phenyl-2-butanol).

A solution of (2R,3S)-3-[N-(E)-2-phenylethenesulfonyl-L-asparaginyl]amido-1-isoamyl-1-(tert-butylcarbamoyl)amino-4-phenyl-2-butanol. (50 mg, 0.79 µmol) in 20 mL of methanol was chrged into a Fisher-porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtered through a pad of celite to remove the catalyst and the filterate concentrated *in vacuo* to give 40 mg, 80% of (2R,3S)-3-(N-2-phenylethanesulfonyl-L-asparaginyl)amido-1-isoamyl-1-(tert-butylcarbamoyl)amino-4-phenyl-2-butanol, FAB mass spectrum (MH+)=632.

Following the procedure shown above, many of the compounds shown in the remaining examples could be prepared as sulfonamides.

### Example 2A

This example illustrates preparation of compounds wherein B represents: 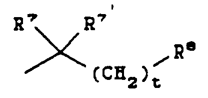 Methyl aminoisobutyrate hydrochloride. 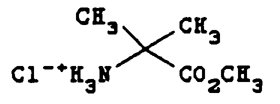 A 500 mL round bottomed flask equipped with nitrogen inlet, thermometer adapter, solids addition funnel, and magnetic stirrer was placed in an ice salt bath and charged with 100 mL of methanol. To this solution was added thionyl chloride(18.0 mL, 0.25 mol) via syringe at such a rate that the internal temperature was maintained at less than 0 °C. This solution was then treated with aminoisobutyric acid(19.6g, 0.19 mol) portion wise from the addition funnel at such a rate that the temperature did not rise above 5 °C. The ice bath was removed and replaced with an oil bath and the solution warmed to 50 °C for 1h and then concentrated *in vacuo.* The salt was thoroughly dried under vacuum to give the desired product as a white solid 28g, 96%, mp 185 °C. ¹H nmr (CDCl₃) 300 MHz 8.87(brs, 3H), 3.74(s, 3H), 1.65(s, 6H).

2,5,9,11-Tetraazatridecan-13-oio acid, 3-(2-amino-2-oxoethyl)-7-hydroxy-12,12-dimethyl-9-(3-methylbutyl)-1,4,10-trioxo-6-(phenylmethyl)-1-(2-quinolinyl)-methyl ester, [3S- (3R*,6R*, 7S*)]- 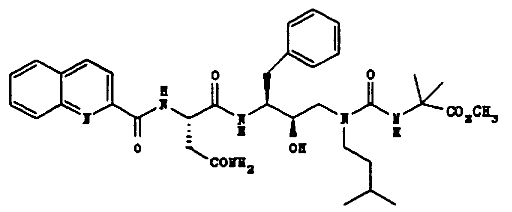 A 100 mL round bottomed flask equipped with a reflux condenser, nitrogen inlet, and magnetic stir bar was charged with methyl aminoisobutyrate hydrochloride (298 mg, 1.95 mmol) and 30 mL of chloroform. The slurry was warmed to reflux whereupon the salt dissolved and then the solution was treated with carbonyldiimidazole (317 mg, 1.95 mmol) and maintained at this temperature for 40 m. In a separate 50 mL round bottomed flask was placed the hydrochloride of (2R,3S)-3-amino-1-(3-methylbutyl)-1-[(1,1-dimethylethyl)amino]carbonyl)-4-phenyl-2-butanol (910 mg, 1.68 mmol), 20 mL of chloroform and triethylamine (648 mg, 6.40 mmol). This mixture was stirred at room temperature for 30 m and then added to the 100 mL round bottomed flask. The entire mixture was heated to 50 °C for 16 h and then poured into a separatory funnel. The mixture was diluted with 5% aq. citric acid and the phases separated. The organic phase was washed with an additional portion of citric acid, sat. aq. NaHCO₃, brine, dried over anhyd. MgSO₄, filtered and concentrated *in vacuo* to give a white solid, 820 mg, 75%, that was further purified by flash chromatography over SiO₂ eluting with methanol/CH₂Cl₂. The pure product was isolated by concentration of the appropriate fraction, 410 mg, 38% yield along with 150 mg of 95% pure product.

### Example 2B

### General Procedure for Curtius Rearrangement and Reaction with Amino Alcohol Derivative.

To a solution of 1 mmol of carboxylic acid in 12 mL of toluene and 3 mmol of triethylamine at 90°C under a nitrogen atmosphere, was added 1 mmol of diphenylphosphoryl azide. After 1 hour, a solution of 1 mmol of amino alcohol derivativbe in 3.5 mL of either N,N-dimethylformamide or toluene was added. After 1 hour, the solvent was removed under reduced pressure, ethyl acetate and water added and the layers separated. The organic layer was washed with 5% citric acid, sat d sodium bicarbonate, brine, dried, filtered and concentrated to afford the crude product. This was then recrystallized or chromatographed on silica gel to afford the purified final compound.

### Preparation of mono-tertiary-butyl 2,2-dimethylmalonate.

To a solution of mono-methyl mono-t-butyl malonate (20.5g, 117.7 mmol) in THF (275 ml) was added NaH (2.95g, 117.7 mmol) in portions at 0°C over 15 min then stirred at r.t. for 30 min. The mixture was then cooled to 0°C and to this was added MeI (7.5 ml, 118 mmol) slowly and stirred at r.t. for 1 h. After it was cooled to 0°C, to this cold solution was added NaH (2.95g, 118 mmol) then MeI (7.5 ml, 118 mmol) by -following the procedure described above. A usual workup (10 ml sat. NH₄Cl, 100 ml Et₂O-pet ether, 5% HCl, 5% NaHCO₃, finally sat. NaCl) gave 16.2g (68%) of desired product as a pale yellow oil. The oil (10.1g, 50.0 mmol) was dissolved in MeOH (150 ml) and to this was added 1.25N NaOH (20 ml of 2.5N NaOH with 20 ml of H₂O, 50.0 mmol) over a period of 2 h and stirred at 0°C for 3 h and r.t. for 16 h. Removal of solvents in vacuo (<40°C) gave an oil. The oil was dissolved in water (125 ml) and extracted with Et₂O (25 ml). The aqueous layer was collected and acidified with 6N HCl (a white precipitate was formed immediately) to pH ∼ 1 and extracted with Et₂O (75 ml x 3). The combined extracts were washed with sat. NaCl (50 ml), dried (Na₂SO₄) and concentrated to afford 7.1g (75%) of mono-tertiary-butyl 2,2-dimethylmalonate as a white solid.

### Preparation of 2,2-Dimethylmalonate, mono-ethyl ester.

To a suspension of NaH (2.5g, 95%, 100 mmol) in dry THF (200 ml) was added diethylmalonate (8.0g, 50 mmol) slowly at 0°C and stirred at r.t. for 1 h. The solution was cooled to 0°C and to this was added a solution of MeI (14.9g, 105 mmol) in THF (20 ml) slowly. The mixture was stirred at 0°C for 1 h, r.t. for 2 h and diluted with Et₂O-Pet ether (5:1, 150 ml) then washed with H₂O (80 ml) and sat. NaCl solution (50 ml). The organic phase was separated, dried (Na₂SO₄) and concentrated to afford 8.2g (87%) of desired product as an oil. This oil was dissolved in EtOH (50 ml) and cooled to 0°C. To this cold solution was added 10% NaOH (20 ml, 50 mmol) dropwise at 0°C and stirred at 0°C for 2 h, at r.t. for 16 h. Removal of solvents gave an oil. The oil was dissolved in H₂O (40 ml) and Et₂O (20 ml). The aqueous layer was separated and acidified with 6N HCl to pH ∼ 1 then extracted with ether (50 ml x 2). The combined extracts were washed with sat. NaCl (20 ml), dried (Na₂SO₄) and concentrated to afford 6.5g (81%) of desired acid as an oil.

### Preparation of 2-Ethyl-2-methylmalonate, mono-ethyl ester.

To a suspension of NaH (1.25g, 95%, 50 mmol) in dry THF (200 ml) was added diethylmalonate (8.0g, 50 mmol) slowly at 0°C. The reaction mixture was allowed to warm up to r.t. and stirred for 1 h then cooled to 0°C. To this cold solution was added MeI (7.1g, 50 mmol) dropwise. After the resulting mixture was stirred at r.t. for 2 h, it was cooled to 0°C. To the cold solution was added EtBr (5.6g, 5.1 mmol) and stirred at r.t. for 2 h. The mixture was diluted with ether-Pet ether (5:1, 150 ml) and washed with H₂O (50 ml), sat. NaCl solution, dried (Na₂SO₄) and concentrated to afford 10g (99%) of desired product as an oil. This oil was dissolved in EtOH (50 ml) and cooled to 0°C. To this cold solution was added 10% NaOH (20 ml, 50 mmol) dropwise via an additional funnel and stirred at 0°C for 2 h, at r.t. for 16 h. Removal of solvents gave an oil. The oil was dissolved in H₂O (40 ml) and Et₂O (20 ml). The aqueous layer was separated and acidified with 6N HCl to pH ∼ 1 then extracted with ether (50 ml x 2). The combined ether solutions were washed with sat. NaCl (20 ml), dried (Na₂SO₄), and concentrated to afford 7.2g (83%) of desired acid as an oil.

### Preparation of 2,2-Dimethyl-3-(4-morpholinyl)propionic Acid.

Dissolve 2.62 ml (30 mmoles,1.2eq.) oxalyl chloride in anhydrous CH₂Cl₂. Cool to -78 degrees C under N₂. Slowly add 2.66 ml (37.5 mmoles,1.Seq.) DMSO. Stir 15 minutes. To this solution add 3.19 ml (25 mmoles,1.0 eq.) methyl 2,2 dimethyl-3-hydroxypropionate. Stir an additional hour at -78 degrees. Quench reaction with 13.94 ml (100 mmoles,4.0 eq.) triethylamine. Warm to room temperature. Wash organic layer 1x0.1NHCl, 1x saturated sodium bicarbonate, 1x saturated NaCl. Dry with MgSO₄ and rotovap. Yield=69% M+H=131

Dissolve 1.00ml (11.53 mmoles, 3eq.) morpholine in 43ml 1% AcOH/MeOH. Add 500mg (3.83 mmoles, leq.) aldehyde from above. Cool to 0 degrees C under N₂. Slowly add 362.0mg (5.76 mmoles,1.5eq) NaCNBH₃. Stir 2-3 days. Strip off MeOH. Dissolve in minimum H₂O. Add Conc HCl to pH=2. Wash 2xEt₂O. Add 6N NaOH to aqueous layer to pH>9. Extract 3xEtOAc. Dry with MgSO₄ and rotovap. Purify by silica flash chromatography (60:1 CH2Cl₂:CH₃OH) Yield=18% M+H=202

Dissolve 337mg (1.7mmoles, 1eq) methyl ester from above in 18ml AcOH. Add 4.5ml HCl. Heat to 60 degrees C. under N₂. Stir overnight. Rotovap off solvent. Azeotrope with toluene. Rotovap 1x 4N HCl/ dioxane. Desscicate over P₂O₅ overnight. Yield=94% M+H=188

### Preparation of 2,2-dimethyl-4-(1-methylpiperazinyl)butanoic acid.

A mixture of 2,2-dimethylpentenoic acid (5.66g, 42 mmol) BnBr (6.84g, 40 mmol) K₂CO₃ (5.8g, 42 mmol) and NaI (3g, 20 mmol) in acetone (65 ml) was heated to reflux (oil bath 80°C) for 16 h. The acetone was stripped off and the residue was dissolved in H₂O (20 ml) and ether (60 ml). The ether layer was separated, dried (Na₂SO₄) and concentrated to afford 8.8g (100%) of desired ester as an oil.

To a solution of ester from above (8.8g, 40.0 mmol) in CH₂Cl₂ (150 ml) was bulbed through a stream of ozone at-78°C until the blue color persisted. Excess ozone was removed by a stream of N₂, dimethylsulfide (10 ml) was added, and the reaction mixture was allowed to warm to room temperature and stirred for 56 h. After the removal of solvents, the residue was dissolved in Et₂O (50 ml) and washed with H₂O (15 ml) then sat. NaCl soltuion (10 ml). The organic layer was dried (Na₂SO₄) concentrated to afford 8.2 g (93%) of aldehyde as an oil.

To a solution of aldehyde from above (4.2g, 19.1 mmol) in MeOH (80 ml) was added NaCNBH₃ (2.4g, 38.2 mmol) and acetic acid (2 ml) at 0°C. To this cold solution was added N-methylpiperizine (2.5g, 25 mmol) slowly at 0°C. The reaction mixture was stirred at 0°C for 2 h and room temperature for 16 h. The removal of solvents gave a solid. To the solid was added H₂O (25 ml) and ether (50 ml). The organic layer was separated and to this was added 5% HCl (25 ml). The aqueous layer was collected and to this was added 2.5N NaOH until pH ∼ 14, and extracted with ether (25 ml x 3). The combined organic extracts were washed with brine (15 ml), dried (Na₂SO₄) and concentrated to afford 5.5g (95%) of desired amine as an oil. This oil was hydrogenated (1.5g of 10% Pd/C, 50 psi H₂) in MeOH (50 ml) at room temperature for 2 h. the reaction mixture was filtered and the filtrate was concentrated to afford 4.0g (98%) of the desired amino acid as a white solid.

### Preparation of 2,2-dimethyl-6-(4-morpholinyl)-4-oxohexanoic acid.

To a suspension of NaH (1.5g, 60.0 mmol) in THF (155 ml) was added a solution of methyl 2,2-dimethyl-3-hydroxypropionate (6.6g, 50.0 mmol) slowly at 0°C. After the addition was completed, the reaction mixture was stirred at room temperature fo 1 1/2 h. It was cooled to 0°C. To this cold solution was added allyl bromide (7.3g, 60.0 mmol) slowly and NaI (150 mg) in one portion. The resulting reaction mixture was stirred at room temperature for 36 h. Diluted with 5:1 etherpetane (100 ml) and washed with H₂O (50 ml), brine (50 ml). The combined organic phases were dried (Na₂SO₄), concentrated to give 8.4g (74%) of olefin.

To a solution of olefin (3.45g, 20 mmol) in CH₂Cl₂ (75 ml) was bulbed through a stream of ozone at -78°C until the blue color persisted. Excess ozone was removed by a stream of N₂, dimethyl sulfide (5 ml) was added, and the reaction mixture was allowed to warm to room temperature and stirred for 36 h. After removal of all solvents, the residue was dissolved in Et₂O (35 ml) and washed with H₂O (10 ml) and sat. NaCl solution (10 ml). The organic extracts were dried (Na₂SO₄), concentrated to afford 3.2g (92%) of aldehyde. To a solution of this aldehyde (3.2g, 18.4 mmol) in MeOH (80 ml) was added Na CNBH₃ (2.3g, 36.8 mmol) and acetic acid (2 ml) at 0°C. To this cold solution was added morpholine (2g, 23 mmol) slowly at 0°C and stirred for 2 h at 0°C, 16 h at room temperature. The removal of solvents gave a solid, to this solid was added H₂O (20 ml) and Et₂O (50 ml). The organic layer was separated and to this was added 5% HCl (20 ml). The aqueous layer was collected and to this was added 2.5N NaOH solution until pH ∼ 14, and extracted with Et₂O (25 ml x 3). The combined organic extracts were washed with brine (15 ml), dried (Na₂SO₄) and concentrated to afford 1.6g (35%) the desired amine. The amine (1.58g, 6.4 mmol) was subjected to a mixture of 10% NaOH (13 ml, 32 mmol) and MeOH (10 ml) and stirred for 16 h. Acetic acid (2.5 ml, 41.6 mmol) was added and the solvents were removed in vacuo to give a solid. The solid was washed with CH₂Cl₂ (25 ml x 4). The combined CH₂Cl₂ solutions were dried (Na₂SO₄) and concentrated to give an oil. The purification of the oil by plug filtration (silica gel, 20% MeOH/CH₂Cl₂ the MeOH) gave 350 mg (24%) pure amine acid as an oil.

Following generally the general procedure set forth below and the procedures set forth in Examples 1A and 2B, the compounds listed in Table 1 were prepared.

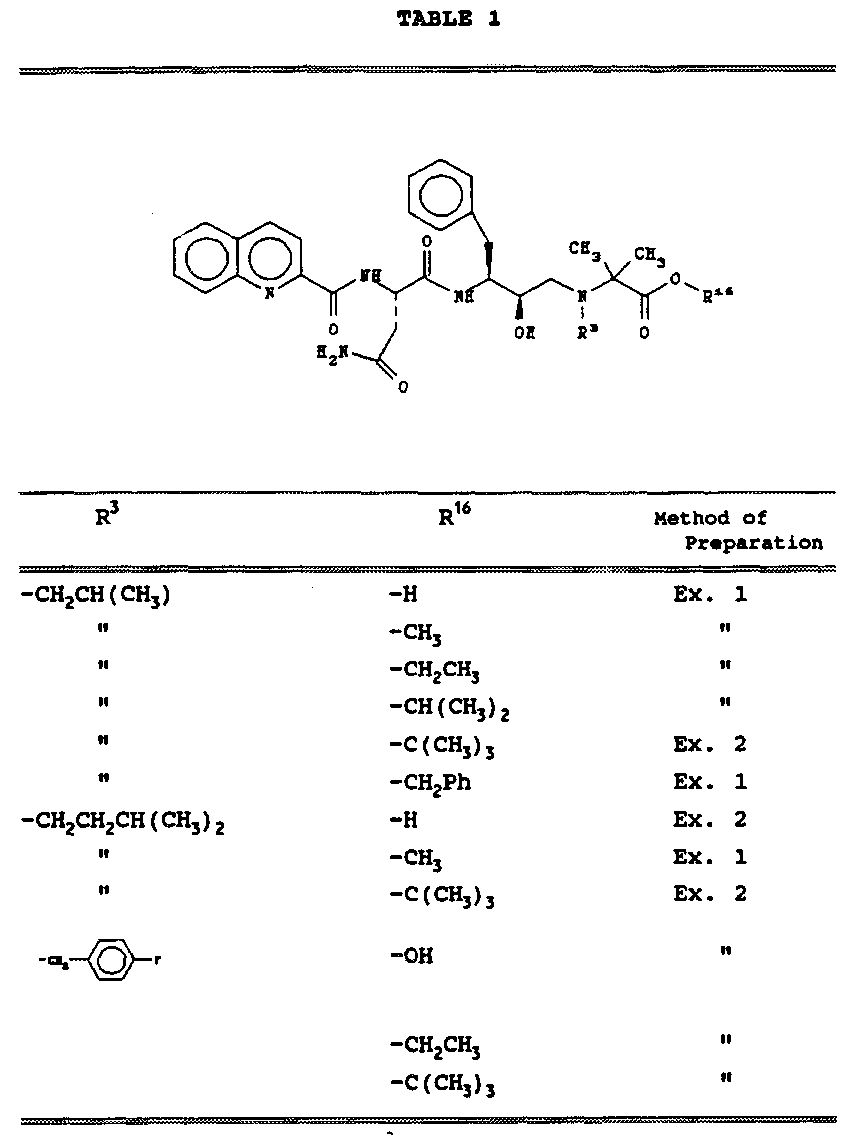

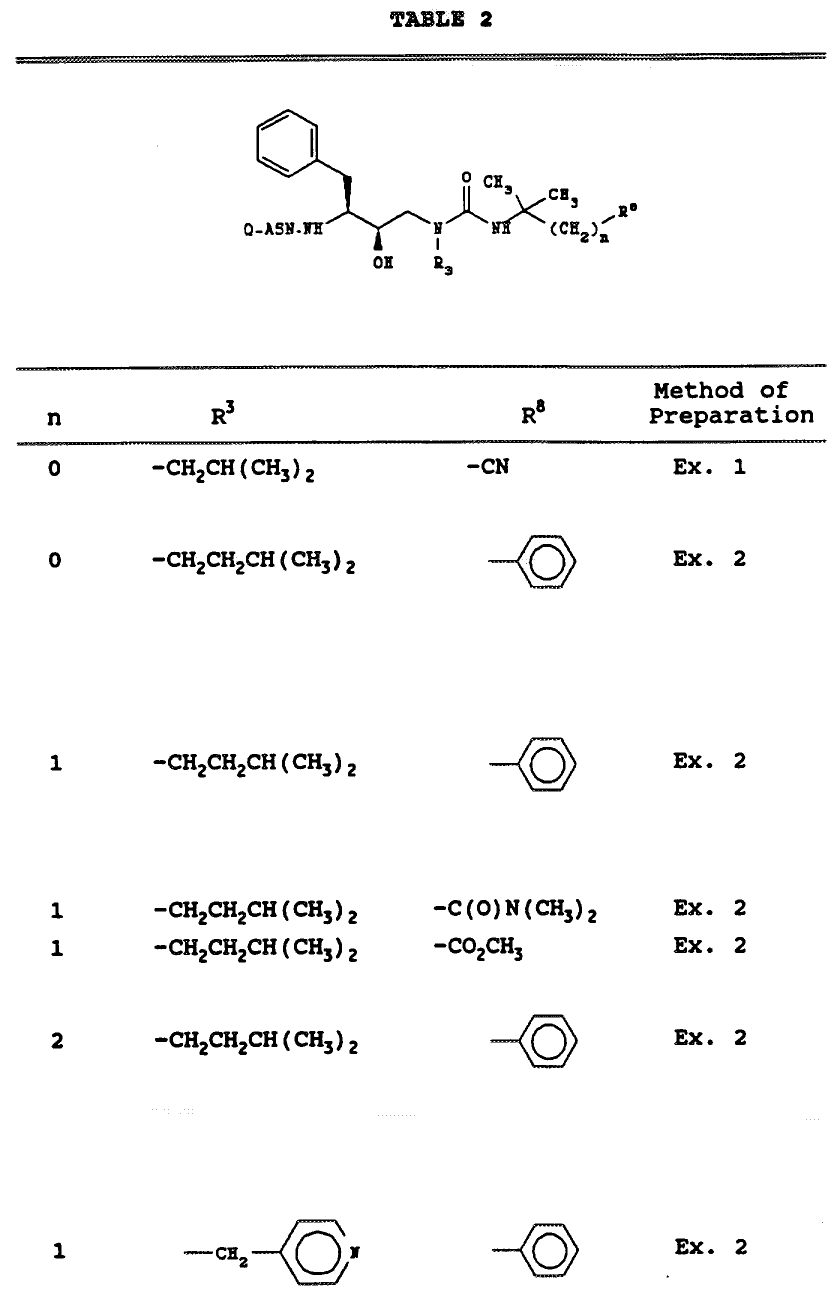

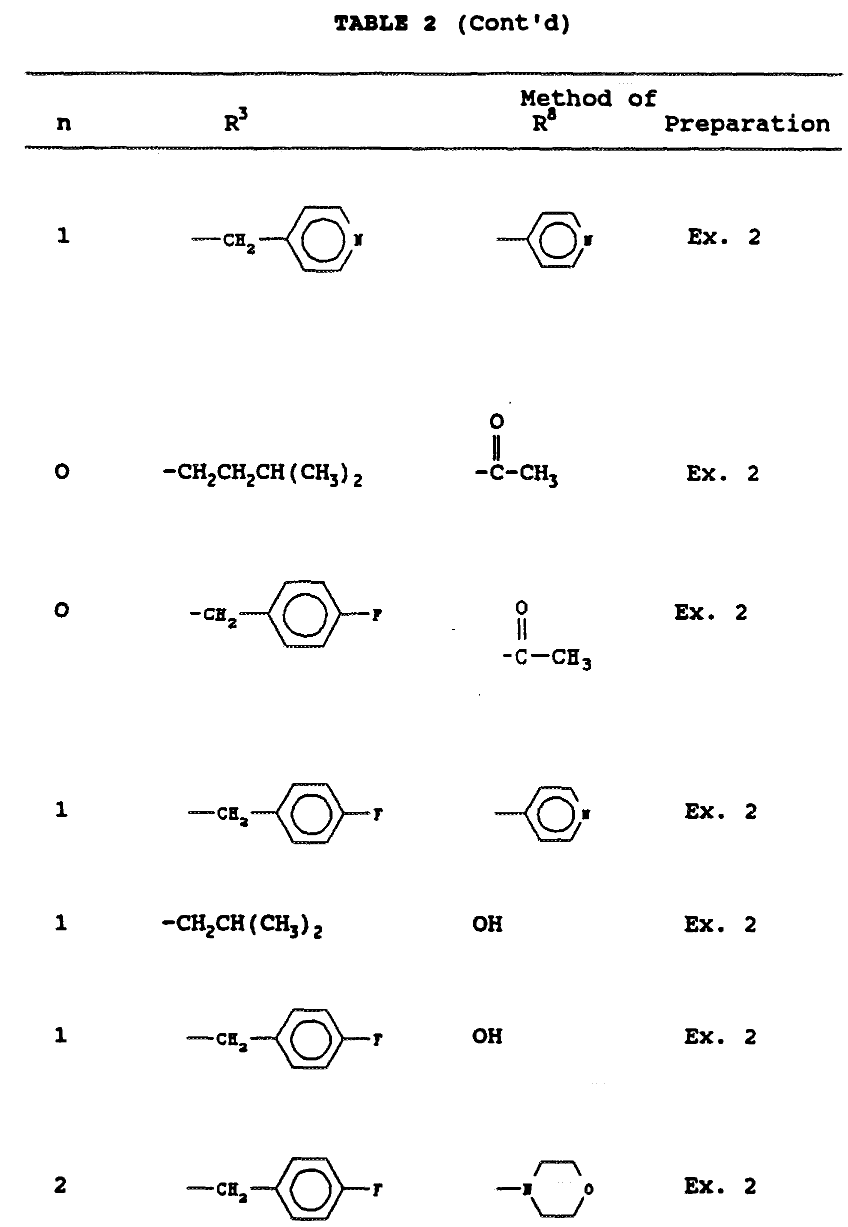

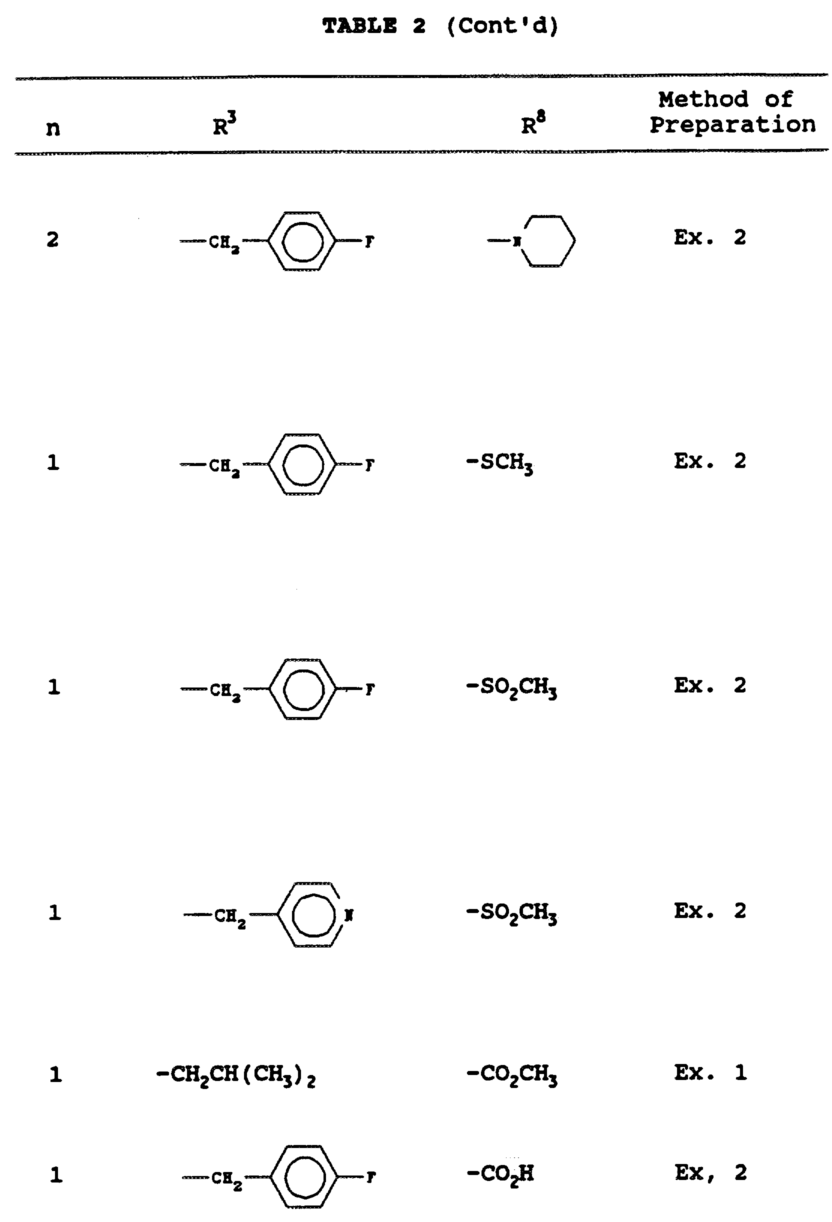

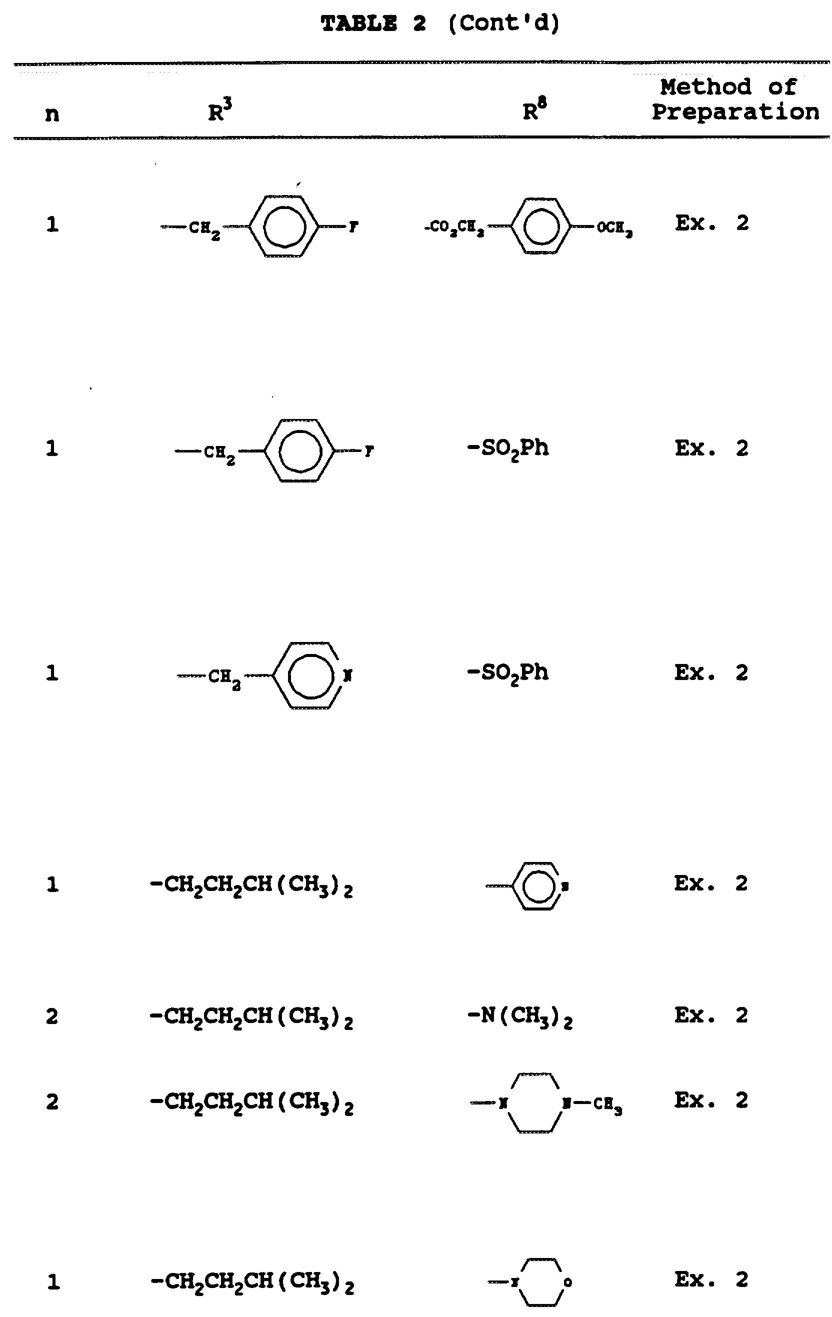

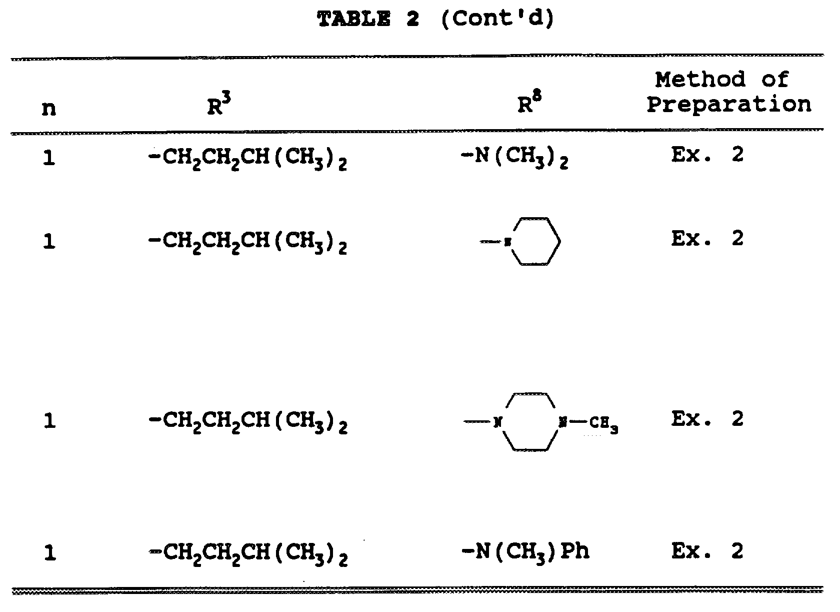

### Example 3

Following generally the procedure of Examples 2A and B, the compounds shown in Table 3 were prepared.

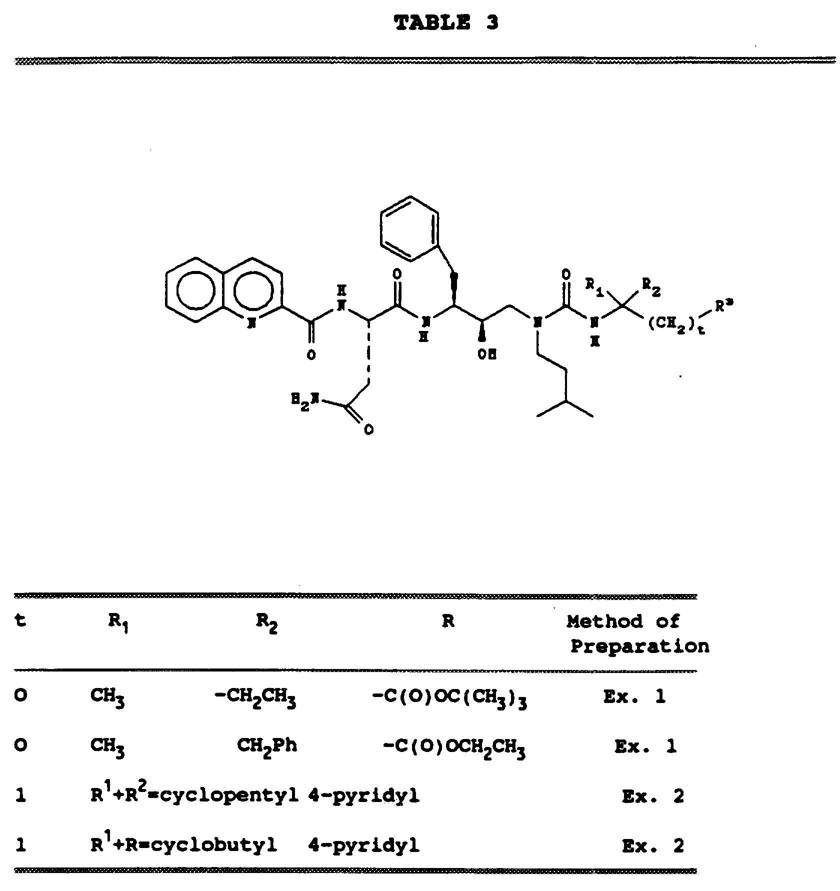

### Example 4

Following generally the procedure of Example 1, the compounds shown in Table 4 were prepared.

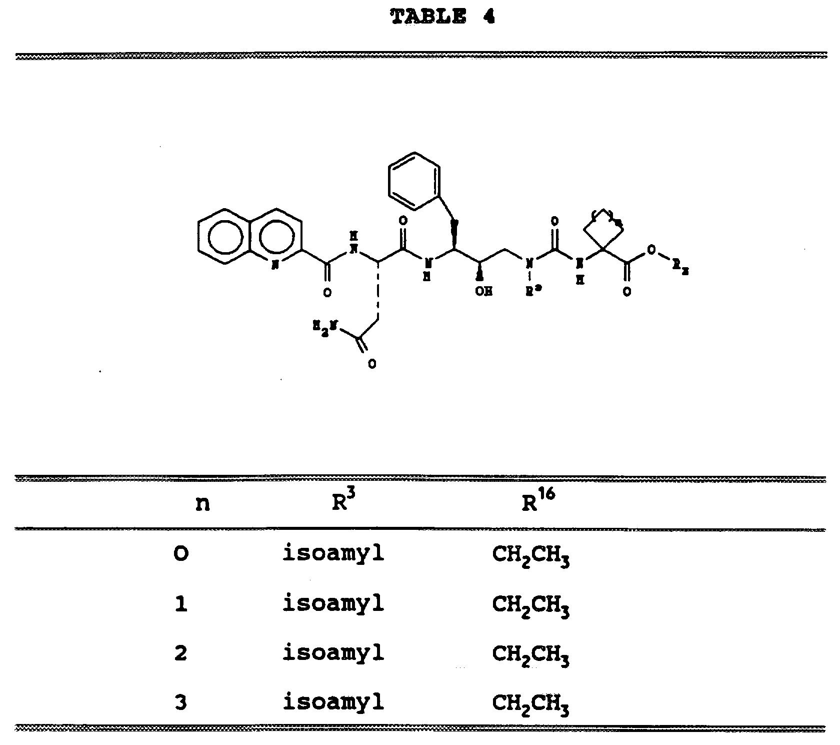

### Example 5

This example illustrates an alternate procedure for preparing compounds of Formula II.

### A. Intermediates

### Preparation of 2,2-Dimethyl-3-phenylpropionic Acid

To a mixture of 1.23g (41.0 mmol) of 80% sodium hydride and 50 mL of anhydrous tetrahydrofuran under a nitrogen atmosphere, was added 3.88g (38.3 mmol) of diisopropylamine and then 3.3g (37.5 mmol) of isobutyric acid. After heating at reflux for 15 minutes and cooling to 0°C, 15 mL (37.5 mmol) of 2.5M n-butyllithium in hexane was added. The mixture was warmed to 35°C for 30 min, cooled to 0°C and 6.40g (37.5 mmol) of benzyl bromide added. The mixture was stirred for 30 minutes at 0°C, then warmed to 35°C for one hour and recooled to 0°C. Water was added and the aqueous layer extracted with diethyl ether, acidified with 6N aqueous HCl and extracted with diethyl ether. The organic layer was dried and concentrated to provide 4.0g of crude product. Chromatography on silica gel using 10% methanol/methylene chloride afforded 1.0g of pure 2,2-dimethyl-3-phenylpropionic acid, m/e 185 (M + Li).

### Preparation of 2,2-Dimethyl-3-(4-pyridyl)propionic Acid

Under a nitrogen atmosphere, 1.23g (41 mmol) of 80% sodium hydride was added to 50 mL of anhydrous tetrahydrofuran, followed by 3.88g (38.3 mmol) of diisopropylamine. To the resulting mixture was added 3.3g (37.5 mmol) of isobutyric acid and the resulting mixture heated to reflux for 15 minutes. Upon cooling to 0°C, 15 mL (37.5 mmol) of 2.5M n-butyllithium in hexane was added and the mixture then warmed to 35°C for 30 minutes. After cooling to 0°C, 4.8g (37.5 mmol) of 4-chloromethylpyridine (freshly prepared by neutralization of the hydrochloride salt with aqueous sodium bicarbonate, extraction with hexane, dried and concentrated) was added. After 30 minutes at 0°C, the mixture wa warmed to 30°C for one hour, cooled to 0°C and 50 mL of water carefully added. The aqueous layer was separated and washed twice with diethyl ether., acidified with 6N aqueous hydrochloric acid, rewashed twice with diethyl ether and then neutralized with aqueous sodium bicarbonate. After the addition of citric acid, the aqueous layer was extracted 3 xs with ethyl acetate to afford 283 mg of a white powder which was identified as 2,2-dimethyl-3-(4-pyridyl)propionic acid, m/e 180 (M + H⁺).

### Preparation of 1-(4-Pyridylmethyl)cyclopentanecarboxylic Acid

To a suspension of 3.69g (123 mmol) of 80% sodium hydride in 150 mL of anhydrous tetrahydro-furan and 11.6g (115 mmol) of diisopropylamine, at 0°C was added 12.82g (112 mmol) of cyclo-pentanecarboxylic acid. This was then heated at reflux for 15 minutes, cooled to 0°C and 45 mL of 2.5M n-butyllithium in hexane added. After 15 minutes at 0°C and 30 minutes at 35°C, the mixture was recooled to 0°C and a solution of 14.4g (112 mmol) of 4-chloromethylpyridine (freshly prepared from 4-chloromethylpyridine hydrochloride by neutralization with aqueous sodium bicarbonate, extraction with hexane, drying and concentrating) was added. After 30 minutes at 0°C and 60 minutes at 35°C, the mixture was cooled to 0°C, water carefully added and extracted twice with diethyl ether. The aqueous layer was acidified to pH3 with 6N hydrochloric acid whereupon a precipitate formed. The pH was adjusted to 5.9 with 10% sodium hydroxide and the solids collected. Recrystallization from hot ethanol and hexane afforded 5.7g of desired product, m/e 206 (M + H).

### Preparation of 2,2-Dimethyl-3-(methylsulfonyl)propionic Acid.

To a suspension of 1.23g (41 mmol) of 80% sodium hydride in 50 mL of anhydrous tetrahydrofuran and 3.88g (38 mmol) of diisopropylamine, was added 3.3g (37 mmol) of isobutyric acid and the mixture refluxed for 30 minutes. Upon cooling to 0°C, 15 mL (37 mmol) of 2.5M n-butyllithium in hexane was added, stirred for 15 minutes at 0°C and then warmed to 35°C for 45 minutes. After cooling to 0°C, 3.62g (37 mmol) of chloromethyl methyl sulfide was added and stirred for 30 minutes at 0°C and then 60 minutes at 35°C. After cooling to 0°C, waster was added, washed with diethyl ether, acidified with 6N hydrochloric acid and extracted with diethyl ether, dried and concentrated to afford 4g of crude material. This was distilled (Bp 85°C, 0.25 mm Hg) to afford 1.2g of 2,2-dimethyl-3-(thiomethyl)propionic acid, m/e 155 (M + Li). To a solution of 525 mg (3.5 mmol) of 2,2-dimethyl-3-(thiomethyl)propionic acid in 8 mL of acetic acid was added 1.2 mL of 30% aqueous hydrogen peroxide and the mixture refluxed for 2 hours. The solution was cooled, 15 mL of 10% sodium sulfite added and concentrated under reduced pressure. The residue was acidified with 12N hydrochloric acid, extracted with ethyl acetate, washed with brine, dried and concentrated to afford 341 mg of 2,2-dimethyl-3-(methylsulfonyl)-propionic acid.

### Preparation of 2,2-Dimethyl-3-(phenylsulfonyl)propionic Acid.

To a mixture of 1.23g (41 mmol) of 80% sodium hydride in 50 mL of dry tetrahydrofuran was added 3.88g (38.3 mmol) of diisopropylamine and then 3.3g (37.5 mmol) of isobutyric acid. After heating at reflux for 15 min, and cooling to 0°C, 15 mL (37.5 mmol) of a 2.5M n-butyllithium in hexane solution was added over 10 min. After 15 min, the solution was heated to 35°C for 30 min, cooled to 0°C and 5.94g (37.5 mmol) of chloromethylphenyl sulfide was added. After 30 min at 0°C and 35°C for 1 hour, the solution was recooled to 0°C, water added and then diethyl ether. The water layer was separated, acidified and extracted with diethyl ether, dried and concentrated to afford 4.23g of crude product. Recrystallization from methylene chloride/hexane afforded 1.49g of 2,2-dimethyl-3-(thiophenyl)propionic acid, m/e 211 (M + H). To a mixture of 1.1g (5.2 mmol) of 2,2-dimethyl-3-(thiophenyl)propionic acid in 12 mL of acetic acid was added 1.8 mL (17.8 mmol) of 30% aqueous hydrogen peroxide. After 10 minutes at room temperature, all the solids dissolved and the solution was heated to reflux for two hours. After cooling in an ice bath, 23 mL of 10% aqueous sodium sulfite was added and the volatiles removed under vacuum. The residue was acidified with 12N aqueous HCl, extracted with ethyl acetate, dried and concentrated to afford 1.23g of 2,2-dimethyl-3-(phenylsulfonyl)propionic acid, m/e 260 (M + NH₄ ⁺).

### B. Compounds of the Invention

### I. Preparation of Butanediamide. N¹-[3-[[[(1'1-dimethyl-2-phenylethyl)amino]carbonyl](3-methylbutyl)amino]-2 -hydroxy-1-(phenylmethyl)propyl]-2-[(2 -guinolinylcarbonyl)amino]-, [1S-[1R*(R*), 2S*]]-

To a solution of 72 mg (0.40 mmol) of 2,2-dimethyl-3-phenylpropionic acid in 5 mL of toluene and 0.12g (1.2 mmol) of triethylamine at 90°C, was added 0.llg (0.40 mmol) of diphenylphosphoryl azide. After one hour, a solution of 208 mg (0.40 mmol) of N-3(S)-[N-(2-quinolinylcarbonyl)-L-asparaginyl]amino-2(R)-hydroxy-4-phenylbutylamine, N-(3-methylbutyl) in 1.5 mL of N,N-dimethylformamide was added. After one hour, the solvent was removed under reduced pressure, ethyl acetate added, washed with water, sat d sodium bicarbonate, sat d sodium chloride, dried, filtered and concentrated to afford 200 mg of crude product. This was recrystallized from ethyl acetate and hexane to afford 42 mg of the desired product, m/e 701 (M + Li).

### II. Preparation of Butanediamide, N¹-[3-[[[(1,1-dimethyl-2-(4-pyridyl)ethyl)amino]carbonyl](3-(4 -fluorophenyl)methyl)amino]-2-hydroxy-1-(phenylmethyl)propyl-2-[(2-quinolinylcarbonyl)amino],-[1S-[1R*(R*), 2S*]]-

To a solution of 96 mg (0.54 mmol) of 2,2-dimethyl-3-(4-pyridyl)propionic acid in 5 mL of toluene and 0.16g (1.62 mmol) of triethylamine at 95°C, was added 149 mg (0.54 mmol) of diphenylphosphoryl azide. After one hour, a solution of 300 mg (0.54 mmol) of N-3(S)-[N-(2-quinolinylcarbonyhl)-L-asparaginyl]amino-2(R)-hydroxy-4-phenylbutylamine, N-(4-fluorophenyl)-methyl in 2 mL of N,N-dimethylformamide was added.. After one hour, the solvents were removed under reduced pressure, ethyl acetate added, washed with water, sat d sodium bicarbonate, brine, dried, filtered and concentrated to afford 320 mg of crude product. Chromatography on silica gel using 5-30% isopropanol/methylene chloride afforded 60 mg of the desired product, m/e 734 (M + H).

### III. Preparation of Butanediamide. N -[3-[[[(1,1-dimethy-2-hydroxyethyl)amino]carbonyl](4-fluoro phenylmethyl)amino]-2-hydroxy-1-(phenylmethyl)propyl] -2-[(2-quinolinylcarbonyl)amino] -[1S-(1R* (R*). 2S*]]-

To a solution of mono-tertiary-butyl 2,2-dimethylmalonate (188 mg, 1.0 mmol), Et₃N (303 mg, 3.0 mmol) in toluene (2 ml) was added DPPA (283 mg, 1.0 mmol) in toluene (0.5 ml) dropwise over 5 min at 95° (oil bath). After stirring at 95°C (oil bath) for another 45 min, the mixture was cooled to r.t. and to this was added a solution of free amine (588 mg, 1.0 mmol) in DMF (5 ml) and stirred at r.t. for 45 min. The mixture diluted with EtOAc (25 ml) and washed with 5% NaHCO₃ (10 ml x 2), 5% citric acid (5 ml) and H₂O (10 ml) then sat. NaCl (10 ml). The organic phase was dried (Na₂SO₄) and concentrated to afford 840 mg crude product. Purification of crude product by flash chromatography (silica gel, 3% then 5% MeOH/CH₂Cl₂) afforded 568 mg (76%) of pure desired product as a white solid, m/e 749 (M + Li). This white solid (520 mg, 0.699 mmol) was dissolved in CH₂Cl₂ (3 ml) and to this was added TFA (1.5 ml). After the mixture was stirred at r.t. for 16 h, the solvents were removed in vacuo to afford 475 mg (98%) of acid as a white solid. This white solid (450 mg, 0.65 mmol) was dissolved in THF (3 ml) and cooled to 0°C. To this cold solution was added BH₃·Me₂S (0.3 ml of 10 M solution, 3 mmol) dropwise via a syringe. After the mixture was stirred at 0°C for another 1 h and at r.t. for 16 h, it was quenched with MeOH (1 ml). The solvents were removed in vacuo and MeOH (2 ml) was added and stripped off again. This procedure was repeated for 3 more times. A white solid was obtained. Purification of the crude product by flash chromatography (silica gel, 10% MeOH/.CH₂Cl₂) gave 108 mg (25%) of pure alcohol as a white solid, m/e 679 (M + Li).

### IV. Preparation of Butanediamide, N -[3-[[[3-(3,3-dimethylpronionic acid, dimethyl amide)amino]-carbonyl](4-fluorophenylmethyl)amino]-2-hydroxy-1 -phenylmethyl)propyl]-2-[(2-quinolinylcarbonyl)amino]-, [1S-[1R*(R*), 2S*]]-

### Part A. Preparation of 2,2-Dimethylsuccinic acid, 4-(mono-para-methoxybenzyl ester).

A 250 ml RB flask equipped with magnetic stir bar, reflux condensor and N₂ inlet was charged with 5.0g (39 mmol) of 2,2-dimethyl succinic anhydride, 5.39g (39 mmol) of p-methoxy benzyl alcohol (MOS-OH) in 65 ml toluene. After overnight reflux the reaction mixture was concentrated in vacuo and triturated with hexane to yield 9.1g crude solid: 6:1 mixture of regioisomers. After washing with hexane the crude solid was recrystallized from 85 ml boiling hexane to yield 5.9g (57%) of white solid. Regioisomeric purity was >25:1 by 400 MHz ¹H-NMR.

### Part B. Preparation of Butanediamide, N -[3-[[[3-(3,3-dimethyl propionic acid, 4-methoxy-phenylmethyl ester)amino]carbonyl](4-fluorophenymethyl)amino]-2-hydroxy-1-(phenylmethyl)propyl]-2-[(2-quinolinylcarbonyl)amino]-[1S-[1R*(R*), 2S*]]-.

A 100 ml RB flask equipped with magnetic stir bar, reflux condensor and N₂ inlet was charged with 120 mg (.45 mmol) of product from Part A, 189 µl (1.35 mmol) NEt₃ in 5 ml dry toluene. The reaction was stirred at 95°C while 125 ml (.45 mmol) DPPA was slowly added. After 1 hour the free amine A : 250 mg (.45 mmol), predissolved in 4 ml DMF, was added. After 1 hour the reaction was concentrated in vacuo and partioned between EA and 5% aq citric acid. Organics washed with H₂O, sat bicarb, brine and dried over Na₂SO₄. Concentration in vacuo yielded 440 mg crude foam. Flash chromatography (100% EA) yielded 270 mg (73%) solid. Pure by ¹H-NMR and FAB mass spec (M + H = 822 ).

### Part C. Preparation of Butanediamide, N -[3-[[[3-(3,3-dimethylpropionic acid)amino]carbonyl]-(4-fluorophenylmethyl)amino]-2-hydroxy-1-(phenylmethyl)propyl]-2-[(2-quinolinylcarbonyl)-amino]-,[1S-[1R*(R*), 2S*]]-.

A 100 liter RB flask equipped with magnetic stir bar and N₂ inlet was charged with 260 mg (.32 mmol) 2 in 20 ml 4N HCl/dioxane. The homogeneous solution was stirred at RT 30 min then to 50°C for 30 min. The reaction mixture was concentrated in vacuo to yield an oily solid that was titurated from excess Et₂O, filtered and dried to yield 150 mg (68%) of white powder suitable for use without further purification. FAB mass spec gave gave M + H = 702, M + Li = 708.

### Part D. Preparation of Butanediamide, N -[3-[[[3-(3,3-dimethylpropionic acid, dimethyl amide)amino]-carbonyl](4-fluorophenylmethyl)amino]-2-hydroxy-1-(phenylmethyl)propyl]-2-[(2-quinolinylcarbonyl)amino]-, [1S-(1R*(R*), 2S*]]-.

A 25 ml RB flask equipped with magnetic stir bar and N₂ inlet was charged with 50 mg (.07 mmol) acid in 1 ml DMF. The reaction was cooled to 0°C when 15 mg HOBt (.11 mmol) was added followed by 15 mg (.08 mmol) EDCI. After stirring 20 minutes 50 ml 40% aq. dimethyl amine was added. The reaction was stirred 2 hours @ 0°C and allowed to stir at room temperature overnight. The reaction was taken up in EA and washed with 2x20 µl sat. bicarb, 2x20 ml 5% aq citric acid, 1x30 ml brine, and over MgSO₄. Concentration in vacuo yield 21 mg crude solid TLC (5% MeOH-CH₂Cl₂). Showed 50% conversion to product with other impurities. Flash chromatography (MeOH-CH₂Cl₂) yield 6.5 mg product (13%). Mass spec M + H = 728.

### Preparation of Butanediamide. N -[3-[[[(1,1-dimethyl-2-oxo-propyl)amino]carbonyl](2-methylbutyl)amino]-2 -hydroxy-1-(phenylmethyl)propyl]-2-[(2 -quinolinylcarbonyl)amino]-, [1S-(1R* (R*), 2S*]]-

### Part A. Preparation of Methyl 2,2-Dimethyl-3-oxobutyric Acid.

A 250 ml RB flask equipped with magnetic stir bar, addition funnel and N₂ inlet was charged with 8.9g of 95% NaH (372 mmol, 2.2 eq) and 125 ml dry THF. The slurry was cooled to -30°C when 20g (170 mmol) of methyl acetoacetate was slowly added. After 5 min 51g (359 mmol, 2.1 eq) of methyl iodide was added and the reaction was stirred 1 hour at -25°C and allowed to sit at room temperature overnight. Upon workup the reaction was diluted with 125 ml EA and the precipated sodium iodide was filtered and the filtrate was washed with 100 ml sat. bicarb, 100 ml 5% aq Na₂S₂O₅ and 100 ml brine. The organics were dried and concentrated in vacuo to yield 19g of a yellow oil suitable for use without further purification.

### Part B. Synthesis of 2,2-Dimethyl-3-oxobutyric acid, ethylene glycol Keml Dicyclohexyl-ammonium Salt.

A 100 ml RB flask equipped with magnetic stir bar and N₂ inlet was charged with 5g (34.7 mmol) 2,2-dimethyl methylacetoacetate in 25 ml anhydrous ethylene glycol, 20 ml trimethylorthoformate with a catalytic amount of p-toluenesulfonic acid. The reaction mixture was stirred @ 55°C overnight then worked up by pouring into 200 ml 20% aq KOH and heating to 95°C for 4 hours. The cooled reaction mixture was extracted with ether and the aqueous phase was acidified to pH 3 with 35 ml conc HCl/ice. The product was extracted with 2x100 ml EA. The organics were dried over Na₂SO₄ and concentrated in vacuo to ∼ 5g crude free acid.

The acid was taken up in 50 ml ether and 5.3g dicyclohexyl amine salt was added and the product was filtered and dried to yield 6.5g (∼ 53%) white solid. Mp 124-127°C.

### Part C. Preparation of N³-3(S)-(Benzyloxycarbonyl)amino-2-(R)-hydroxy-4-pentylbutyl amine, N -(3-methyl)butyl, N -[[(1,1-dimethyl-2-oxopropyl)amino]carbonyl, ethylene glycol ketal].

A 250 ml RBF equipped with magnetic stir bar and N₂ inlet was charged with 2.0g (11.5 mmol) free acid: free acid liberated from DCHA salt by partioning between Et₂O and 5% aq KHSO₄, 6.11 ml (43.7 mmol - 3.8 eq) NEt₃ in 75 ml dry toluene. The solution was heated to 95°C and 3.16g (11.7 mmol) DPPA was added. The reaction was stirred at 95°C for 1 hour when 4g (11.7 mmol) A in 50 ml toluene was added. The reaction was stirred at 90°C for 1 hour than at room temperature overnight. The reaction was concentrated in vacuo and partionned between EA and 5% aq citric acid. The organic phase was washed with sat. bicarb, brine, dried, and concentrated to 6g of crude foam. The product was purified by flash chromatography on silica gel to yield 4.3g (73%) white foam, pure by TLC and high field NMR. FAB mass spec, M + Li = 562 z/m.

### Part D. Preparation of butanediamide, N -[3-[[[(1,1-dimethyl-2-oxo-propyl)amino]carbonyl](2-methylbutyl)amino]-2-hydroxy-1-(phenylmethyl)propyl]-2-[(2-quinolinylcarbonyl)amino]-, [1S-[1R*(R*), 2S*]]-.

A Fisher Porter tube equipped with magnetic stir bar was charged with 1.5g of product from Part C, 45 mL of methanol and a catalytic amount of 10% Pd-C. The mixture was hydrogenated at 45 psi for 20 hours. The reaction was filtered through celite and concentrated in vacuo to yield 1.1g (>95%) of free amine, which was used without further purification.
A 100 ml RBF equipped with magnetic stir bar and N₂ inlet was charged with 824 mg (3.14 mmol, 1.15 eq) Z-asparagine in 100 ml DMF. The solution was cooled to 0°C and 550 mg (4.07 mmol, 1.5 eq) HOBt was added followed by 60 mg (3.13 mmol, 1.15 eq) EDCI. The reaction was stirred 10 minutes when 1.13g (2.7 mmol) crude free amine was added. The reaction was stirred 1 hour at 0°C then overnight at room temperature. The reaction was poured into 100 ml sat. bicarb and extracted with 100 ml EA. The organics were washed with 5% citric acid, brine, dried over Na₂SO₄, and concentrated in vacuo to yield 1.37g (75%) white solid which was identified as the CBZ-asparagine adduct. FAB mass spec gave M + Li 676 z/m.

A Fisher Porter tube equipped with magnetic stir bar was charged with 1.37g of CBZ-asparagine adduct, 50 mL MeOH and a catalytic amount of 10% Pd-C. The reaction mixture was hydrogenated at 50 psi for 16 hours. The reaction was filtered through celite and concentrated in vacuo to yield 1.05g (97%) of free asparagine amine as a foam that was used without further purification. FAB mass spec gave M + Li = 542.

A 100 ml RBF equipped with magnetic stir bar and N₂ inlet was charged with 356 mg (2.06 mmol, 1 eq) 2-quinaldic acid in 10 ml dry DMF. The solution was cooled to 0°C and 415 mg (3.07 mmol, 1.5 eq) HOBt was added followed by 415 mg EDCI (2.16 mmol, 1.05 eq). The reaction was stirred 2 hours at 0°C when 1.1g (2.06 mmol, 1 eq) of free asparagine amine in 10 ml dry DMF was added. The reaction was stirred at 0°C for 2 hours then room temperature overnight. The reaction was poured into sat. bicarb and extracted with 2x65 mL ethyl acetate. The combined organics were washed with 5% aq. citric acid, brine, dried and concentrated to 1.4g crude foam. Purification by flash chromatography on silica gel (100% EA) yielded 850 mg (61%) of quinoline adduct.

A 100 ml RBF equipped with magnetic stir bar was charged with 117 mg quinoline Ketal, 30 ml of THF and 15 ml 30% aq HCl. The reaction was stirred 2 hours at room temperature then diluted with 50 ml EA. The organic phase was washed with sat. bicarb, brine, dried and concentrated in vacuo to 105 mg pure ketone. FAB mass spec gave M + Li at 653 z/mol.

### Preparation of Butanediamide, N -[3-[[[(1,1-dimethyl-2-oxo-propyl)amino]carbonyl](2-methylbutyl)amino]-2 -hydroxy-1-(phenylmethyl)propyl]-2-[(2 -quinolinylcarbonyl)amino]-, [1S-[1R*(R*), 2S*1]-.

To a solution of 0.22g of 2(S)-methyl-3-(methylsulfonyl)propionic acid, 0.29g of N-hydroxybenzotriazole in 5 mL of N,N-dimethylformamide at 0°C was added 0.31g of EDCI. After 30 minutes, a solution of 3-[[[1,1-dimethyl-2-(4-pyridyl)ethyl]amino]carbonyl](3-methylbutyl)amino-2-(R)-hydroxy-1(S)-(phenylmethyl)propyl amine in 2 mL N,N-dimethylformamide was added and the solution stirred for 17 hours at room temperature, poured into saturated aqueous bicarbonate, chilled and filtered, and the solids washed with aqueous bicarbonate and water. The resulting solids were dissolved in methyhlene chloride, washed with aqueous bicarbonate, brine, dried, filtered and concentrated to afford 160 mg of the desired product.

### Preparation of butanediamide, N -[3-[[[[1,1-dimethyl-2-(4-morpholinyl)ethyl]amino]carbonyl](3 -methylbutyl)amino]-2-hydroxy-1-(phenylmethyl)propyl]-2 -[(2-quinolinylcarbonyl)amino]-, [1S-[1R*(R*), 2S*]-.

Dissolve 90.40 mg (0.4 mmoles, 1.05 eq.) amino acid (3) in toluene. Heat to 95 degrees C. under N₂. Add 225µl (1.61mmoles, 4.2 eq.) triethylamine.

Slowly add 87.1µl (0.4 mmoles, 1.05eq.) diphenylphosphoryl azide. Stir 1 hour. To this solution add 200 mg (0.38 mmoles, 1 eq.) 3(S)-[N-(2-quinolinylcarbonyl)-L-asparaginyl]amino-2(R)-hydroxy-4-phenylbutyl amine, N-(3-methylbutyl) dissolved in DMF. Stir 1 hour. Cool to room temperature. Strip off toluene. Dissolve in EtOAc. Wash 1xH₂O, 1x saturated NaHCO₃, 1x saturated NaCl. Dry with MgSO₄ and rotovap. Purify by silica flash chromatography (30:1 CH₂Cl₂:CH₃OH) Yield=50% M+H=704

### Preparation of butanediamide, N -[3-[[[[1,1-dimethyl-3-(4-(1-methylpiperazinyl))propyl]amino]carbonyl](3 -methylbutyl)amino]-2-hydroxy-1-(phenylmethyl)propyl]-2 -[(2-quinolinylcarbonyl)amino]-, [1S-[1R*(R*), 2S*]-.

Dissolve 122.2 mg (0.57 mmoles, 1.5 eq.) amino acid of 2,2-dimethyl-4-(1-(4-methylpiperazinyl))butanoic acid in toluene. Heat to 95 degrees C. under N₂. Add 238µl (1.71mmoles, 4.5 eq.) triethylamine. Slowly add 122.8µl (0.57 mmoles, 1.5eq.) diphenylphosphoryl azide. Stir 1 hour. To this solution add 200 mg (0.38 mmoles, 1 eq.) 3(S)-[N (2-quinolinylcarbonyl)-L-asparaginyl]amino-2(R)-hydroxy-4-phenylbutyl amine, N-(3-methylbutyl) dissolved in DMF. Stir 1 hour. Cool to room temperature. Strip off toluene. Dissolve in EtOAc. Wash 1xH₂O, 1x saturated NaHCO₃, 1x saturated NaCl. Dry with MgSO₄ and rotovap. Purify by recrystalizing in EtOAc/pet. ether. Yield=66% M+Li=737

### Preparation of butanediamide, N-[3-[[[[(1,1-dimethyl-5-(4-morpholinyl)-3-oxapentyl]-amino]carbonyl](3 -methylbutyl)amino]-2-hydroxy-1-(phenylmethyl)propyl]-2 -[(2-quinolinyl-carbonyl)amino]-, [1S-[1R*(R*), 2S*]-.

To a mixture of acid 10 (231 mg, 1.0 mmol) EtN₃ (303 mg, 3.0 mmol) in toluene (2 ml) was added DPPA (283 mg, 1.0 mmol) in toluene (0.5 ml) dropwise over 5 min at 95°C (oil bath). After stirring at 95°C (oil bath) for another 45 min, the mixture was cooled to room temperature and to this was added a solution of 3(S)-[N-(2-quinolinylcarbonyl)-L-asparaginyl]amino-2(R)-hydroxy-4-phenylbutyl amine, N- (3-methylbutyl) (519.6 mg, 1.0 mmol) in DMF (5 ml) and stirred at room temperature for 45 min. The mixture was diluted with EtOAc (25 ml) and washed with 5% NaHCO₃ (10 ml x 2), 5% citric acid (5 ml and H₂O (10 ml) then sat. NaCl solution (10 ml). The combined extracts were dried (Na₂SO₄) and concentrated to give a solid. The purification of the crude product by flash chromatography (silica gel, 3% MeOH/CH₂Cl₂) gave 262 mg (35%) pure product.

The above procedures could be utilized to prepare compounds of the present invention wherein the group B as defined above is incorporated into the compound shown below in Examples 6-36. It is contemplated that the resulting compounds would inhibit retroviral proteases with activities similar to the activities of the compounds of Examples 1-5. Thus, a compound of the formula 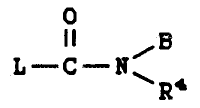 can be utilized in place of the isocyanate of Step D of Example 1. Alternatively, the compounds represented by the formula: 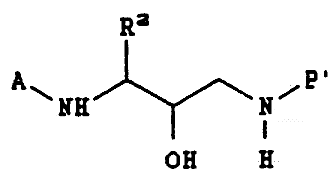 can be prepared and, following deprotection, can be reacted with the above compound as in Example 5.

### Example 6A

### Preparation of [1S-[1R*(R*), 2S*]]-N¹[3-[[[(1,1-dimethylethyl)amino]carbonyl](2-methylpropyl)amino]-2 -hydroxy-1-(phenylmethyl)propyl]-2-[(2 -quinolinylcarbonyl)amino]-butanediamide

### Part A:

To a solution of 75.0g (0.226 mol) of N-benzyloxycarbonyl-L-phenylalanine chloromethyl ketone in a mixture of 807 mL of methanol and 807 mL of tetrahydrofuran at -2°C, was added 13.17g (0.348 mol, 1.54 equiv.) of solid sodium borohydride over one hundred minutes. The solvents were removed under reduced pressure at 40°C and the residue dissolved in ethyl acetate (approx. 1L). The solution was washed sequentially with 1M potassium hydrogen sulfate, saturated sodium bicarbonate and then saturated sodium chloride solutions. After drying over anhydrous magnesium sulfate and filtering, the solution was removed under reduced pressure. To the resulting oil was added hexane (approx. 1L) and the mixture warmed to 60°C with swirling. After cooling to room temperature, the solids were collected and washed with 2L of hexane. The resulting solid was recrystallized from hot ethyl acetate and hexane to afford 32.3g (43% yield) of N-benzyloxycarbonyl-3(S)-amino-1-chloro-4-phenyl-2(S)-butanol, mp 150-151°C and M+Li⁺ = 340.

### Part B:

To a solution of 6.52g (0.116 mol, 1.2 equiv.) of potassium hydroxide in 968 mL of absolute ethanol at room temperature, was added 32.3g (0.097 mol) of N-CBZ-3(S)-amino-1-chloro-4-phenyl-2(S)-butanol. After stirring for fifteen minutes, the solvent was removed under reduced pressure and the solids dissolved in methylene chloride. After washing with water, drying over magnesium sulfate, filtering and stripping, one obtains 27.9g of a white solid. Recrystallization from hot ethyl acetate and hexane afforded 22.3g (77% yield) of N-benzyloxycarbonyl-3(S)-amino-1,2(S)-epoxy-4-phenylbutane, mp 102-103°C and MH⁺ 298.

### Part C:

A solution of N-benzyloxycarbonyl 3(S)-amino-1,2-(S)-epoxy-4-phenylbutane (1.00g, 3.36 mmol) and isobutylamine (4.90g, 67.2 mmol, 20 equiv.) in 10 mL of isopropyl alcohol was heated to reflux for 1.5 hours. The solution was cooled to room temperature, concentrated in vacuo and then poured into 100 mL of stirring hexane whereupon the product crystallized from solution. The product was isolated by filtration and air dried to give 1.18g, 95% of N=[[3(S)-phenylmethylcarbamoyl)amino-2(R)-hydroxy-4-phenylbutyl]N-[(2-methylpropyl)]amine mp 108.0-109.5°C, MH⁺ m/z = 371.

### Part D:

A solution of [2(R), 3(S)]-N-[[3-(phenylmethylcarbamoyl)amino]-2-hydroxy-4-phenylbutyl]N-[(2-methylpropyl)]amine in 10 ml of tetrahydrofuran was treated with tert-butylisocyanate (267 mg, 2.70 mmol) at room temperature for 5 minutes. The solvent was removed in vacuo and replaced with ethyl acetate. The ethyl acetate solution was washed with 5% citric acid, water, and brine, dried over anhydrous MgSO₄, filtered and concentrated in vacuo to give 1.19g, 97% of [2(R), 3(S)]-N-[[3-(phenylmethylcarbamoyl)amino]-2-hydroxy-4-phenyl]-1-(2-methylpropyl)]amino-2-(1,1-dimethyl)amino]carbonyl]butane, MH⁺ m/z - 470.

### Part E:

A solution of (1.00g, 2.21 mmol) [2(R), 3(S)]-N-[[3-(phenylmethylcarbamoyl)amino]-2-hydroxy-4-phenyl]-1-[(2-methylpropyl)]amino-1-(1,1-dimethylethyl)amino]carbonyl]butane in 20 mL of methanol was hydrogenated over 10% palladium-on-carbon for 4 hours to give [2(R), 3(S)]-N-[[3-amino]-2-hydroxy-4-phenyl]-1-[(2-methylpropyl)amino-1-(1,1-dimethylethyl)amino]carbonyl]butane 720 mg, 97%.

### Part F:

A solution of N-Cbz-L-asparagine (602mg, 2.26 mmol) and N-hydroxybenzotriazole (493 mg, 3.22 mmol) in 2mL of dimethylformamide was cooled to 0°C and treated with EDC (473 mg, 2.47 mmol). The solution was allowed to stir at 0°C for 20 minutes and then treated with [2(R), 3(S)]-N-[[3-amino]-2-hydroxy-4-phenyl]-1-[(2-methylpropyl)]amino-1-(1,1-dimethylethyl)amino]carbonyl]butane (720 mg, 2.15 mmol) in 1mL of dimethylformamide. The solution was allowed to warm to room temperature and held at this temperature for 7 hours. The reaction mixture was then poured into 100 mL of 60% saturated aqueous sodium bicarbonate whereupon a white precipitate formed that was isolated by filtration. The filter cake was washed with water, 5% aqueous citric acid, water and then dried in vacuo to give 1.04g, 83% of [1S-[1R*(R*), 2S*]]- N¹[3-[[[(1,1-dimethylethyl)amino]carbonyl](2-methylpropyl)amino], mp. 164.0-166.5°C, MH⁺ m/z = 584.

### Part G:

A solution of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-[(phenylmethylcarbamoyl)amino]-butanediamide (1.00g, 1.72 mmol) in 10 mL of methanol was hydrogenated over 10% palladium-on-carbon for 4 hours to give [1S-[1R*(R*), 2S*]]- N¹[3-[[[(1,1-dimethylethyl)amino]carbonyl](2-methylpropyl)amino]-2-hydroxy-1-(phenylmethyl)propyl]-2-amino]-butanediamide, 784mg, 99%.

### Part H:

A mixture of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-amino]-butanediamide, (784 mg, 1.70 mmol), 2-quinoline carboxylic acid N-hydroxysuccinimide ester (459 mg, 1.70 mmol), N-methylmorpholine (343 mg, 3.40 mmol) in 5 mL of dichloromethane was stirred at room temperature for 15 minutes. The solvent was removed in vacuo and replaced with ethyl acetate and the solution washed with 5% aqueous citric acid, saturated aqueous sodium bicarbonate, brine, dried over anhydrous MgSO₄, filtered and concentrated in vacuo. The crude product was recrystallized from acetone/hexane to give 790 mg, 77% of [1S-[1R*(R*), 2S*]]- N¹[3-[[[(1,1-dimethylethyl)amino]carbonyl](2-methylpropyl)amino]-2-hydroxy-1-(phenylmethyl)propyl]-2-[(2-quinolinylcarbonyl)amino]-butanediamide, mp 107.0-109.8°C, MH⁺ = 605.

### Example 6B

The procedure described in Example 1, part C-H, was used to prepare [1S-[1R*(R*), 2S*]]- N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide.
a) From the reaction of 1.06g (3.56mmol) of N-benzyloxycarbonyl 3(S)-amino-1,2-(S)-epoxy-4-phenylbutane and 6.25g (71.7mmol) of isoamylamine, one obtains 1.27g (92%) of [2(R), 3(S))-N-[(3-(phenylmethylcarbamoyl)amino]-2-hydroxy-4-phenylbutyl]N-[(3-methylbutyl)]amine, mp 130-132C and MH* 385. This amine (400mg, 1.04mmol) was then reacted with tert-butylisocyanate (110mg, 1.11mmol) to afford 500mg (100%) of [2(R), 3(S))-N-[[3-(phenylmethylcarbamoyl)amino]-2-hydroxy-4-phenyl]-1-[(3-methylbutyl)]amino-1-(1,1-dimethylethy)amino]carbonyl]butane; as an oil, MH⁺ 484.
b) The CBZ protected compound (530mg, 1.10mmol) was then deprotected by hydrogenation over 10% palladium-on-carbon and the resulting free amine coupled with N-CBZ-L-asparagine (377mg, 1.42mmol) in the presence of N-hydroxybenzotriazole (290mg, 2.15mmol) and EDC (300mg, 1.56mmol) to yield 430mg (53%) of [1S-[1R*(R*) , 2S*]]- N¹[3-[[[(1,1-dimethylethyl)amino]carbonyl](3-methylbutyl)amino]-2-hydroxy-1-(phenylmethyl)propyl]-2-[(phenylmethylcarbamoyl)amino]-butanediamide, mp 148-151 C (dec) and MH⁺ 598. This compound (370mg, 0.619mmol) was then deprotected by hydrogenation over 10% palladium-on-carbon and the resulting free amine coupled with 2-quinolinecarboxylic acid N-hydroxy-succinimide ester (193mg, 0.714mmol), in the presence of N-methylmorpholine, to afford 310mg (70%) of pure [1S-[1R*(R*), 2S*]]- N¹[3-[[[(1,1-dimethylethyl)amino]carbonyl](3-methylbutyl)amino]-2-hydroxy-1-(phenylmethyl)propyl]-2-[(2-quinolinylcarbonyl)amino]-butanediamide; mp 93.5-95.5C and MH⁺ 619.

### Example 6C

The procedure described in Example 1, part C-H, was used to prepare [1S-[1R*(R*), 2S*]]- N¹[3-[[[(1,1-dimethylethyl)amino]carbonyl]2-napthylmethyl)amino]-2-hydroxy-1-(phenylmethyl)propyl]-2-[(2-quinolinylcarbonyl)amio]-butanediamide.
a) From the reaction of 1.80g (6.05mmol) of N-benzyloxycarbonyl 3(S) -amino-1,2-(S)-epoxy-4-phenylbutane and 1.15g (7.31mmol) of 2-(aminomethyl)naphthalene, one obtains 2.11g (77%) of [2(R), 3(S)]-N-[[3-(phenylmethylcarbamoyl)amino]-2-hydroxy-4-phenylbutyl]N-[(2-napthylmethyl)]amine, MH⁺ 455. This amine (366.8mg, 0.807mmol) was then reacted with tert-butylisocyanate (66.4mg, 0.67mmol) to afford 350.0mg (94%) of [2(R), 3(S))-N-[[3-(phenylmethylcarbamoyl)amino]-2-hydroxy-4-phenyl]-1-[(2-napthylmethyl)]amino-1-(1,1-dimethylethyl)amino]carbonyl]butane; as an oil, MH⁺ 554.
b) The CBZ protected compound (330mg, 0.596mmol) was then deprotected by hydrogenation over 10% palladium-on-carbon and the resulting free amine coupled with N-CBZ-L-asparagine (165.1mg, 0.62mmol) in the presence of N-hydroxybenzotriazole (142.3mg, 0.93mmol) and EDC (130.7mg, 0.68mmol) to yield 161.7mg (41%) of [1S-[1R*(R*), 2S*]]- N¹[3-[[[(1,1-dimethylethyl)amino]carbonyl](2-napthylmethyl)amino]-2-hydroxy-1-(phenylmethyl)propyl]-2-[(phenylmethylcarbamoyl)amino]-butanediamide; mp 151-152 C (dec) and MH⁺ 668. This compound (91.0mg, 0.136mmol) was then deprotected by hydrogenation over 10% palladium-on-carbon and the resulting free amine coupled with 2-quinolinecarboxylic acid N-hydroxysuccinimide ester (36.8mg, 0.136mmol), in the presence of N-methylmorpholine, to afford 65.8mg (70%) of pure [1S-[1R*(R*), 2S*]]- N¹[3-[[[(1,1-dimethylethyl)amino]carbonyl](2-napthylmethyl)amino]-2-hydroxy-1-(phenylmethyl)propyl]-2-[(2-quinolinylcarbonyl)amino]-butanediamide; mp 119-120C and MH⁺ 689.

### Example 6D

The procedure described in Example 1, part C-H, was used to prepare [1S-(1R*(R*), 2S*]]- N¹[3-[[[(1,1-dimethylethyl)amino]carbonyl](2-phenylethyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 8.19g (67.0mmol) of 2-phenethyl amine, one obtains 1.10g (79%) of [2(R), 3(S)]-N-[[3-(phenylmethylcarbamoyl)amino]-2-hydroxy-4-phenylbutyl]N-[(2-phenylethyl)]amine, mp 137-138 C and MH⁺ 419. This amine (750mg, 1.79mmol) was then reacted with tert-butylisocyanate (178mg, 1.79mmol) to afford 897mg = (97%) of [2(R), 3(S)]-N-[[3-(phenylmethylcarbamoyl)amino]-2-hydroxy-4-phenyl]-1-[(2-phenylethyl)]amino-1-(1,1-dimethylethyl)amino]carbonyl]butane; as an oil, MH⁺ 518.
b) The CBZ protected compound (897mg, 1.73mmol) was then deprotected by hydrogenation over 10% palladium-on-carbon and the resulting free amine coupled with N-CBZ-L-asparagine (620.7mg, 2.33mmol) in the presence of N-hydroxybenzotriazole (509.5mg, 3.33mmol) and EDC (488.0mg, 2.55mmol) to yield 1.00g (92%) of [1S-[1R*(R*), 2S*]]- N¹[3[[[(1,1-dimethylethyl)amino]carbonyl](2-phenylethyl)amino]-2-hydroxy-1-(phenylmethyl)propyl]-2-[(phenylmethylcarbamoyl)amino]-butanediamide; mp 145 (dec) and MH⁺ 632. This compound (860mg, 1.36mmol) was then deprotected by hydrogenation over 10% palladium-on-carbon and the resulting free amine coupled with 2-quinolinecarboxylic acid N-hydroxysuccinimide ester (338mg, 1.25mmol), in the presence of N-methylmorpholine, to afford 450.4mg (55%) of pure [1S-[1R*(R*), 2S*]]- N¹[3[[[(1,1-dimethylethyl)amino]carbonyl](2-phenylethyl)amino]-2-hydroxy-1-(phenylmethyl)propyl]-2-[(2-quinolinylcarbonyl)amino]-butanediamide; mp 139-140°C and MH⁺ 653.

### Example 6E

The procedure described in Example 1, part C-H, was used to prepare [1S-[1R*(R*), 2S*]]- N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide.
a) From the reaction of 1.00g (3.36mmol) of N-benzyloxycarbonyl 3(S)-amino-1,2-(S)-epoxy-4-phenylbutane and 7.9mL (approx. 67mmol) of neopentyl amine, one obtains 0.69g (49%) of [2(R), 3(S)]-N-[[3-(phenylmethylcarbamoyl)amino]-2-hydroxy-4-phenylbutyl]N-[(2,2-dimethylpropyl)]amine, MH⁺ 385. This amine (686mg, 1.78mmol) was then reacted with tert-butylisocyanate (180mg, 1.78mmol) to afford 860mg (100%) of [2(R), 3(S))-N-[[3-(phenylmethylcarbamoyl)amino]-2-hydroxy-4-phenyl]-1-[(2,2-dimethylpropyl)]amino-1-(1,1-dimethylethyl)amino]carbonyl]butane; MH⁺ 484.
b) The CBZ protected compound (860mg, 1.78mmol) was then deprotected by hydrogenation over 10% palladium-on-carbon and the resulting free amine coupled with N-CBZ-L-asparagine (471mg, 1.77mmol) in the presence of N-hydroxybenzotriazole (406mg, 2.66mmol) and EDC (374mg, 1.95mmol) to yield 326mg (34%) of [1S-[R*(R*), 2S*]]- N¹[3-[[[(1,1-dimethylethyl)amino]carbonyl](2,2-dimethylpropyl)amino]-2-hydroxy-1-(phenylmethyl)propyl]-2-[(phenylmethylcarbamoyl)amino]-butanediamide; mp 177-178C and MH⁺ 598. This compound (245mg, 0.41mmol) was then deprotected by hydrogenation over 10% palladium-on-carbon and the resulting free amine coupled with 2-quinolinecarboxylic acid N-hydroxysuccinimide ester (111mg, 0.41mmol), in the presence of N-methylmorpholine, to afford 150mg (59%) of pure [1S-[R*(R*), 2S*]]- N¹[3-[[[(1,1-dimethylethyl)amino]carbonyl](2,2-dimethylpropyl)amino]-2-hydroxy-1-(phenylmethyl)propyl]-2-[(2-quinolinylcarbonyl)amino]-butanediamide; mp 115-117C and MH⁺ 619.

The procedure described in Example 1, part C-H, was used to prepare [1S-[R*(R*), 2S*]]- N¹[3-[[[(1,1-dimethlethyl)amino]carbonyl](4-methoxyphenylmethyl)amino]-2-hydroxy-1-(phenylmethyl)propyl]-2-[(2-quinolinylcarbonyl)amino]-butanediamide;

### Example 6F

a) From the reaction of 1.00g (3.36mmol) of N-benzyloxycarbonyl 3 (S)-amino-1,2-(S)-epoxy-4-phenylbutane and 9.2g (67mmol) of 4-methoxybenzyl amine, one obtains 1.12g (76%) of [2(R), 3(S)]-N-[[3-(phenylmethylcarbamoyl)amino]-2-hydroxy-4-phenylbutyl]N-[(4-methoxyphenylmethyl)]amine, MH⁺ 435. This amine (1.12g, 2.58mmol) was then reacted with tert-butylisocyanate (260mg, 2.58mmol) to afford 1.35g (98%) of [2(R), 3(S)]-N-[[3-(phenylmethylcarbamoyl)amino]-2-hydroxy-4-phenyl]-1-[(4-methoxyphenylmethyl)]amino-1-(1,1-dimethylethyl)amino]carbonyl]butane; MH⁺ 534.
b) The CBZ protected compound (1.35g, 2.53mmol) was then deprotected by hydrogenation over 10% palladium-on-carbon and the resulting free amine coupled with N-CBZ-L-asparagine (684mg, 2.57mmol) in the presence of N-hydroxybenzotriazole (590mg, 3.85mmol) and EDC (543mg, 2.83mmo1) to yield 442mg (29%) of [1S-[1R*(R*), 2S*]]- N¹[3-[[[(1,1-dimethylethyl)amino]carbonyl](4-methoxyphenylmethyl)amino]-2-hydroxy-1-(phenylmethyl)propyl]-2-[phenylmethylcarbamoyl)amino]-butanediamide; mp 175C (dec) and MH⁺ 648. This compound (345mg, 0.53mmol) was then deprotected by hydrogenation over 10% palladium-on-carbon and the resulting free amine coupled with 2-quinolinecarboxylic acid N-hydroxy-succinimide ester (118mg, 0.44mmol), in the presence of N-methylmorpholine, to afford 108mg (31%) of pure [1S-[1R*(R*) , 2S*]]- N¹[3-[[[(1,1-dimethylethyl)amino]carbonyl](4-methoxyphenylmethyl)amino]-2-hydroxy-1-(phenylmethyl)propyl]-2-[(2-quinolinylcarbonyl)amino]-butanediamide; mp 220C (dec) and MLi⁺ 675.

### Example 7

The procedure described in Example 1, part C-H, was used to prepare [1S-[1R*(R*), 2S*]]- N¹[3-[[[(1,1-dimethylethyl)amino]carbonyl](n-butyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 7.314g (100.0mmol) of n-butyl amine, one obtains 1.50g (80%) of [2(R), 3(S)]-N-[[3-(phenylmethylcarbamoyl)amino]-2-hydroxy-4-phenylbutyl]N-[n-butyl)]amine. This amine (1.48g, 4.0mmol) was then reacted with tert-butylisocyanate (396mg, 4.0mmol) to afford 1.87g (100%) of [2(R), 3(S)]-N-[[3-(phenylmethylcarbamoyl)amino]-2-hydroxy-4-phenyl]-1-[(n-butyl)]amino-1-(1,1-dimethylethyl)amino]carbonyl] butane as an oil.
b) The CBZ protected compound (1.87g, 4.0mmol) was then deprotected by hydrogenation over 10% palladium-on-carbon and the resulting free amine coupled with N-CBZ-L-asparagine (1.05g, 3.96mmol) in the presence of N-hydroxybenzotriazole (535mg, 7.9mmol) and EDC (759mg, 3.96mmol) to yield 1.75g (76%) of [1S-[1R*(R*), 2S*]]- N¹[3-[[[(1,1-dimethylethyl)amino]carbonyl](n-butyl)amino]-2-hydroxy-1-(phenylmethyl)propyl]-2-[(phenylmethylcarbamoyl)amino]-butanediamide; mp 166-167C and MH⁺ 584.

A solution of[1S-[1R*(R*), 2S*]]- N¹[3-[[[(1,1-dimethylethyl)amino]carbonyl]n-butyl)amino]-2-hydroxy-1-(phenylmethy)propyl]-2-[(phenylmethylcarbamoyl)amino]-butanediamide (1.03g, 1.77 mmol) in 40 mL of abs. ethanol ws then deprotected by hydrogenolysis in the presence of 10% palladium on carbon catalyst to give, after filtration and concentration, the free amine (428mg) which was coupled with N-hydroxysuccinimide ester of 2-quinoline carboxylate (270mg, 1.0 mmol) in dichloromethane for 16 h to give 380 mg, 63% of [1S-[1R*(R*), 2S*]]- N¹[3-[[[1,1-dimethylethyl)amino]carbonyl](n-butyl)amino]-2-hydroxy-1-(phenylmethyl)propyl]-2-[(2-quinolinylcarbonyl)amino]-butanediamide, mp 102.1-103°C.

### Example 8

The procedure described in Example 1, part C-H, was used to prepare [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(2-quinolinylcarbonyl)amino]-butanediamide.
a) From the reaction of 1.48g (5.0mmol) of N-benzyloxycarbonyl 3(S)-amino-1,2-(S)-epoxy-4-phenylbutane and 10.68g (100.0mmol) of benzyl amine, one obtains 1.88g (95%) of [2(R), 3(S)]-N-[[3-(phenylmethylcarbamoyl)amino]-2-hydroxy-4-phenylbutyl]N-[(phenylmethyl)]amine. This amine (1.88g, 4.65mmol) was then reacted with tert-butylisocyanate (460.Omg, 4.6mmol) to afford 2.24g (96%) of [2(R), 3(S)]-N-[[3-(phenylmethylcarbamoyl)amino]-2-hydroxy-4-phenyl]-1-[(phenylmethyl)]amino-1-(1,1-dimethylethyl)amino]carbonyl] butane.
b) The CBZ protected compound (2.22g, 4.4mmol) was then deprotected by hydrogenation over 10% palladium-on-carbon and the resulting free amine coupled with N-CBZ-L-asparagine (1.17g, 4.4mmol) in the presence of N-hydroxybenzotriazole (1.19g, 8.8mmol) and EDC (843mg, 4.4mmol) to yield 2.11g (78%) of [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(phenylmethylcarbamoyl)amino]-butanediamide; mp 156-158C and MH⁺ 618. This compound (1.0g, 1.62mmol) was then deprotected by hydrogenation over 10% palladium-on-carbon and the resulting free amine coupled with 2-quinolinecarboxylic acid N-hydroxysuccinimide ester (437mg, 1.62mmol), in the presence of N-methylmorpholine, to afford 640mg (62%) of pure [1S-[1R*(R*), 2S*]]- N¹[3-[[[(1,1-dimethylethyl)amino]carbonyl](phenylmethyl)amino]-2-hydroxy-1-(phenylmethyl)propyl]-2-[(2-quinolinylcarbonyl)amino]-butanediamide; mp 110.5-112.5C and MH⁺ 639.

### EXAMPLE 9

Additional exemplary compounds of the present invention are listed in Table 1. These compounds were prepared according to the following general procedures.

### General Procedure for the Synthesis of 1,3-Diamino 4-phenyl Butan-2-ol Derivatives.

A mixture of the amine R³NH₂ (20 equiv.) in dry isopropyl alcohol (20mL/mmol of epoxide to be converted) was heated to reflux and then treated with an N-Cbz amino epoxide of the formula: 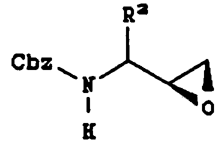 from a solids addition funnel over a 10-15 minute period. After the addition is complete the solution was maintained at reflux for an additional 15 minutes and the progress of the reaction monitored by TLC. In nearly all cases the reaction was found to be complete after this time period. The reaction mixture was then concentrated in vacuo to give an oil that was treated with n-hexane with rapid stirring whereupon the ring opened material precipitated from solution. Precipitation was generally complete within 1 hr and the product was then isolated by filtration on a Büchner funnel and then air dried. The product was further dried in vacuo. This method affords amino alcohols of sufficient purity for most purposes.

### General procedure for the Reaction of Amino Alcohols with Isocyanates: Preparation of Ureas

A solution from the amino alcohol in tetrahydrofuran (THF) was treated at room temperature with the appropriate isocyanate of formula R⁴NCO via syringe under nitrogen. After the reaction has stirred for ∼5m the progress of the reaction was monitored by TLC. In nearly all cases the reaction was complete. The solvent was removed in vacuo and the product obtained was of sufficient purity for most purposes. The product may be further purified by dissolution in ethyl acetate and washing with 5% aqueous citric acid, water, and brine. The solvent is dried over anhydrous magnesium sulfate, filtered and concentrated in vacuo to give the pure urea.

### General Procedure for the Removal of the Protecting Groups by Hydrogenolysis with Palladium on Carbon

### A. Alcohol Solvent

The Cbz-protected peptide derivative was dissolved in methanol (ca.20mL/mmol) and 10% palladium on carbon catalyst is added under a nitrogen atmosphere. The reaction vessel is sealed and flushed 5 times with nitrogen and then 5 times with hydrogen. The pressure is maintained at 50 psig for 1-16 hours and then the hydrogen replaced with nitrogen and the solution filtered through a pad of celite to remove the catalyst. The solvent is removed in vacuo to give the free amino derivative of suitable purity to be taken directly on to the next step.

### B. Acetic Acid Solvent

The Cbz-protected peptide derivative was dissolved in glacial acetic acid (20mL/mmol) and 10% palladium on carbon catalyst is added under a nitrogen atmosphere. The reaction vessel is flushed 5 times with nitrogen and 5 times with hydrogen and then maintained at 40 psig for about 2h. The hydrogen was then replaced with nitrogen and the reaction mixture filtered through a pad of celite to remove the catalyst. The filtrate was concentrated and the resulting product taken up in anhydrous ether and evaporated to dryness 3 times. The final product, the acetate salt, was dried in vacuo and is of suitable purity for subsequent conversion.

### General Procedure for Removal of Boc-protecting Group with 4N Hydrochloric Acid in Dioxane

The Boc-protected amino acid or peptide is treated with a solution of 4N HCl in dioxane with stirring at room temperature. Generally the deprotection reaction is complete within 15 minutes, the progress of the reaction is monitored by thin layer chromatography (TLC). Upon completion, the excess dioxane and HCl are removed by evaporation in vacuo. The last traces of dioxane and HCl are best removed by evaporation again from anhydrous ether or acetone. The hydrochloride salt thus obtained is thoroughly dried in vacuo and is suitable for further reaction.

### EDC/HOBt Coupling of Cbz-Asparagine (General Procedure)

N-CBZ-(L-asparagine (1.10eq) and N-hydroxybenzotriazole (HOBt) (1.5eq) are dissolved in dry dimethylformamide (DMF) (2-5mL/mmol) and cooled in an ice bath. 1-(3-dimethylaminopropyl)-3-ethylcarbodiimide hydrochloride (EDC) (1.10eq) is added to the stirring solution and maintained at 0°C for 10 minutes. A solution of the amino component (free amine), 1.Oeq in DMF (1-2mL/mmol), is added. [In the case of the amine hydrochloride or acetate salt, an equivalent of N-methylmorpholine is also added.] The reaction mixture is stirred at 0°C for 1 hour and then at room temperature for ∼5-6 hours. The reaction mixture is then poured into a rapidly stirring solution of 60% saturated aqueous sodium bicarbonate (ca-50mL/mmol). An immediate white precipitate forms which is collected on a Büchner funnel and the solid washed thoroughly with saturated aqueous sodium bicarbonate, water, 5% aqueous citric acid solution and water. The product is thoroughly dried in vacuo and redissolved in DMF, filtered and reprecipitated by the addition to water. The precipitated product is isolated by filtration, washed again with water and dried in vacuo.

### General Procedure for Acylation with 2-Quinoline Carboxylic Acid N-Hydroxysuccinimide Ester

A solution of the free amine (or amine acetate salt) and 1.0 equivalent of N-hydroxysuccinimide 2-quinoline carboxylate in anhydrous dichloromethane was treated with 1.5 equivalents of N-methylmorpholine (NMM) at room temperature. The progress of the reaction was monitored by TLC and when the reaction was complete the reaction mixture was diluted with additional dichloromethane and the solution washed with saturated aqueous sodium bicarbonate, 5% aqueous citric acid, water and brine. The solution was dried over anhydrous magnesium sulfate, filtered and concentrated in vacuo. The product thus obtained was recrystallized from a mixture of acetone and hexane.

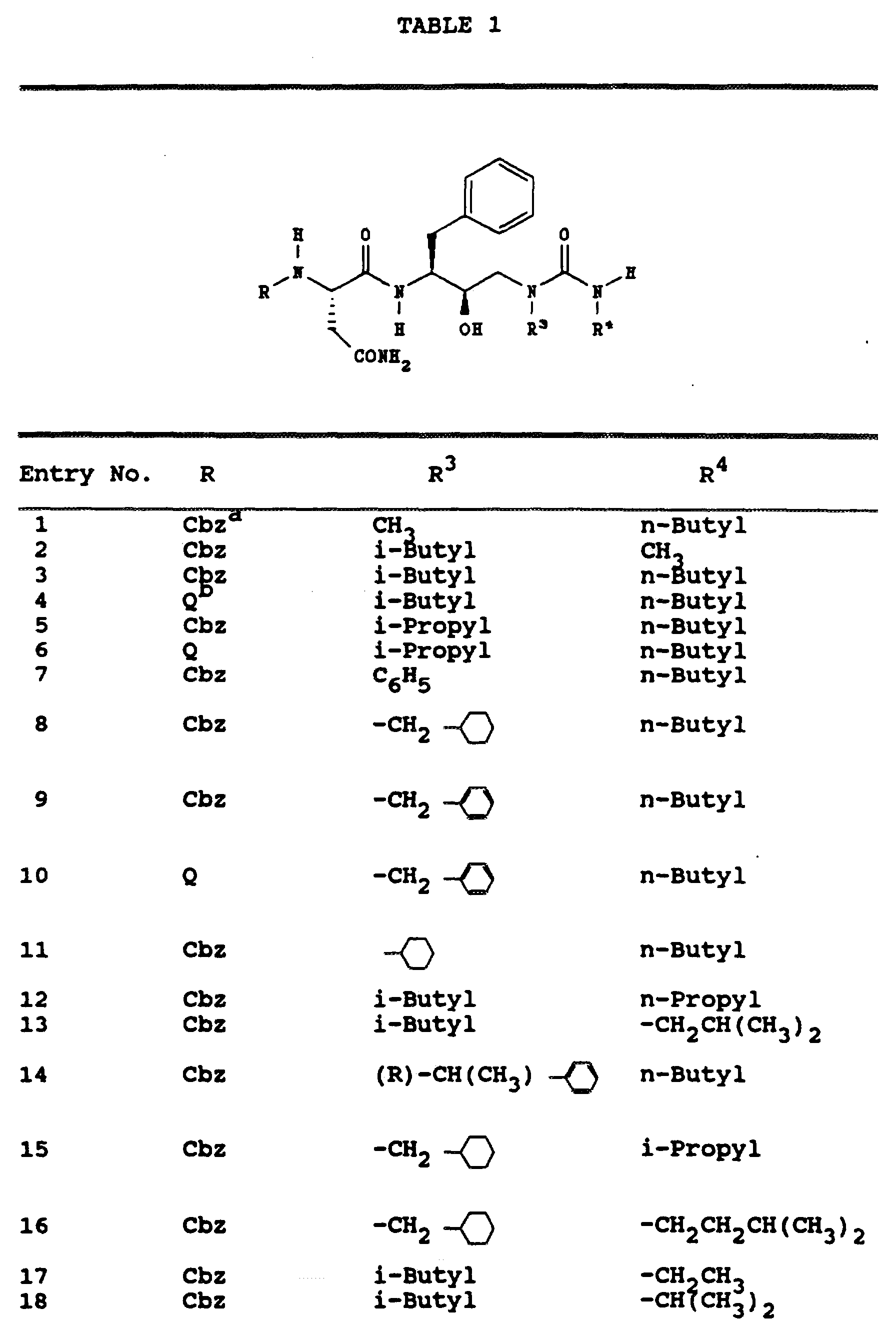

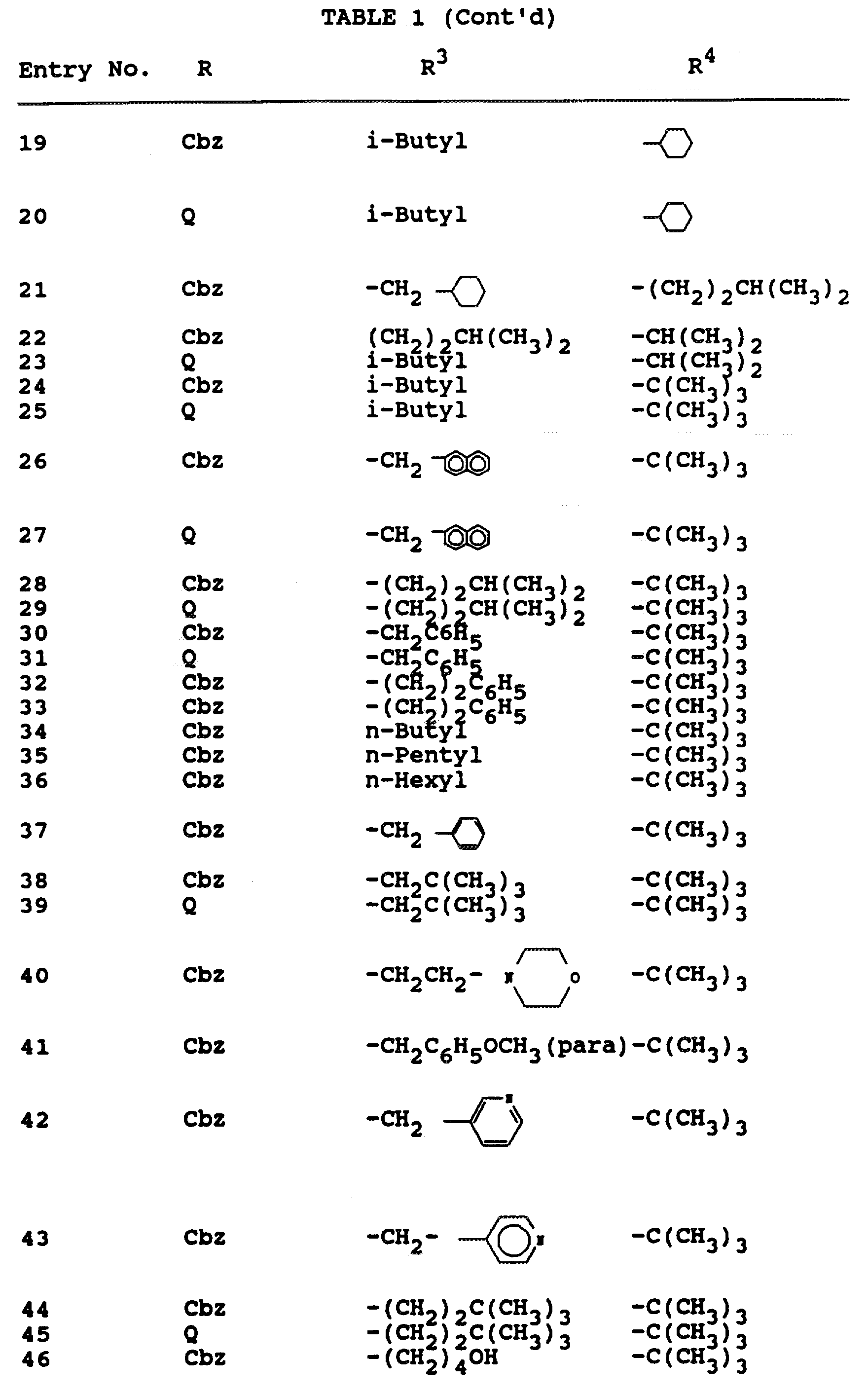

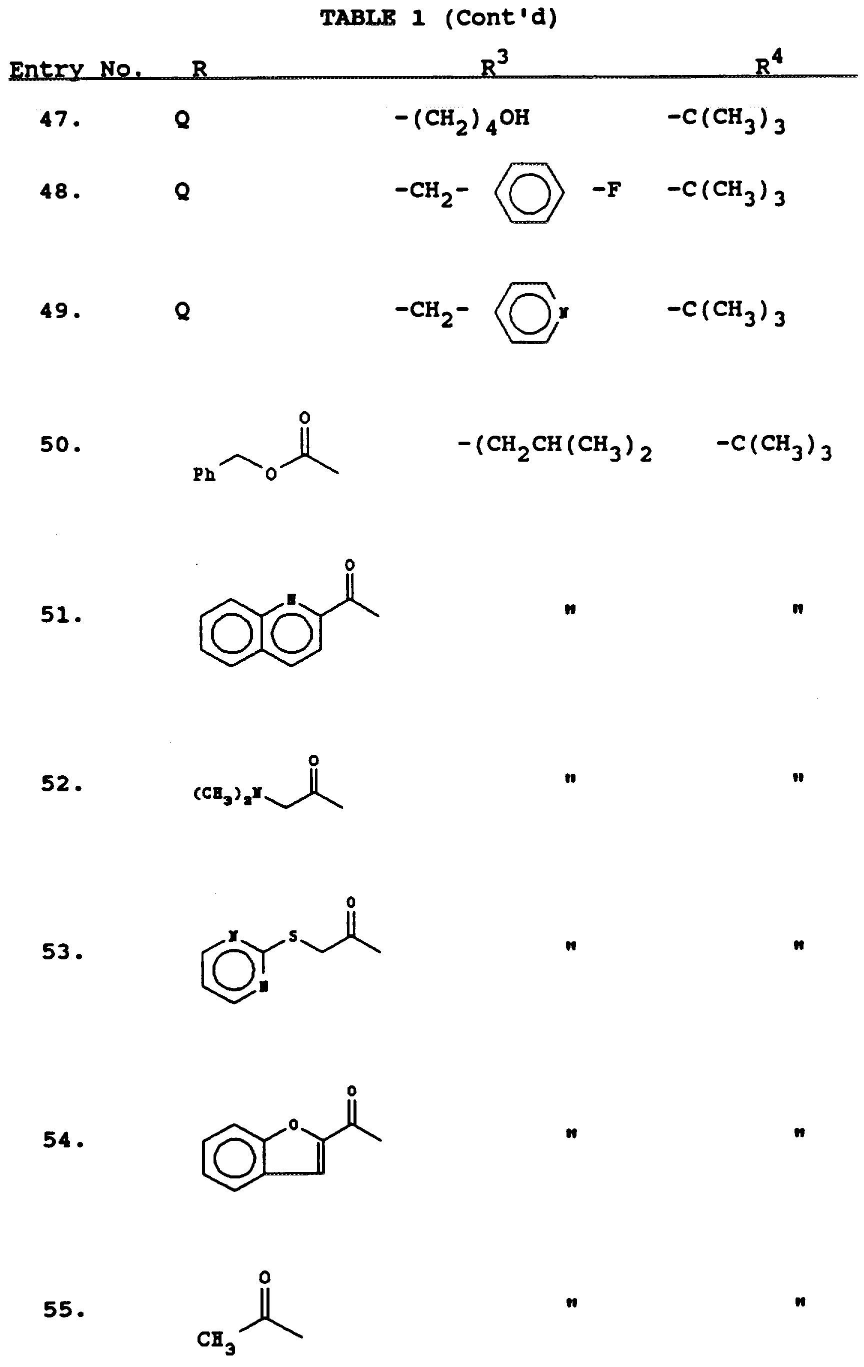

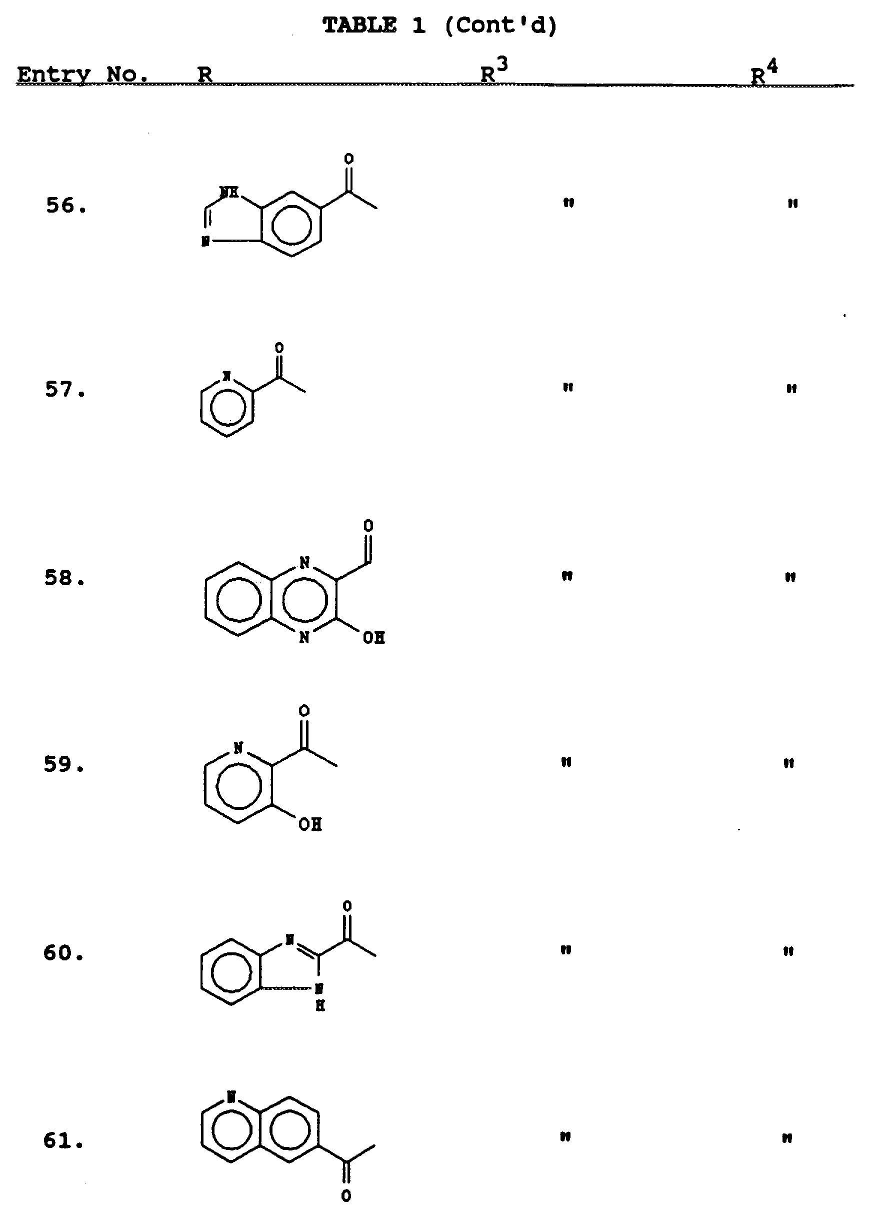

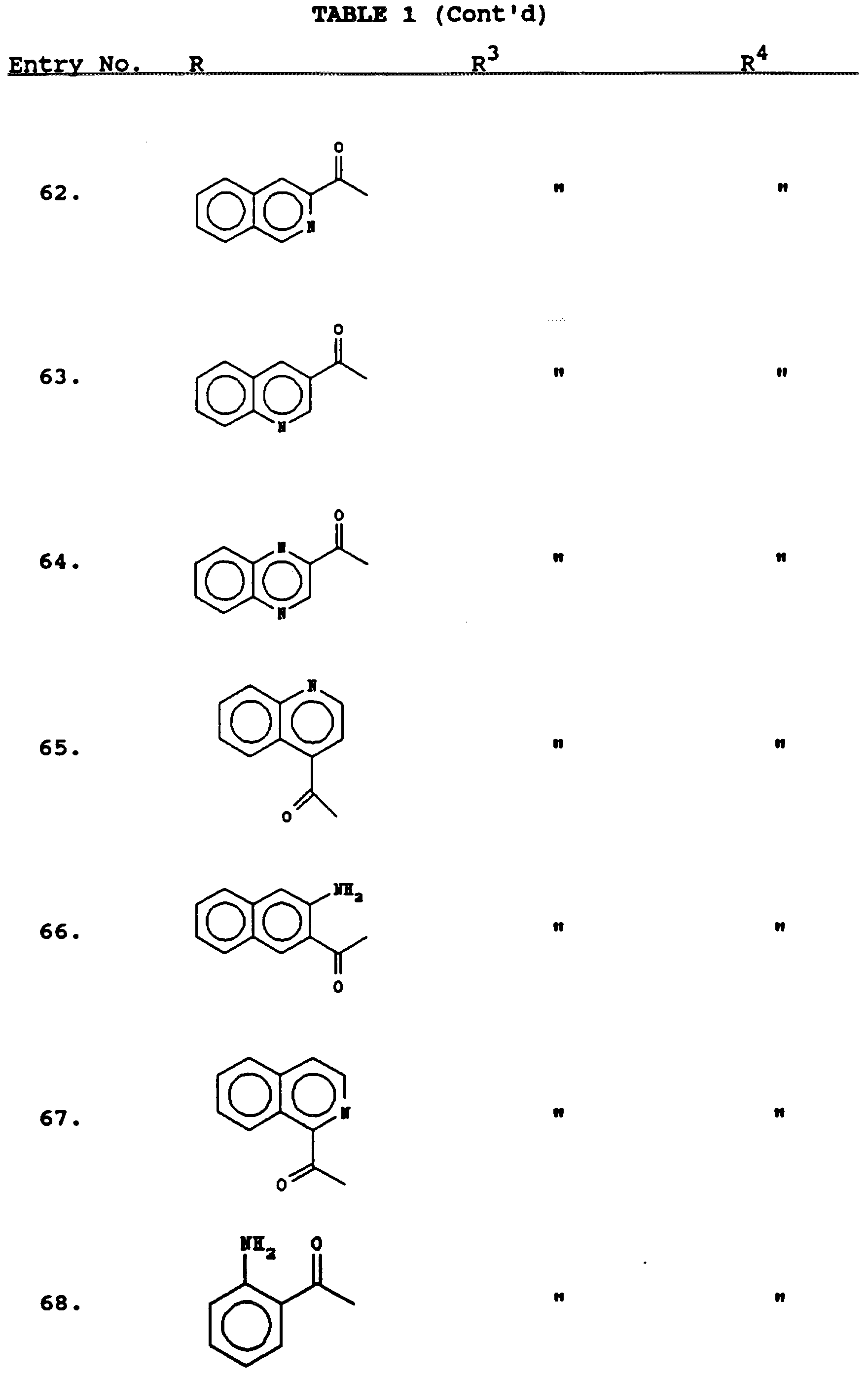

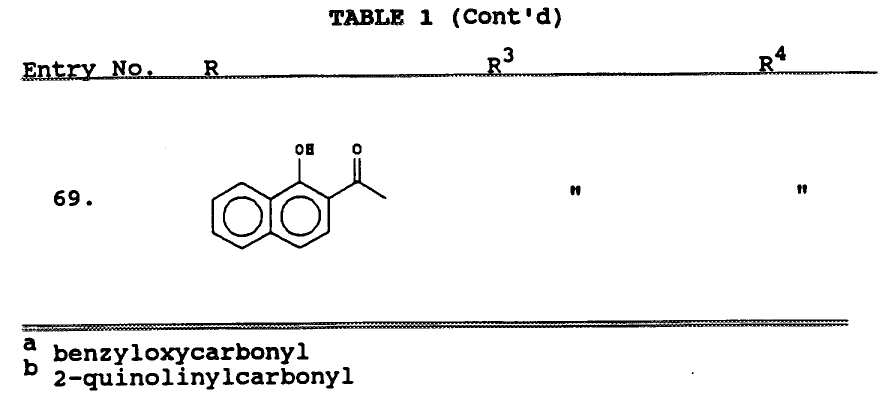

### EXAMPLE 10

Following the generalized procedures set forth in Example 9, the compounds set forth in Table 2 were prepared.

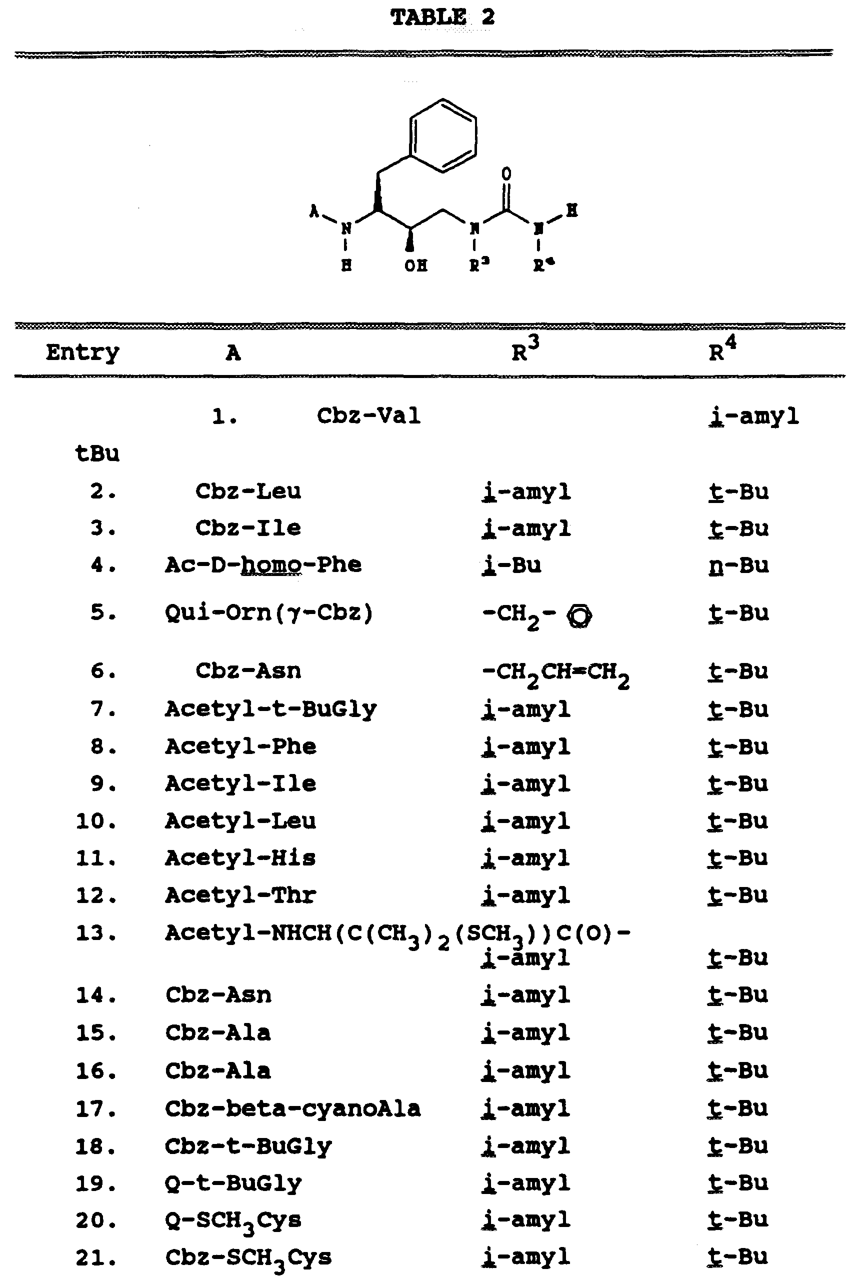

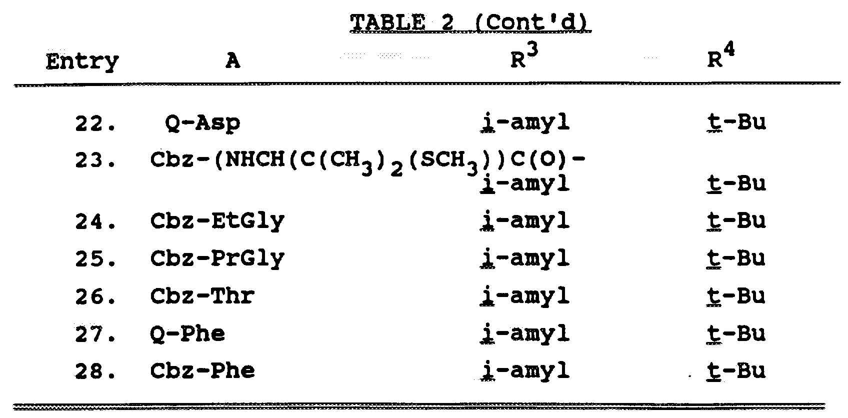

### EXAMPLE 11

Following the generalized procedure of Example 9, the compounds listed in Table 3 were prepared.

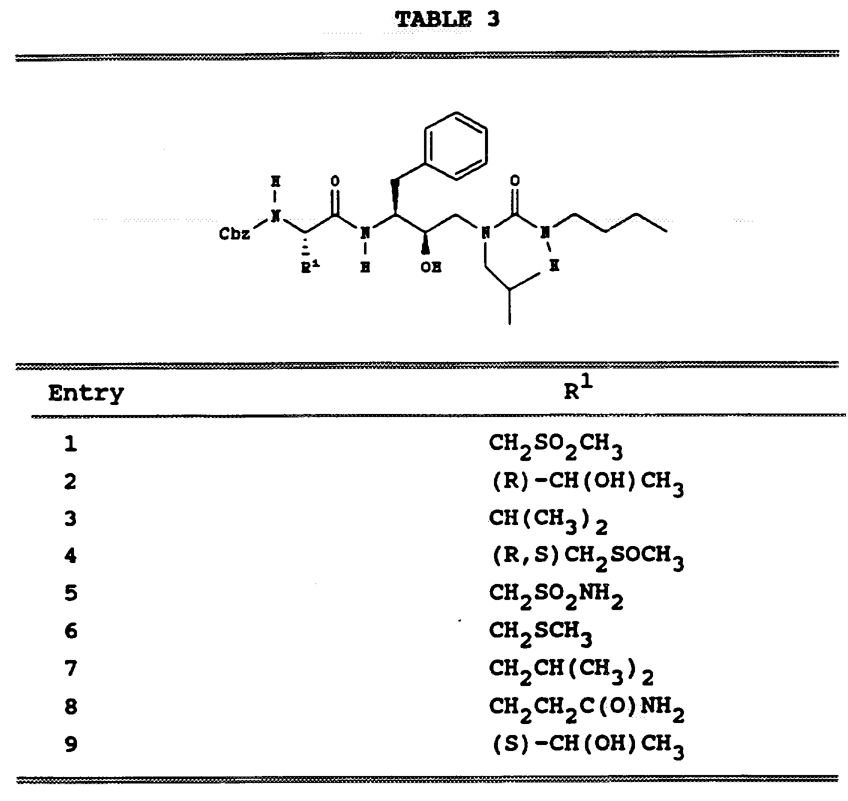

### EXAMPLE 12

Following the generalized procedures of Example 6, Part D and Example 9, the compounds set forth in Table 4 were prepared.

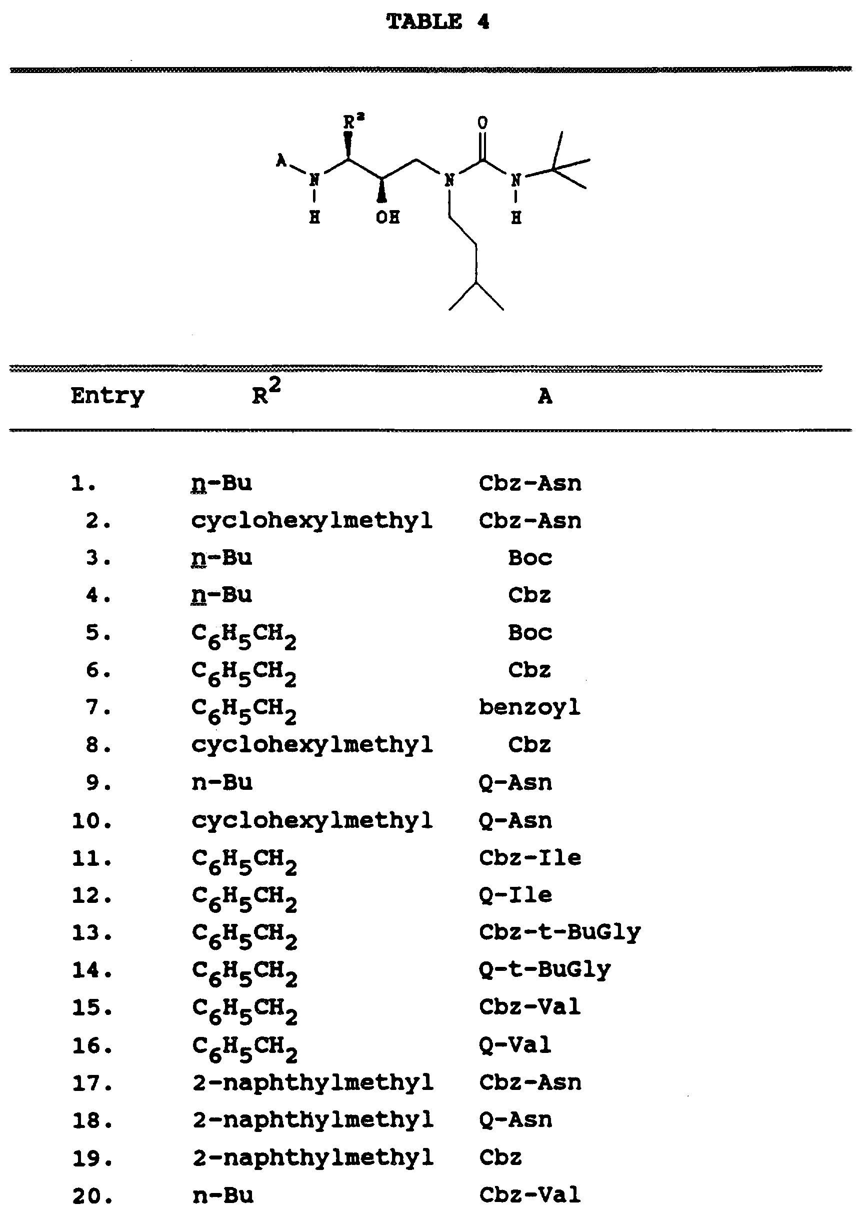

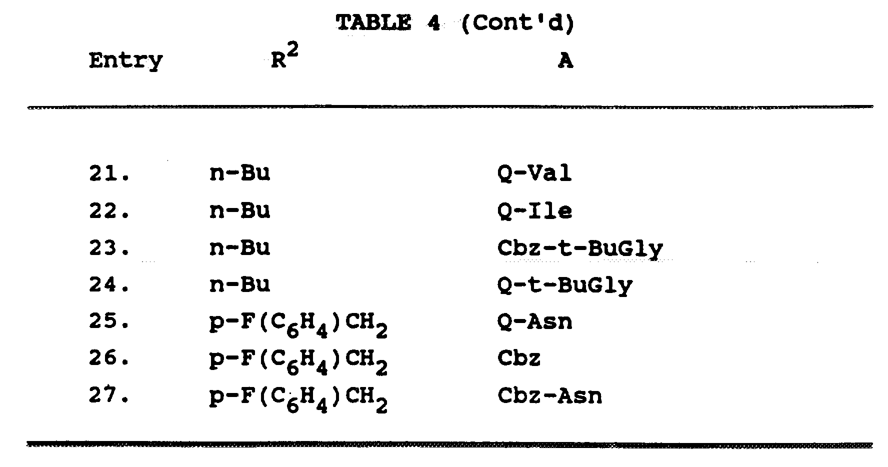

### EXAMPLE 13

The compounds listed in Table 5 were prepared according to the generalized procedures of Example 9.

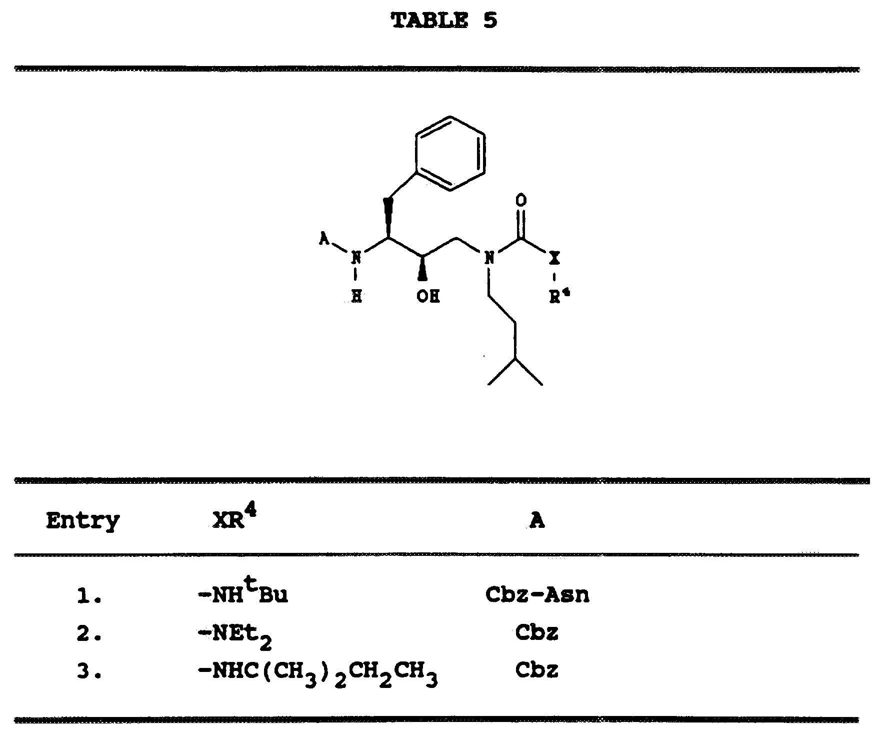

### EXAMPLE 14

The compounds of Table 6 were prepared according to the generalized procedures set forth in Example 9 except that instead of an isocyanate, an isothiocyanate equivalent was utilized.

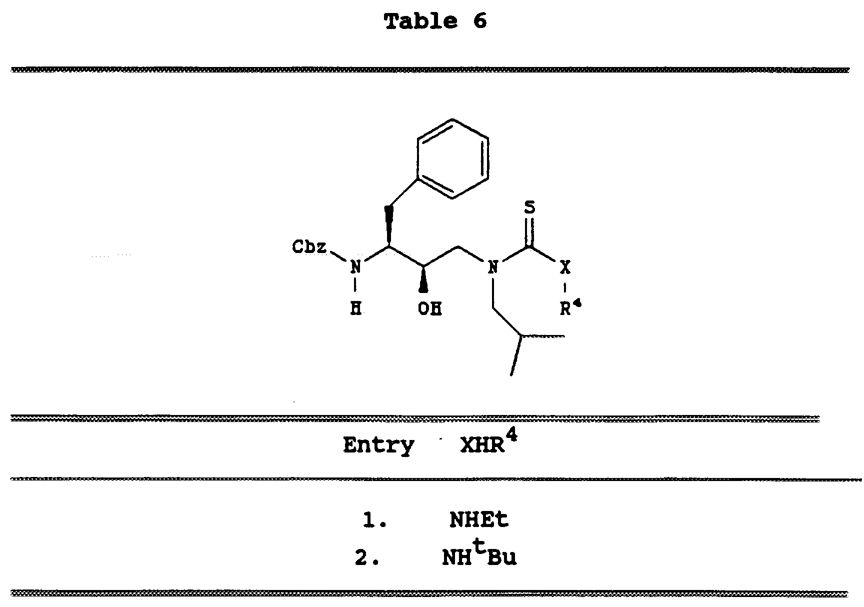

The Cbz group of the compounds shown in Examples 13 and 14 can be removed as described in Example 9 and the resulting compound can be coupled to a desired α- or β-amino acid or the like to produce compounds of the present invention.

### Example 15

The compounds shown in Table 7 were prepared according to the following general procedure. This general procedure represents a Curtius Rearrangement and reaction with the amino alcohol derivative as prepared following the general procedure in Example 9.

To a solution of 1 mmol of carboxylic acid in 12 mL of toluene and 3 mmol of triethylamine at 90°C under a nitrogen atmosphere, was added 1 mmol of diphenylphosphoryl azide. After 1 hour, a solution of 1 mmol of amino alcohol derivative in 3.5 mL of either N,N-dimethylformamide or toluene was added. After 1 hour, the solvent was removed under reduced pressure, ethyl acetate and water added and the layers separated. The organic layer was washed with 5% citric acid, sodium bicarbonate, brine, dried, filtered and concentrated to afford the crude product. This was then recrystallized or chromatographed on silica gel to afford the purified final compound.

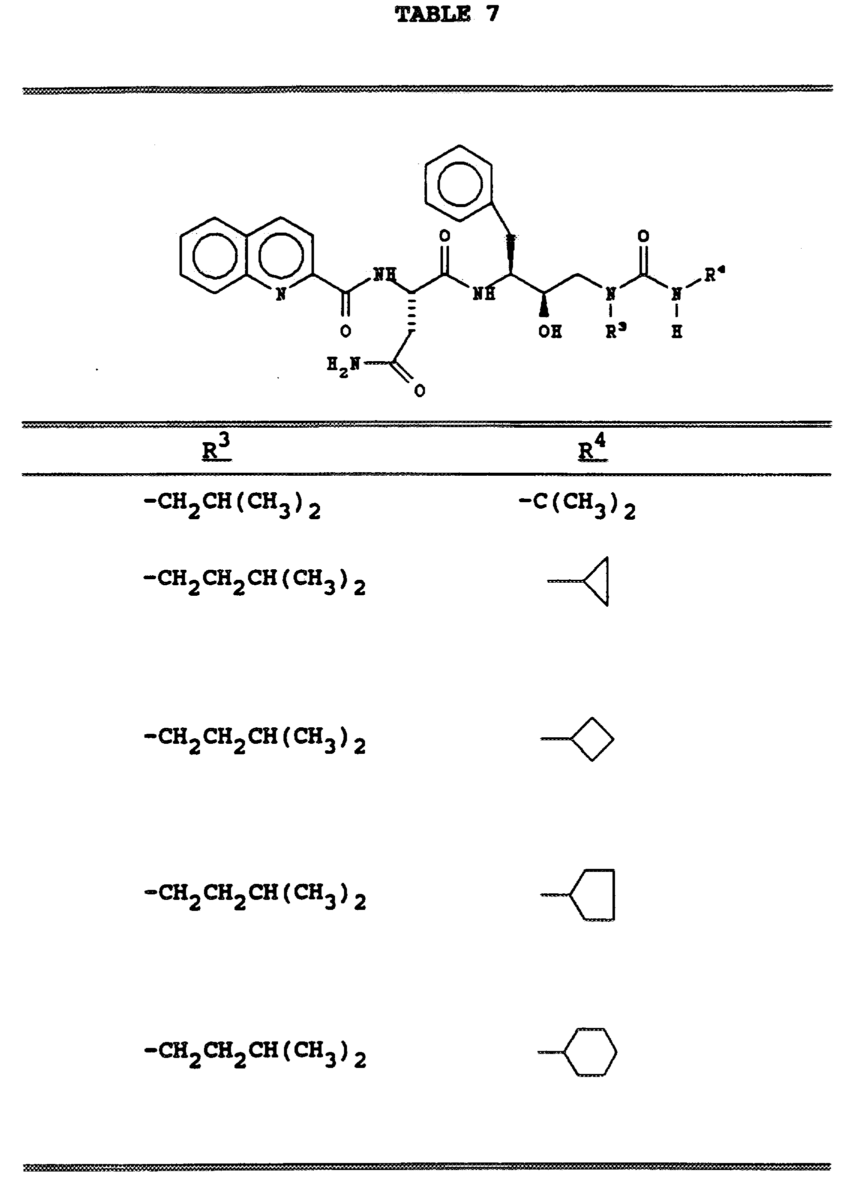

### Example 16

### A. Preparation of 4(4-methoxybenzyl)itaconate

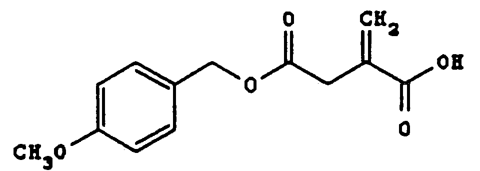

A 5 L three-necked round bottomed flask equipped with constant pressure addition funnel, reflux condenser, nitrogen inlet, and mechanical stirrer was charged with itaconic anhydride (660.8g, 5.88 mol) and toluene (2300 mL). The solution was warmed to reflux and treated with 4-methoxybenzyl alcohol (812.4g, 5.88 mol) dropwise over a 2.6h period. The solution was maintained at reflux for an additional 1.5h and then the contents were poured into three 2 L erlenmeyer flasks to crystallize. The solution was allowed to cool to room temperature whereupon the desired mono-ester crystallized. The product was isolated by filtration on a Buchner funnel and air dried to give 850.2g, 58% of material with mp 83-85°C, a second crop, 17% was isolated after cooling of the filtrate in an ice bath. ¹H NMR (CDCl₃) 300 MHz 7.32(d, J=8.7 Hz, 2H), 6.91(d, J=8.7 Hz, 2H), 6.49(s, 1H), 5.85(s, 1H), 5.12(s, 2H), 3.83(s, 3H), 3.40(s, 2H).

### B. Preparation of Methyl 4(4-methozybenzyl) itaconate

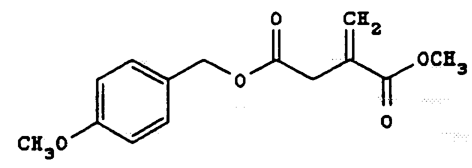

A 5 L three-necked round bottomed flask equipped with reflux condenser, nitrogen inlet, constant pressure addition funnel and mechanical stirrer was charged with 4(4-methoxybenzyl) itaconate (453.4g, 1.81 mol) and treated with 1,5-diazabicyclo[4.3.0]non-5-ene (275.6g, 1.81 mol), (DBU), dropwise so that the temperature did not rise above 15°C. To this stirring mixture was added a solution of methyl iodide (256.9g, 1.81 mol) in 250 mL of toluene from the dropping funnel over a 45m period. The solution was allowed to warm to room temperature and stirred for an additional 3.25h.

The precipitated DBU hydroiodide was removed by filtration, washed with toluene and the filtrate poured into a separatory funnel. The solution was washed with sat. aq. NaHCO₃ (2 X 500 mL), 0.2N HCl (1 X 500 mL), and brine (2 X 500 mL), dried over anhyd. MgSO₄, filtered, and the solvent removed in vacuo. This gave a clear colorless oil, 450.2g, 94% whose NMR was consistent with the assigned structure. ¹H NMR (CDCl₃) 300 MHz 7.30(d, J=8.7 Hz, 2H), 6.90(d, J=8.7 Hz, 2H), 6.34(s, 1H), 5.71(s, 1H), 5.09(s, 2H), 3.82(s, 3H), 3.73(s, 3H), 3.38(s, 2H). ¹³C NMR (CDl₃) 170.46, 166.47, 159.51, 133.55, 129.97, 128.45, 127.72, 113.77, 66.36, 55.12, 51.94, 37.64.

### C. Preparation of Methyl 4(4-methoxybenzyl) 2(R)-methylsuccinate

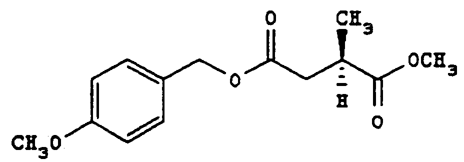

A 500 mL Fisher-Porter bottle was charged with methyl 4(4-methoxybenzyl) itaconate (71.lg, 0.269 mol), rhodium (R,R) DiPAMP catalyst (204mg, 0.269 mmol, 0.1 mol%) and degassed methanol (215 mL). The bottle was flushed 5 times with nitrogen and 5 times with hydrogen to a final pressure of 40 psig. The hydrogenation commenced immediately and after ca. 1h the uptake began to taper off, after 3h the hydrogen uptake ceased and the bottle was flushed with nitrogen, opened and the contents concentrated on a rotary evaporator to give a brown oil that was taken up in boiling iso-octane (ca. 200 mL, this was repeated twice), filtered through a pad of celite and the filtrate concentrated in vacuo to give 66.6g, 93% of a clear colorless oil, ¹H NMR (CDCl₃ 300 MHz 7.30(d, J=8.7 Hz, 2H), 6.91(d, J=8.7 Hz, 2H), 5.08(s, 2H), 3.82(s, 3H), 3.67(s, 3H), 2.95(ddq, J=5.7, 7.5, 8.7 Hz, 1H), 2.79(dd, J=8.1, 16.5 Hz, 1H), 2.45(dd, J=5.7, 16.5 Hz, 1H), 1.23(d, J=7.5 Hz, 3H).

### D. Preparation of Methyl 2(R)-methylsuccinate

A 3 L three-necked round-bottomed flask equipped with a nitrogen inlet, mechanical stirrer, reflux condenser and constant pressure addition funnel was charged with methyl 4(4-methoxybenzyl) 2(R)-methylsuccinate (432.6g, 1.65 mol) and toluene (1200 mL). The stirrer was started and the solution treated with trifluoroacetic acid (600 mL) from the dropping funnel over 0.25h. The solution turned a deep purple color and the internal temperature rose to 45°C. After stirring for 2.25h the temperature was 27°C and the solution had acquired a pink color. The solution was concentrated on a rotary evaporator. The residue was diluted with water (2200 mL) and sat. aq. NaHCO₃ (1000 mL). Additional NaHCO₃ was added until the acid had been neutralized. The aqueous phase was extracted with ethyl acetate (2 X 1000 mL) to remove the by-products and the aqueous layer was acidified to pH=1.8 with conc. HCl. This solution was extracted with ethyl acetate (4 X 1000 mL), washed with brine, dried over anhyd. MgSO₄, filtered and concentrated on a rotary evaporator to give a colorless liquid 251g, >100% that was vacuum distilled through a short path apparatus cut 1: bath temperature 120°C @ >1mm, bp 25-29°C; cut 2: bath temperature 140°C @ 0.5mm, bp 95-108°C, 151g, [α]_{D} @ 25°C=+1.38°C(c=15.475, MeOH), [α]_{D}=+8.48°C (neat); cut 3: bath temperature 140°C, bp 108°C, 36g, [α]_{D} @ 25°C=+1.49°C(c=15.00, MeOH), [α]_{D}=+8.98°C (neat). Cuts 2 and 3 were combined to give 189g, 78% of product, ¹H NMR (CDCl₃) 300 MHz 11.6(brs, 1H), 3.72(s, 3H), 2.92(ddq, J=5.7, 6.9, 8.0 Hz, 1H), 2.81(dd, J=8.0, 16.8 Hz, 1H), 2.47(dd, J=5.7,16.8 Hz, 1H), 1.26(d, J=6.9 Hz, 3H).

### E. Preparation of Methyl Itaconate

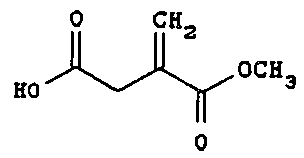

A 50 mL round bottomed flask equipped with reflux condenser, nitrogen inlet and magnetic stir bar was charged with methyl 4(4-methoxybenzyl) itaconate (4.00g, 16 mmol). The solution was kept at room temperature for 18 hours and then the volatiles were removed in vacuo. The residue was taken up in ethyl acetate and extracted three times with saturated aqueous sodium bicarbonate solution. The combined aqueous extract was acidified to pH=1 with aqueous potassium bisulfate and then extracted three times with ethyl acetate. The combined ethyl acetate solution was washed with saturated aqueous sodium chloride, dried over anhydrous magnesium sulfate, filtered, and concentrated in vacuo. The residue was then vacuum distilled to give 1.23g, 75% of pure product, bp 85-87 @ 0.1 mm. ¹H NMR (CDCl₃) 300 MHz 6.34(s, 1H), 5.73(s, 2H), 3.76(s, 3H), 3.38(s, 2H). ¹³C NMR (CDCl₃) 177.03, 166.65, 129.220, 132.99, 52.27, 37.46.

### F. Curtius Rearrangement of Methyl 2 (R)-methylsuccinate: Preparation of Methyl N-Moz-α-methyl β-alanine.

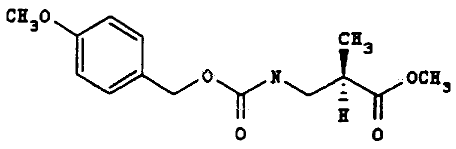

A 5L four"necked round bottomed flask equipped with a nitrogen inlet, reflux condenser, mechanical stirrer, constant pressure addition funnel, and thermometer adapter was charged with methyl 2(R)-methylsuccinate (184.1g,1.26 mol), triethylamine (165.6g, 218 mL, 1.64 mol, 1.3 equivalents), and toluene (1063 mL). The solution was warmed to 85°C and then treated dropwise with a solution of diphenylphosphoryl azide (346.8g, 1.26 mol) over a period of 1.2h. The solution was maintained at that temperature for an additional 1.0h and then the mixture was treated with 4-methoxybenzyl alcohol (174.1g, 1.26 mol) over a 0.33h period from the dropping funnel. The solution was stirred at 88°C for an additional 2.25h and then cooled to room temperature. The contents of the flask were poured into a separatory funnel and washed with sat. aq. NaHCO₃ (2 X 500 mL), 0.2N HCl (2 X 500 mL), brine (1 X 500 mL), dried over anhyd. MgSO₄, filtered, and concentrated in vacuo to give 302.3g, 85% of the desired product as a slightly brown oil. ₁H NMR (CDCl₃) 300 MHz 7.32(d, J=8.4 Hz, 2H), 6.91(d, J=8.4 Hz, 2H), 5.2(brm, 1H), 5.05(s, 2H), 3.83(s, 3H), 3.70(s, 3H), 3.35(m, 2H), 2.70(m, 2H), 1.20(d, J=7.2 Hz, 3H).

### G. Hydrolysis of Methyl N-Moz-α-methyl β-alanine: Preparation of α-methyl β-alanine Hydrochloride

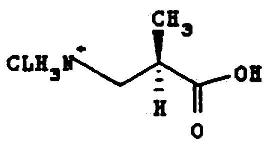

A 5 L three-necked round bottomed flask equipped with a reflux condenser, nitrogen inlet and mechanical stirrer was charged with methyl N-Moz-α-methyl β-alanine (218.6g, 0.78 mol), glacial acetic acid (975 mL) and 12N hydrochloric acid (1960 mL). The solution was then heated to reflux for 3h. After the solution had cooled to room temperature (ca. 1h) the aqueous phase was decanted from organic residue (polymer) and the aqueous phase concentrated on a rotary evaporator. Upon addition of acetone to the concentrated residue a slightly yellow solid formed that was slurried with acetone and the white solid was isolated by filtration on a Buchner funnel. The last traces of acetone were removed by evacuation to give 97.7g, 90% of pure product, mp 128.5-130.5°C [α]_{D} @ 25°C=9.0°C (c=2.535, Methanol). ¹H NMR (D₂O) 300 MHz 3.29(dd, J=8.6, 13.0 Hz, 1H), 3.16(dd, J=5.0, 13.0m Hz, 1H), 2.94(ddq, J=7.2, 5.0, 8.6 Hz, 1H), 1.30(d,J=7.2 Hz, 3H); ¹³C NMR (D₂O) 180.84, 44.56, 40.27, 17.49.

### H. Preparation of N-Boc-α-Methyl β-Alanine

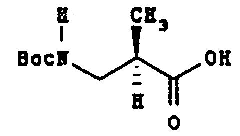

A solution of α-methyl β-alanine hydrochloride (97.7g, 0.70 mol) in water (1050 mL) and dioxane (1050 mL) the pH was adjusted to 8.9 with 2.9N NaOH solution. This stirring solution was then treated with di-tert-butyl pyrocarbonate (183.3g, 0.84 mol, 1.2 equivalents) all at once. The pH of the solution was maintained between 8.7 and 9.0 by the periodic addition of 2.5N NaOH solution. After 2.5h the pH had stabilized and the reaction was judged to be complete. The solution was concentrated on a rotary evaporator (the temperature was maintained at <40°C). The excess di-tert-butyl pyrocarbonate was removed by extraction with dichloromethane and then the aqueous solution was acidified with cold 1N HCl and immediately extracted with ethyl acetate (4 X 1000 mL). The combined ethyl acetate extract was washed with brine, dried over anhyd. MgSO₄, filtered and concentrated on a rotary evaporator to give a thick oil 127.3g, 90% crude yield that was stirred with n-hexane whereupon crystals of pure product formed, 95.65g, 67%, mp 76-78°C, [α]_{D} @ 25°C=-11.8°C (c=2.4, EtOH). A second crop was obtained by concentration of the filtrate and dilution with hexane, 15.4g, for a combined yield of 111.05g, 78%. ¹H NMR (acetone D₆) 300 MHz 11.7 (brs, 1H), 6.05 (brs 1H), 3.35 (m, 1H), 3.22 (m, 1H), 2.50 (m, 1H), 1.45(s, 9H), 1.19 (d, J=7.3 Hz, 3H); ¹³C NMR (acetone D₆) 177.01, 79.28, 44.44, 40.92, 29.08, 15.50. Elemental analysis calc'd. for C₉H₁₇NO₄: C, 53.19, H, 8.42; N, 6.89. Found: C, 53.36; H, 8.46; N, 6.99.

### I. Preparation of N-4-Methoxybenzyloxycarbonyl α-Methyl β-Alanine

A solution of N-4-methoxybenzyloxycarbonyl α-methyl β-alanine methyl ester (2.81g, 10.0 mmol) in 30 mL of 25% aqueous methanol was treated with lithium hydroxide (1.3 equivalents) at room temperature for a period of 2h. The solution was concentrated in vacuo and the residue taken up in a mixture of water and ether and the phases separated and the organic phase discarded. The aqueous phase was acidified with aqueous potassium -hydrogen sulfate to pH=1.5 and then extracted three times with ether. The combined ethereal phase was washed with saturated aqueous sodium chloride solution, dried over anhydrous magnesium sulfate, filtered and concentrated in vacuo to give 2.60 g, 97% of N-4-Methoxybenzyloxycarbonyl α-methyl β-alanine (N-Moz-AMBA) which was purified by recrystallization from a mixture of ethyl acetate and hexane to give 2.44g, 91% of pure product, mp 96-97°C, MH+=268. ¹H NMR (D₆-acetone/300 MHz) 1.16 (3H, d, J-7.2Hz), 2.70 (1H, m), 3.31 (2H, m), 3.31 (3H, s), 4.99 (2H, s), 6.92 (2H, 4, J=8.7 Hz), 7.13 (2H, d, J=8.7 Hz).

### J. Preparation of Propanamide, 3-(4-methoxybenzyloxycarbonyl)-N [3-[[[(1,1-dimethylethyl)amine]carbonyl](3-methylbutyl)amino]-2 -hydroxy-1-(phenylmethyl)propyl]-2-methyl-[IS-[IR*(S*), 2S*]]-

N-Moz-AMBA (468mg, 1.75mmol) was dissolved in 5mL of DMF, HOBT (355mg, 2.6mmol) was added and the solution was cooled to 0°C. The solution was treated with (336mg, 1.75mmol) EDC for 15 minutes. To this was added (612mg, 1.75mmol) of [2R,3S 3-amino-1-isoamyl-1-(t-butylcarbonyl)amino 4-phenyl-2-butanol in 10mL of DMF and the reaction stirred for 16 hours at room temperature. The DMF was concentrated to 5mL and the product was precipitated by addition to 60% saturated aqueous NaHCO₃. The solid was taken up in ethyl acetate and washed with KHSO₄, NaHCO₃, NaCl(saturated), dried over MgSO₄ and concentrated to yield 680mg of crude product which was crystallized from CH₂Cl₂, Et₂O, hexane, to yield 300mg of pure product.

### Example 17

The compounds of Table 8 were prepared according to the procedure listed below and that utilized in Example 16.

### Propaneamide 3-[(1,1--dimethylethyl)butoxycarbonyl]amino-N-[3-[[[(1,1 -dimethylethyl)amino]carbonyl](3-methylbutyl)amino]-2 -hydroxy-1-(phenylmethyl)propyl]-2-methyl-, [1S-[1R*(S*),2S*]-

### Part A.

A solution of N-t-butyloxycarbonyl-2-(R)-methyl-3-aminopropionic acid (372 mg, 1.83 mmol) and N-hydroxybenzotriazole (371 mg, 2.75 mmol) in 5 mL of dimethylformamide was cooled to 0 degrees C. To this was added EDC (351 mg, 1.83 mmol) and the solution was stirred for 15 minutes. To this chilled solution was added a solution of 3-[[[(1,1-dimethylethyl)amino]carbonyl](3-methylbutyrl)amino]-2(R)-hydroxy-1(S)(phenylmethyl)propylamine in 5 mL of dimethylformamide and stirred for 15 hours. The dimethylformamide was removed and replaced with 50 mL of ethyl acetate, and the organic phase was extracted with 5% potassium hydrogen sulfate, saturated sodium bicarbonate and brine. The ethyl acetate layer was dried over magnesium sulfate, filtered and concentrated to yield 613 mg of product after recrystallization from ethyl acetate , hexanes. (63 % yield). M+Li 541

### Part B.

Preparation of Propaneamide,_3-amino-N-[3-[[[(1,1-dimethylethyl)amino] carbonyl]- (3-methylbutyl)amino]-2-hydroxy-1-(phenylmethyl)propyl]-2-methyl-,[1S-[1R*(S*), 2S*]-hydrochloride

The product from part A. (577 mg, 1.08 mmol) was dissolved in 40 mL of 4N HCl in dioxane and the solution stirred for 2 hours, and concentrated to yield the hydrochloride salt in quantitative yield.

### Part C.

Preparation of Propaneamide, 3-(2-methylpropanoylamino)-N-[3-[[[(1,1-dimethylethyl)-amino]carbonyl](3-methylbutyl)amino]-2-hydroxy-1-(phenylmethyl)propyl]-2-methyl-,[1S-[1R*(S*),2S*]-

The product from part B. (236 mg, 0.5 mmol) was dissolved in anhydrous tetrahydrofuran and to this was added N-methylmorpholine (160 mg, 1.5 mmol) upon which time a precipitate formed. To this suspension was added isobutyryl chloride ( 53.5 mg, 0.5 mmol) and the suspension stirred for 15 hours. The suspension was diluted with ethyl acetate and washed with 5% potassium hydrogen sulfate, saturated sodium bicarbonate and brine. the organic layer was dried over magnesium sulfate, filtered and concentrated to yield 195 mg of crude product which was chromatographed on silica gel with 5% methanol methylene chloride to yield 121.5 mg ( 50 % yield) of pure product. M+Li 511

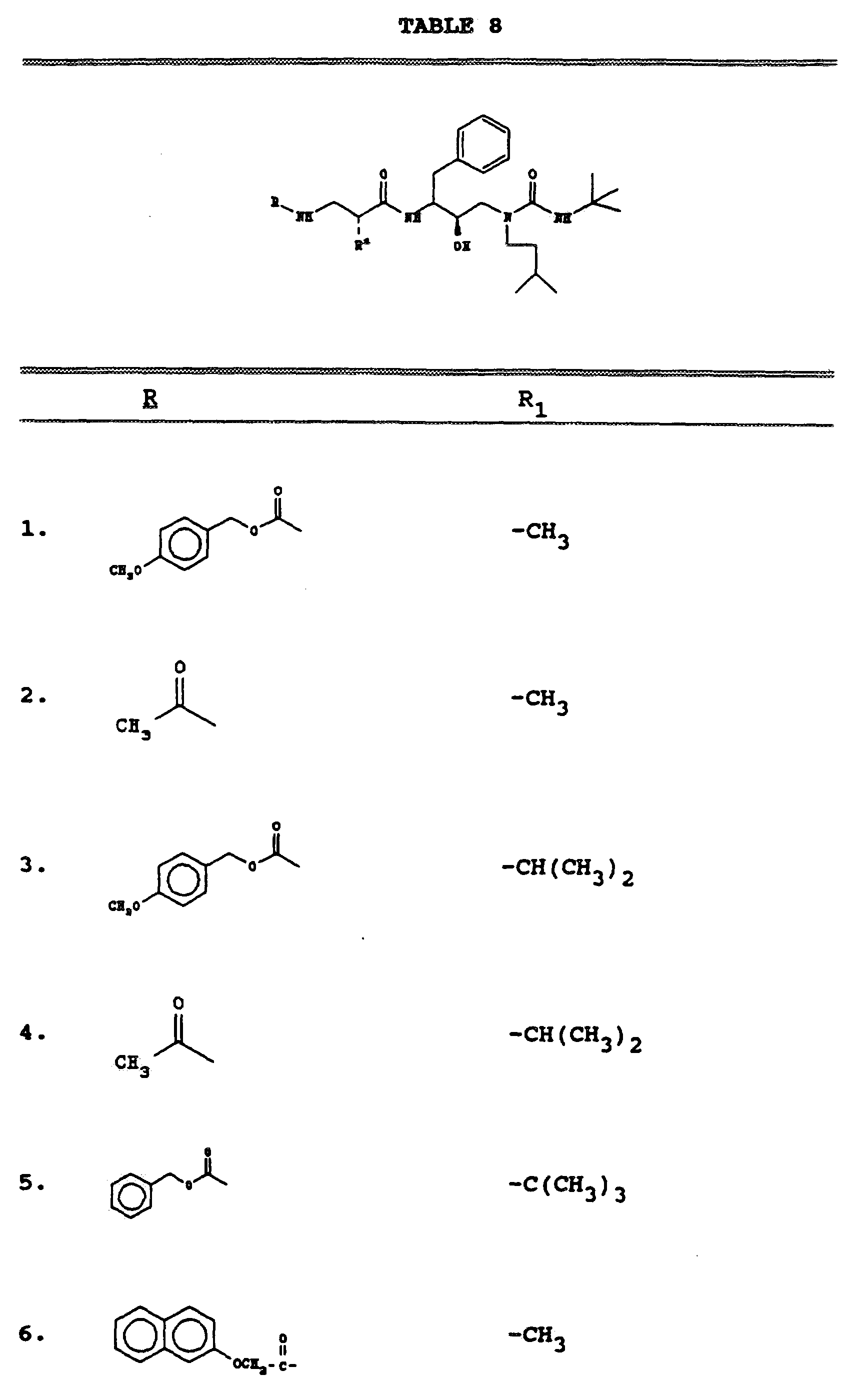

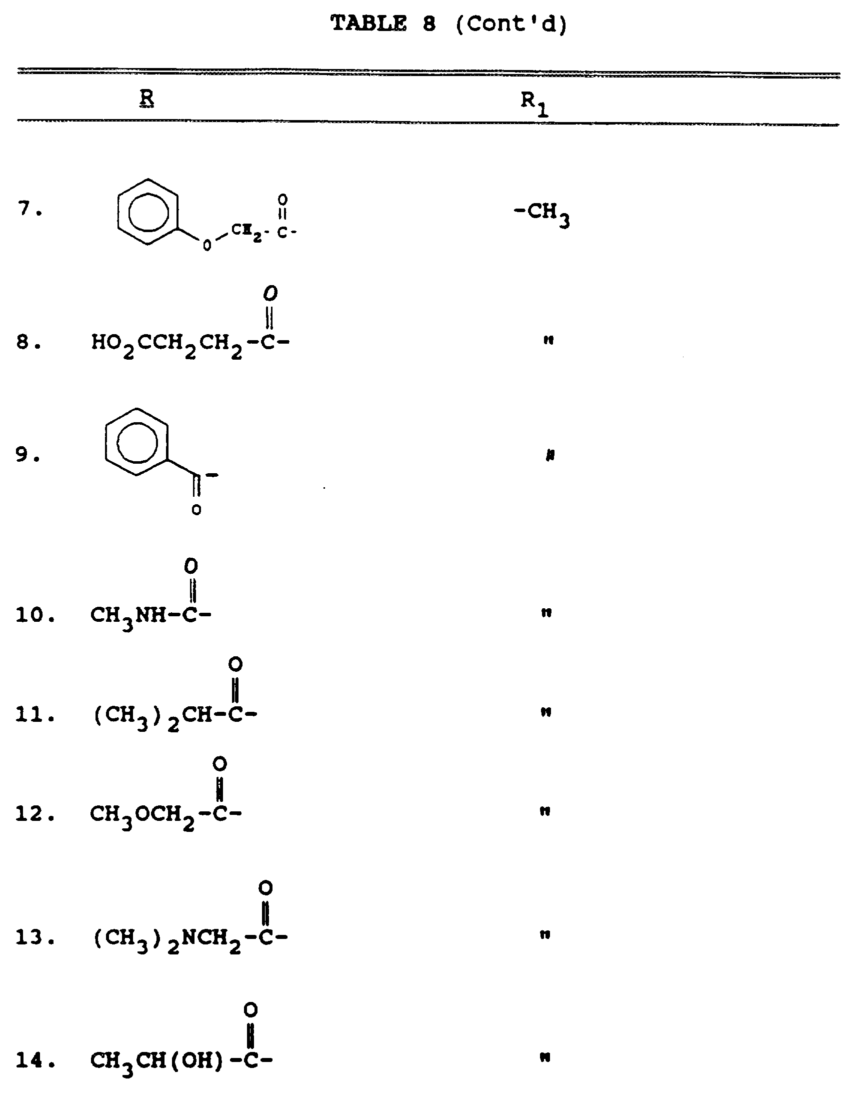

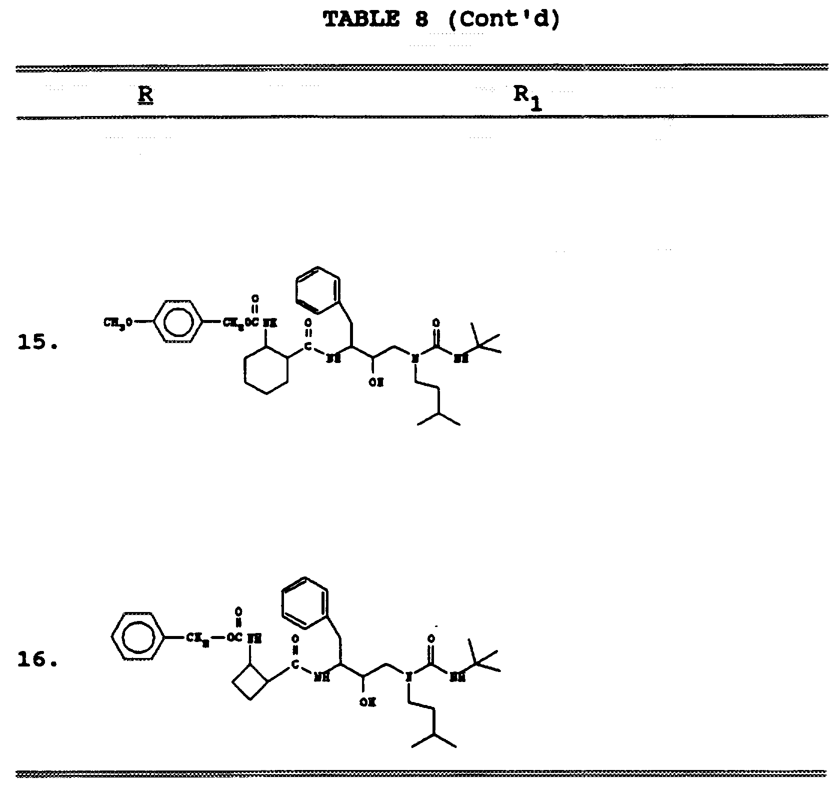

### Example 18

Following generally the procedure set forth in Example 16, the compounds shown in Table 9 were prepared.

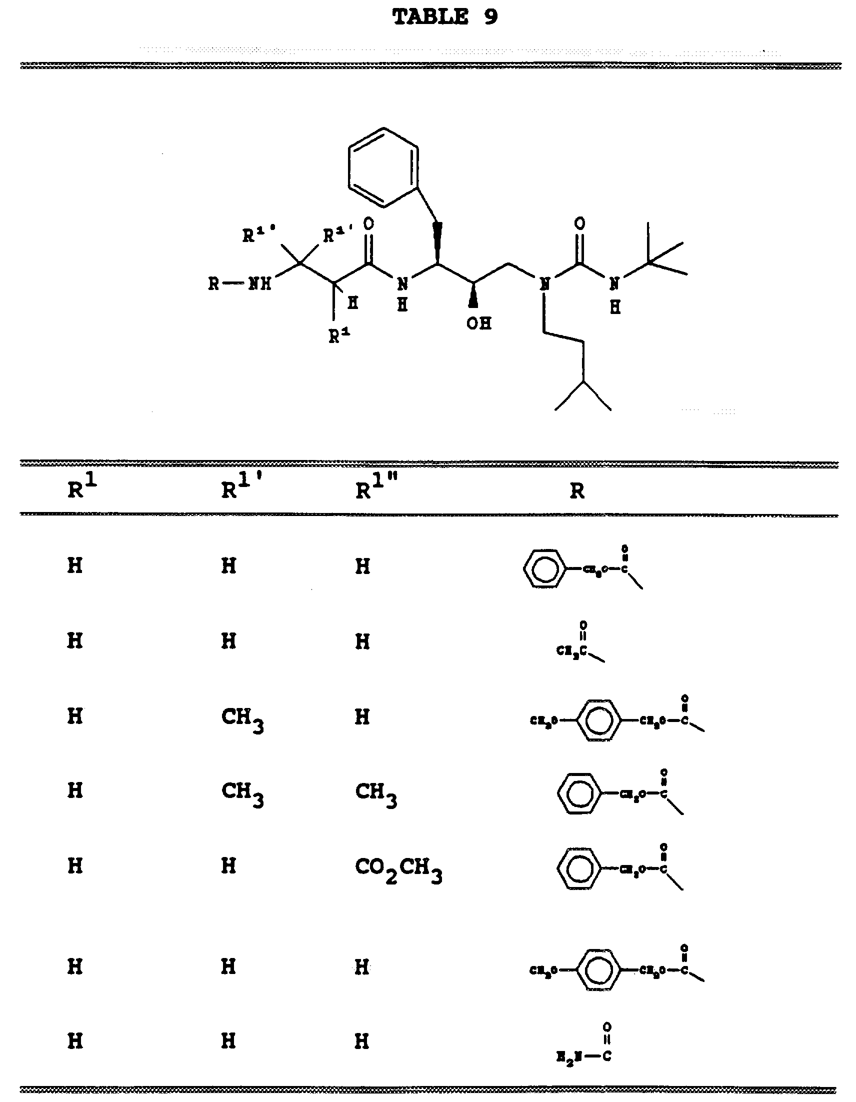

### Example 19

The procedure set forth below was generally utilized to prepare the compounds shown in Table 9

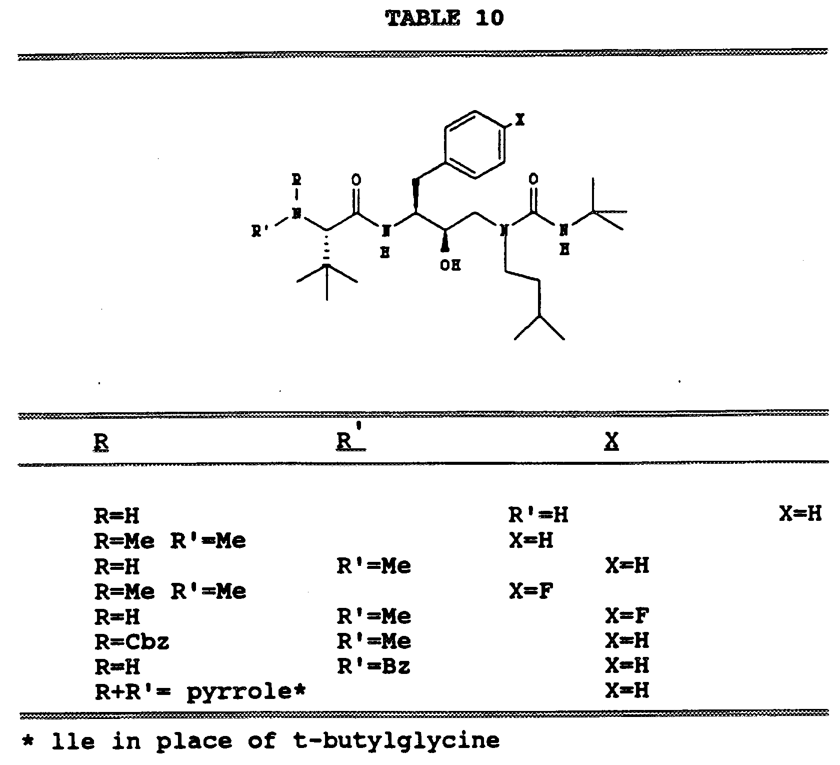

### Example 20

This example illustrates preparation of compounds wherein R⁴ and R⁵ together with N, forms a heterocycloalkyl radical.

### a) Pyrrolidine carbamoyl chloride.

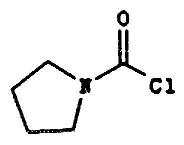

A stirring solution of triphosgene (27.78g, 0.103 mol) in 40 mL toluene was cooled to -20 °C in an ice/salt bath under a blanket of nitrogen and treated with a solution of N-methylmorpholine (27.3 g, 0.27 mol) in 20 mL of toluene dropwise over 1h. This solution was then treated with a solution of pyrrolidine (19.8 g, 0.27 mol) in 30 mL of toluene over a period of 30 m. The solution was allowed to warm to room temperature, filtered and the filtrate concentrated in vacuo to give an oil that was purified by vacuum distillation through a 12" Vigeraux column to give 20.7g, 56%, bp 58 °C @ 0.6 mm, of pure product.

### b) Butanediamide, N¹-[3-[[(4-fluorophenyl)methyl)](1-pyrrolidinylcarbonyl)amino]-2-hydroxy-1-(phenylmethyl)propyl]-2-[(2-quinolinylcarbonyl) amino]-[1S[1R*(R*) ,2S*]]-

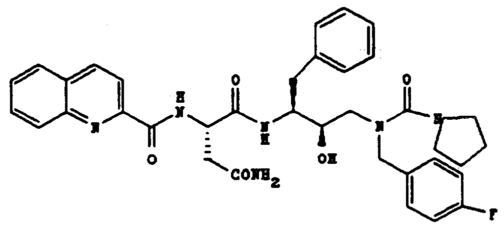

A stirring solution of [1S-[1R*(R*),2S*]]-N¹-[3-[[(4-fluorophenyl)methyl]amino]-2-hydroxy-1-(phenylmethyl)propyl]-2-[(2-quinolinylcarbonyl)amino-butanediamide (1.08 g, 1.91 mmol) in 7 mL of anhydrous DMF was treated with pyrrolidine carbamoyl chloride (260 mg, 1.95 mmol), 4-dimethylaminopyridine (15 mg), and N-methylmorpholine (380 mg, 3.76 mmol). The solution was stirred at room temperature for 3h and then concentrated in vacuo to give a semi-solid that was dissolved in methanol/water ca. 2:1. A solid formed from this solid that was isolated by filtration on a Büchner funnel and washed with water, 5% aq. citric acid and water and air dried to give 130 mg of pure product, TLC on SiO₂ eluting with 7% methanol in ethyl acetate showed one spot with R_{f}=0.64, 11%.

### c) Butanediamide, N¹-[3-[[(4-fluorophenyl)methyl)](4-morpholinylcarbonyl)amino]-2-hydroxy-1-(phenylmethyl)propyl]-2-[(2-quinolinylcarbonyl) amino]-[1S[1R*(R*),2S*]]-

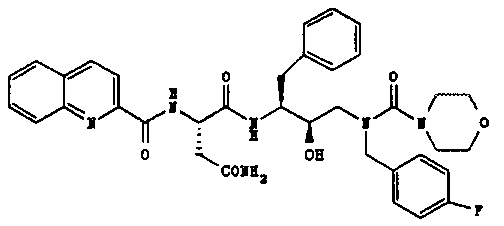

To a stirring solution of [1S-[1R*(R*),2S*]]-N¹-[3-[[(4-fluorophenyl)methyl]amino]-2-hydroxy-1-(phenylmethyl)propyl]-2-[(2-quinolinylcarbonyl)amino-butanediamide (520 mg, 0.922 mmol), triethylamine (172 mg, 1.70 mmol), 4-dimethylaminopyridine (50 mg), and morpholino carbamoyl chloride (157.3 mg, 1.05 mmol) in 5 mL of chloroform. The initially heterogeneous mixture was heated to reflux for 6 h. The solution was then diluted with additional chloroform, poured into a separatory funnel and washed with 1N KHSO₄, sat. aq. NaHCO₃, dried over anhyd. MgSO₄, filtered, and concentrated in vacuo to give a white solid that was purified by column chromatography on siO₂ eluting with ethanol/ethyl acetate to give 380 mg, 61%, of pure product.

### Example 21

This example illustrates preparation of compounds wherein R⁴ and R⁵ are both other than H.
Butanediamide, N¹-[3-[[(diethylamino)carbonyl](3-methylbutyl)amino]-2- hydroxy-1-(phenylmethyl)propyl]-2-[(2-quinolinylcarbonyl)amino]-[1S-[1R*(R*),2S*]]- 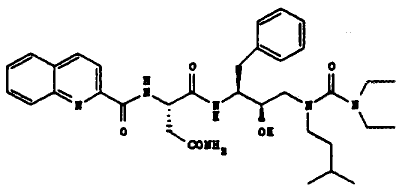 To a stirring solution of [1S-[1R*(R*),2S*]]-N¹-[3-(methylbutyl)amino]-2-hydroxy-1-(phenylmethyl)propyl]-2-[(2-quinolinylcarbonyl)amino-butane diamide] (119 mg, 0.21mmol) triethylamine (59 mg, 0.58 mmol), 4-dimethylaminopyridine (9 mg), and diethyl carbamoyl chloride (157.3 mg, 1.05 mmol) in 4 mL of chloroform. The mixture was kept at room temperature for 26 h. The solution was then diluted with additional chloroform, poured into a separatory funnel and washed with 1N KHSO₄, sat. aq. NaHCO₃, dried over anhyd. MgSO₄, filtered, and concentrated in vacuo to give a white solid that was purified by column chromatography on SiO₂ eluting with methanol/CH₂Cl₂ to give 20 mg, 15%, of pure product.

### Example 22

Following the procedures set forth in Example 26, the compounds listed in Table 11 were prepared.

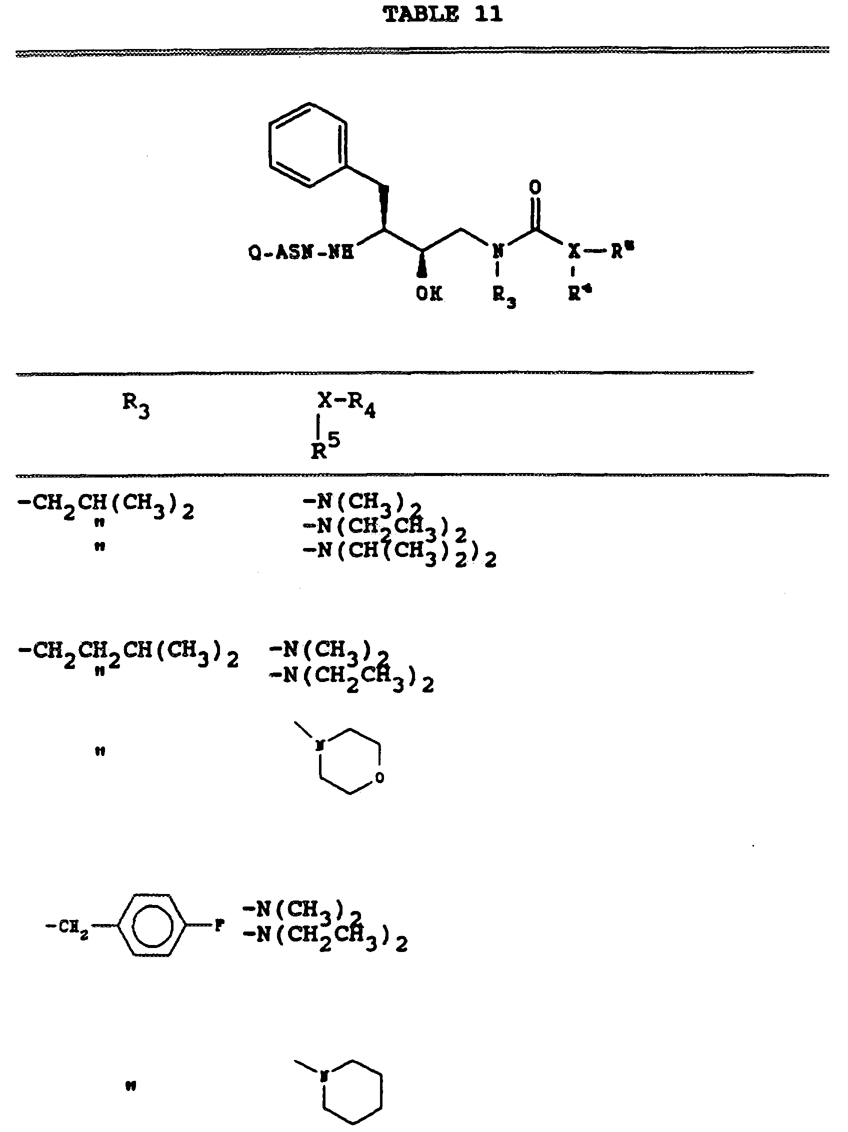

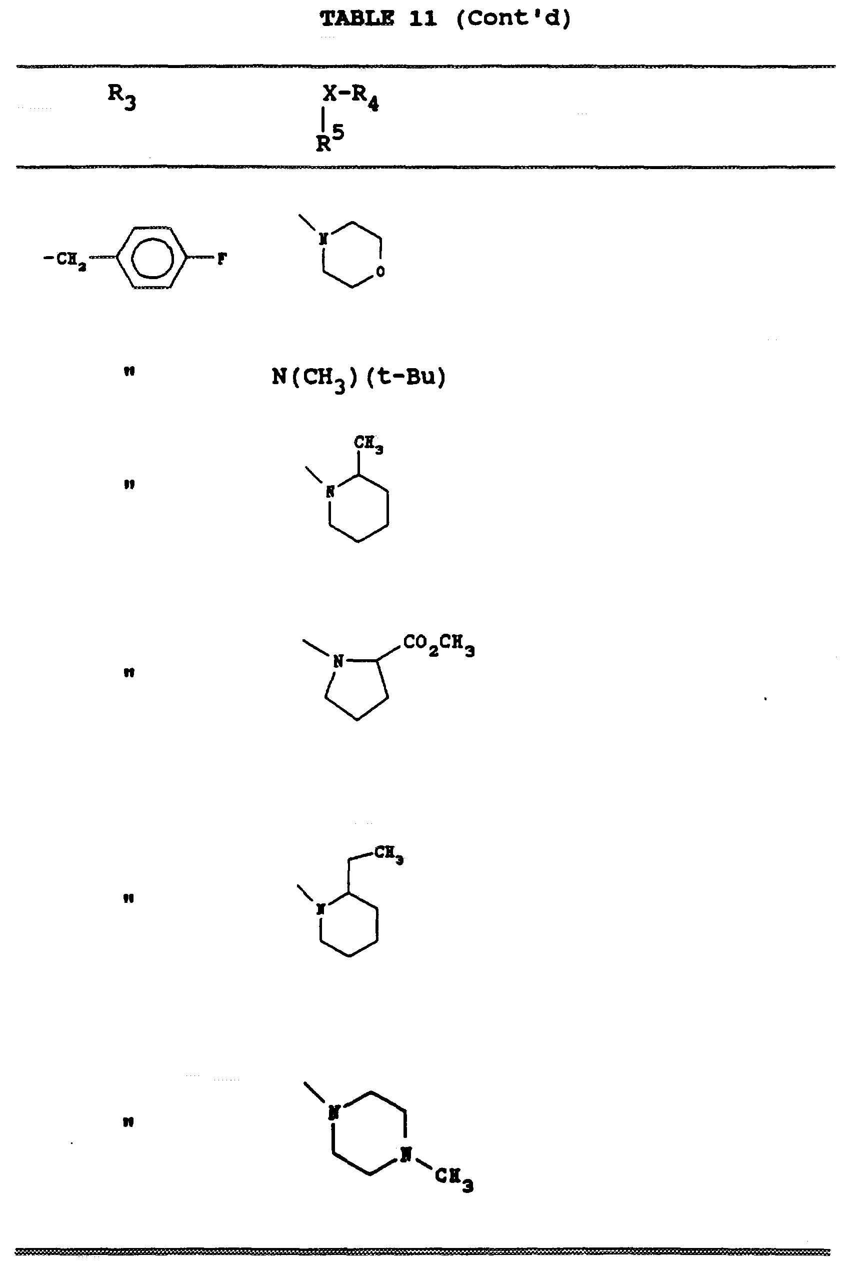

### Example 23

### 3-[[(1,1-dimethylethyl)amino]carbonyl](3-methylbutyl)amino-2(R)-hydroxy-1(S)-(phenylmethyl)propyl amine

This example illustrates preparation of compounds of Formula II wherein R¹ is an alkyl group other than an alkyl group of a naturally occurring amino acid side chain.

### Part A:

3-[[(1,1-dimethylethyl)amino]carbonyl](3-methylbutyl)amino-2(R)-hydroxy-1(S)-[N-(benzyloxycarbonyl)(phenylmethyl()propyl amine] (4.7 gm, 9.7 mmol) was combined with 10% Pd on carbon (200 mg) and conc. HCl (3 mL) in ethanol (35 mL) and hydrogenated at 50 psi of hydrogen for 2.5 h. The reaction mixture was filtered through diatomaceous earth and concentrated on a rotary evaporator to a yellow hygroscopic solid; 3.7 gm, 100%.

### Part B:

### Butaneamide, 2-[(phenylmethyloxycarbonyl)amino]-N-[3-[[[(1,1-dimethylethyl)amino]carbonyl](3 -methylbutyl)amino]-2-hydroxy-1-(phenylmethyl)propyl -3,3-dimethyl-[1S-[1R*(R*), 25*]]-

N-Cbz-L-tert-leucine (172 mg, 0.65 mmol) and N-hydroxybenzotriazole (100 mg, 0.65 mmol) in DMF (3 mL) was cooled to 0 C and EDC (115 mg, 0.60 mmol) added. After 45 min the amine from Part A (193 mg, 0.50 mmol) and N-methylmorpholine (60 uL, 0.55 mmol) were added. The reaction was stirred at ambient temperature for 18 h and poured into a solution of 50% saturated NaHCO₃ (25 mL). The solid was collected by suction filtration, washed with water and dried in-vacuo. The solid was chromatographed on SiO₂ using 2% MeOH in CH₂Cl₂ . The appropriate fractions were pooled and concentrated to afford a white solid; 220 mg, MH⁺ 597, TLC (SiO₂ 2%MeOH/CH₂Cl₂) R_{f} = .2 . CHN requires: C, 68.42, H, 8.78, N, 9.39; found: C, 68.03, H, 8.83, N, 9.33.

### Part C:

### Butaneamide, 2-amino-N-[3-[[[(1,1-dimethylethyl)amino]carbonyl](3-methylbutyl)amino]-2 -hydroxy-1-(phenylmethyl)propyl-3,3-dimethyl-, [1S-[1R*(R*), 2S*]-

The product from Part B (570 mg, 0.95 mmol) and 4% Pd on carbon (150 mg) in ethanol (30 mL) was hydrogenated at 5 psi for 2.75 h. The reaction mixture was filtered through diatomaceous earth and concentrated on a rotary evaporator to an oil; 438 mg, 100%.

### Part D:

### Butaneamide, 2-(acetylamino)-N-[3-[[[(1,1-dimethylethyl)amino]carbonyl](3-methylbutyl)amino]-2 -hydroxy-1-(phenylmethyl)propyl-3,3-dimethyl-, [1S-[1R*(R*), 2S*]-

The product from Part C (206 mg, 0.41 mmol) and N-methylmorpholine (45 uL, 0.41 mmol) were dissolved in CH₂Cl₂ (2.5 mL) and cooled to 0 C. Acetic anhydride (39 uL, 0.41 mmol) was then added and the reaction stirred 30 min at 0 C, then allowed to warm to ambient temperature and stir for 30 min. The solvent was removed on a rotary evaporator and the residue dissolved in ethanol (2 mL). The ethanolic solution was slowly poured into 50 % saturated NaHCO₃ (20 mL) and stirred vigorously. The solid was collected by suction filtration and washed with water, 5% citric acid, and again with water; 157 mg, 75%. CHN /1.5 H₂O requires: C 63.24, H, 9.67, N, 10.54; found:
C, 63.40, H, 9.41, N, 10.39.
Butaneamide, 2-amino-N-[3-[[[(1,1-dimethylethyl)amino]carbonyl](3-methylbutyl)amino]-2-hydroxy-1-(phenylmethyl)propyl-3,3-dimethyl-, [1S-[1R*(R*), 2S*]- was also capped with the acyl groups shown in Table 12.

**TABLE 12**

| |
|---|
| Acyl Group (R) |
| benzyloxycarbonyl |
| tert-butoxycarbonyl |
| acetyl |
| 2-quinoylcarbonyl |
| phenoxyacetyl |
| benzoyl |
| methyloxaloyl |
| pivaloyl |
| trifluoracetyl |
| bromoacetyl |
| hydroxyacetyl |
| morpholinylacetyl |
| N,N-dimethylaminoacetyl |
| N-benzylaminoacetyl |
| N-phenylaminoacetyl |
| N-benzyl-N-methylaminoacetyl |
| N-methyl-N-(2-hydroxyethyl)aminoacetyl |
| N-methylcarbamoyl |
| 3-methylbutyryl |
| N-isobutylcarbamoyl |
| succinoyl (3-carboxypropionyl) |
| carbamoyl |

### Example 24A

The procedure described below illustrates preparation of compounds of Formula III. Propanamide, N-[3-[[[(1,1-dimethylethyl)amino]carbonyl](3-methylbutyl)amino]-2-hydroxy-1-(phenylmethyl)propyl]-2-methyl-3-(2-phenylethylsulfonyl)-,[1S-[1R*(R*),2S*]] and its diastereomer.

### Part A

A solution of methyl methacrylate (7.25 g, 72.5 mmol) and phenethyl mercaptan (10.0 g, 72.5 mmol) in 100 mL of methanol was cooled in an ice bath and treated with sodium methoxide (100 mg, 1.85 mmol). The solution was stirred under nitrogen for 3 h and then concentrated *in vacuo* to give an oil that was taken up in ether and washed with 1 N aqueous potassium hydrogen sulfate, saturated aqueous sodium chloride, dried over anhydrous magnesium sulfate, filtered and concentrated to give 16.83 g, 97.5% of methyl 2-(R,S)-methyl-4-thia-6-phenyl hexanoate as an oil. TLC on SiO₂ eluting with 20:1 hexane:ethyl acetate (v:v) R_{f}=0.41.

### Part B

A solution of methyl 2-(R,S)-methyl-4-thia-6-phenyl hexanoate (4.00 g, 16.8 mmol) in 100 mL of dichloromethane was stirred at room temperature and treated portion wise with meta-chloroperoxybenzoic acid (7.38 g, 39.2 mmol) over approximately 40 m. The solution was stirred at room temperature for 16 h and then filtered and the filterate washed with saturated aqueous sodium bicarbonate, 1N sodium hydroxide, saturated aqueous sodium chloride, dried over anhydrous magnesium sulfate, filtered, and concentrated to give 4.50 g, 99% of desired sulfone. The unpurified sulfone was dissolved in 100 mL of tetrahydrofuran and treated with a solution of lithium hydroxide (1.04 g, 24.5 mmol) in 40 mL of water. The solution was stirred at room temperature for 2 m and then concentrated *in vacuo.* The residue was then acidified with 1N aqueous potassium hydrogen sulfate to pH=1 and then extracted three times with ethyl acetate. The combined ethyl acetate solution was washed with saturated aqueous sodium chloride, dried over anhydrous magnesium sulfate, filtered and concentrated to give a white solid. The solid was taken up in boiling ethyl acetate/hexane and allowed to stand undisturbed whereupon white needles formed that were isolated by filtration and air dried to give 3.38 g, 79% of 2-(R,S)-methyl-3(β-phenethylsulfonyl)-propionic acid, mp 91-93°C.

### Part C

A solution of 2-(R,S)-methyl-3(β-phenethylsulfonyl)-propionic acid (166.1 mg, 0.65 mmol), N-hydroxybenzotriazole (HOBT) (146.9 mg, 0.97 mmol), and 1-(3-dimethylaminopropyl)-3-ethylcarbodiimide hydrochloride (EDC) (145.8 mg, 0.75 mmol) in 4 mL of anhydrous dimethylformamide (DMF) cooled to 0°C and stirred under nitrogen for 0.5 h. This solution was then treated with 3-[[(dimethylethyl)amino]carbonyl](3-methylbutyl)amino-2(R)-hydroxy-1(S)-(phenylmethyl)propyl amine (201.9 mg, 0.59 mmol) and stirred at room temperature for 16 h. The solution was poured into 30 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 110.0 mg, 32% of (2R,3S)-3-[N-2-(R)-methyl-3-(β-phenethylsulfonyl)propionyl)amido-1-isoamyl-1-(tert-butylcarbamoyl)amino-4-phenyl-2-butanol and (2R,3S)-3-[N-2-(S)-methyl-3-(β-phenethylsulfonyl)propionyl]amido-1-isoamyl-1-(tert-butylcarbamoyl)amino-4-phenyl-2-butanol, FAB mass spectrum (MH⁺) =588. Flash chromatography of the mixture on silica gel eluting with 1:1 hexane:ethyl acetate afforded the separated diastereomers.

### Example 24B

### Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)- [1S-[1R*(R*), 2S*]], and its diastereomer.

### Part A

A solution of methyl 2-(bromomethyl)-acrylate (26.4 g, 0.148 mol) in 100 mL of methanol was treated with sodium methanesulfinate (15.1 g, 0.148 mol) portion wise over 10 m at room temperature. The solution was then stirred at room temperature for a period of 1.25 h and the solution concentrated *in vacuo.* The residue was then taken up in water and extracted four times with ethyl acetate. The combined ethyl acetate solution was washed with saturated sodium chloride, dried over anhydrous magnesium sulfate, filtered and concentrated to give a white solid, 20.7 g which was taken up in boiling acetone/methyl tert-butyl ether and allowed to stand whereupon crystals of pure methyl 2-(methylsulfonylmethyl) acrylate 18.0 g, 68% formed, mp 65-68 0°C.

### Part B

A solution of methyl 2-(methylsulfonylmethyl) acrylate (970 mg, 5.44 mmol) in 15 mL of tetrahydrofuran was treated with a solution of lithium hydroxide (270 mg, 6.4 mmol) in 7 mL of water. The solution was stirred at room temperature for 5 m and then acidified to pH=1 with 1 N aqueous potassium hydrogen sulfate and the solution extracted three times with ethyl acetate. The combined ethyl acetate solution was dried over anhydrous magnesium sulfate, filtered, and concentrated to give 793 mg, 89% of 2-(methylsulfonylmethyl) acrylic acid, mp 147-149 0°C.

### Part C

A solution of 2-(methylsulfonylmethyl) acrylic acid (700 mg, 4.26 mmol) in 20 mL of methanol was charged into a Fisher-Porter bottle along with 10% palladium on carbon catalyst under a nitrogen atmosphere. The reaction vessel was sealed and flushed five times with nitrogen and then five times with hydrogen. The pressure was maintained at 50 psig for 16 h and then the hydrogen was replaced with nitrogen and the solution filtered through a pad of celite to remove the catalyst and the filterate concentrated *in vacuo* to give 682 mg 96% of 2-(R,S)-methyl-3-methylsulfonyl propionic acid.

### Part D

A solution of 2-(R,S)-methyl-3(methylsulfonyl) propionic acid (263.5 mg, 1.585 mmol), N-hydroxybenzotriazole (HOBT) (322.2 mg, 2.13 mmol), and 1-(3-dimethylaminopropyl)-3-ethylcarbodiimide hydrochloride (EDC) (339.1 mg, 1.74 mmol) in 4 mL of anhydrous dimethylformamide (DMF) cooled to 0°C and stirred under nitrogen for 0,5 h. This solution was then treated with 3-[[(1,1-dimethylethyl)amino]carbonyl](3-methylbutyl)amino-2(R)-hydroxy-I(S)-(phenylmethyl)propyl amine (543.5 mg, 1.58 mmol) and stirred at room temperature for 16 h. The solution was poured into 60 mL of 60% saturated aqueous sodium bicarbonate solution. The aqueous solution was then decanted from the organic residue. The organic residue was taken up in dichloromethane and washed with 10% aqueous citric acid, brine, dried over anhydrous magnesium sulfate, filtered and concentrated to give 471.8 mg, 60% of Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*), 2S*]]- and its diastereomer.

### Example 25

### Preparation of Sulfone Inhibitors From L-(+)-S-acetyl-β-mercaptoisobutyric Acid

### Part A:

Propanamide, N-[3-[[[(1,1-dimethylethyl)amino]carbonyl](3-methylbutyl)amino]-2-hydroxy-1-(phenylmethyl)propyl]-2-methyl-3-S-acetyl)-[1S-[1R*),2S*]]-.

A round-bottomed flask was charged with (2R,3R)-3-amino-1-isoamyl-1-(tert-butylcarbamoyl)amino-4-phenyl-2-butanol (901.5 mg, 2.575 mmol), L-(+)-S-acetyl-b-mercaptoisobutyric acid (164.5 mg, 2.575 mmol), 1-(3-dimethylaminopropyl)-3-ethylcarbodiimide hydrochloride (EDC) (339.1 mg, 1.74 mmol), and 10 mL of CH₂Cl₂ and allowed to stir at room temperature for 16 h. The solution was concentrated in vacuo and the residue taken up in ethyl acetate, washed with 1N KHSO₄ sat. aq. NaHCO₃, brine, dried over anhydrous MgSO₄, filtered and concentrated to give an oil that was purified by radial chromatography on SiO₂ eluting with ethyl acetate to give the pure product, 800 mg, 63%.

### Part B:

Propanamide, N-[3-[[[1,1-dimethylethyl)amino]carbonyl](3-methylbutyl)amino]-2-hydroxy-1-(phenylmethyl)propyl]-2-methyl-3-mercapto)-, [1S-[1R*(R*),2S*]]-.

A solution of [1S-[1R*(R*),2S*]]-N-[3-[[[(1,1-dimethylethyl)amino]carbonyl](3-methylbutyl)amino]-2-hydroxy-1-(phenylmethyl)propyl]-2-methyl-3-S-acetyl)-propanamide (420 mg, 0.85 mmol) in 10 mL of methanol was treated with anhydrous ammonia for ca. 1 m at 0°C. The solution was stirred at that temperature for 16 h and then concentrated in vacuo to give 380 mg, 99%, of the desired product that was used directly in the next step without further purification.

### Part C:

Propanamide, N-[3-[[[(1,1-dimethylethyl)amino]carbonyl](3-methylbutyl)amino]-2-hydroxy-1-(phenylmethyl)propyl]-2-methyl-3-S-methyl-, [1S-[1R*(R*),2S*]]-.

A solution of [1S-[1R*(R*),2S*]]- N-[3-[[[(1,1-dimethylethyl)amino]carbonyl](3-methylbutyl)amino]-2-hydroxy-1-(phenylmethyl)propyl]-2-methyl-3-mercapto)-propanamide (380 mg, 0.841 mmol) in 10 mL of dry toluene under nitrogen was treated in rapid succession with 1,8-diazabicyclo[5.4.0]undec-7-ene, (DBU), (128.1 mg. 0.841 mmol) and iodomethane (119.0 mg, 0.841 mmol). After 0.5 h at room temperature the reaction was found to be complete and the solution was diluted with ethyl acetate washed with 1N KHSO₄, sat. aq. NaHCO₃, brine. After the solution was dried over anhydrous MgSO₄, filtered and concentrated in vacuo the desired product was obtained as white foam was obtained, 370 mg, 94.5%, that was used directed in the next step.

### Part D:

Propanamide, N-[3-[[[(1,1-dimethylethyl)amino]carbonyl](3-methylbutyl)amino]-2-hydroxy-1-(phenylmethyl)propyl]-2-methyl-3-(methylsulfonyl)-, [1S-[1R*(R*),2S*]]-.

A solution of [1S-[1R*(R*),2S*]]-N-[3-[[[(1,1-dimethylethyl)amino]carbonyl](3-methylbutyl)amino]-2-hydroxy-1-(phenylmethyl)propyl]-2-methyl-3-S-methyl)-propanamide (340 mg, 0.73 mmol) and sodium perborate (500 mg, 3.25 mmol) in 30 mL of glacial acetic acid was warmed to 55°C for 16 h. The solution was concentrated in vacuo and then the residue taken up in ethyl acetate, washed with water, sat. aq. NaHCO₃, brine, dried over anhydrous MgSO₄, filtered and concentrated to give the desired product as a white solid, 350 mg, 96%.

### Example 26

The compounds shown in Table 12 was prepared generally according to the procedure set forth in Examples 24 and 25.

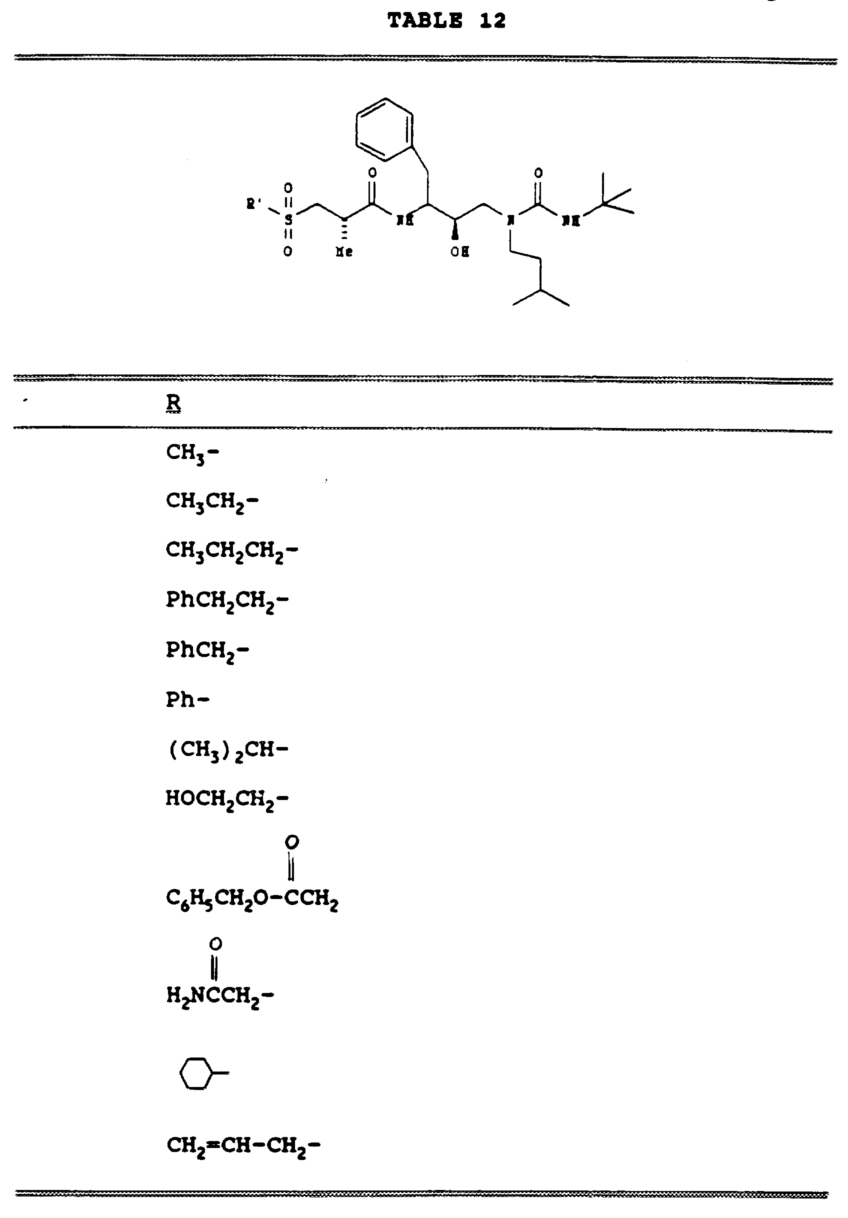

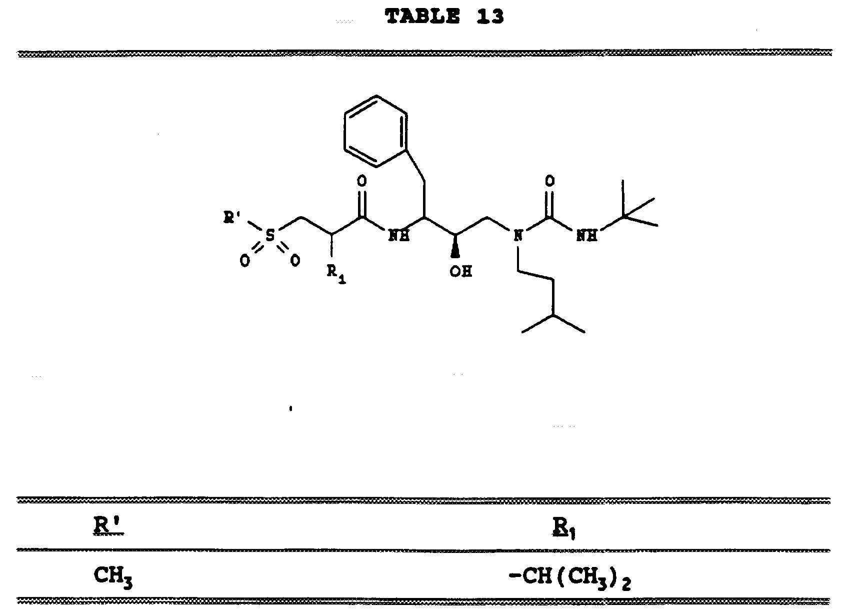

### Example 27

### Preparation of 2(S)-methyl-3-(methylsulfonyl)propionic Acid.

To a solution of 10g of D-(-)-S-benzoyl-b-mercaptioisobutyric acid t-butyl ester in 20 mL of methanol was bubbled in gaseous ammonia at 0°C. The reaction was allowed to then warm to room temperature, stirred overnight and concentrated under reduced pressure. The resulting mixture of a solid (benzamide) and liquid was filtered to provide 5.21g of a pale oil which then solidified. This was identified as 2(S)-methyl-3-mercaptopropionic aid t-butyl ester.

To a solution of 5.21g of 2(S)-methyl-3-mercaptopropionic acid t-butyl ester in 75 mL of toluene at 0°C was added 4.50g of 1,8-diazabicyclo [5.40] undec-7-ene and 1.94 mL of methyl iodide. After stirring at room temperature for 2.5 hours, the volatiles were removed, ethyl acetate added, washed with dilute hydrochloric acid, water, brine, dried and concentrated to afford 2.82g of a pale oil, identified as 2(S)-methyl-3-(thiomethyl)propionic acid t-butyl ester.

To a solution of 2.82g of 2(S)-methyl-3-(thiomethyl)propionic acid t-butyl ester in 50 mL of acetic acid was added 5.58g of sodium perborate and the mixture heated to 55°C for 17 hours. The reaction was poured into water, extracted with methylene chloride, washed with aqueous sodium bicarbonate, dried and concentrated to afford 2.68g of 2(S)-methyl-3-(methylsulfonyl)propionic acid t-butyl ester as a white solid.

To 2.68g of 2(S)-methyl-3-(methylsulfonyl)propionic acid t-butyl ester was added 20 mL of 4N hydrochlorid acid/dioxane and the mixture stirred at room temperature for 19 hours. The solvent was removed under reduced pressure to afford 2.18g of crude product, which was recrystallized from ethyl acetate/hexane to yield 1.44g of 2(S)-methyl-3-(methylsulfonyl)propionic acid as white crystals.

### Example 28

This example illustrates preparation of compounds of Formula IV wherein t is 1.

### 4-N-benzyl itaconamide.

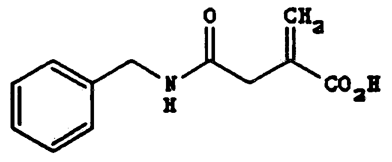

A 500 mL three necked round bottomed flask equipped with a dropping funnel, mechanical stirrer, nitrogen inlet and reflux condenser was charged with itaconic anhydride (33.6g, 0.3 mol) and 150 mL of toluene. This solution was added a solution of benzylamine (32.1g, 0.3 mol) in 50 mL of toluene dropwise over 30 m at room temperature. The solution was stirred at this temperature an additional 3h and then the solid product isolated by filtration on a Büchner funnel. The crude product, 64.6g 98%, was recrystallized from 300 mL of isopropyl alcohol to give after two crops 52.1g, 79% of pure product, mp 149-150°C

### 2(R)-Methyl 4-N-benzyl succinamide.

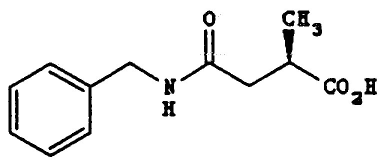

A large Fisher-Porter bottle was charged with the acid from the above reaction (10.95g, 0.05 mol), rhodium (R,R)-DiPAMP (220mg, 0.291 mmol) and 125 mL of degassed methanol. The solution was then hydrogenated at 40 psig for 16h at room temperature. After the hydrogen uptake ceased, the vessel was opened and the solution concentrated *in vacuo* to give a yellow solid, 11.05g, 100%. The product was then taken up in absolute ethanol and allowed to stand whereupon crystals of the desired product formed, 7.98g, 72%, mp 127-129 °C [a]_{D} @ 25 °C=+14.9° (c=1.332, EtOH), ¹H nmr (CDCl₃) 300MHz 7.30(m,5H), 6.80(brs, 1H), 4.41(d, J=5.8Hz, 2H), 2.94(m, 1H), 2.62(dd, J=8.1, 14.9Hz, 1H), 2.33(dd, J=5.5, 14.9Hz, 1H), 1.23(d, J=7.2Hz, 3H).

### 4-N(4-methoxybenzyl)itaconamide.

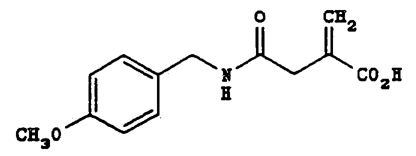

A 500 mL three necked round bottomed flask equipped with a dropping funnel, mechanical stirrer, nitrogen inlet and reflux condenser was charged with itaconic anhydride (44.8g, 0.4 mol) and 150 mL of toluene. This solution was added a solution of 4-methoxybenzylamine (54.8g, 0.4 mol) in 50 mL of toluene dropwise over 30 m at room temperature. The solution was stirred at this temperature an additional 2h and then the solid product isolated by filtration on a Büchner funnel. The crude product was recrystallized from ethyl acetate/ethanol to give after two crops 64.8g, 65% of pure product, mp 132-134 °C, ¹H nmr (CDCl₃) 300MHz 7.09(d, J=9.lHz, 2H), 6.90(brt, J=5.9Hz, 1H), 6.74(d, J=9.1Hz, 2H), 6.22(s, 1H), 5.69(s, 1H), 4.24(d, J=5.9Hz, 2H), 3.69(s, 3H), 3.15(s, 2H). ¹³C nmr (CDCl₃) 170.52, 169.29, 159.24, 135.61, 131.08, 129.37, 128.97, 114.36, 55.72, 43.37, 40.58.

### 2(R)-Methyl 4-N(4-methoxybenzyl)succinamide.

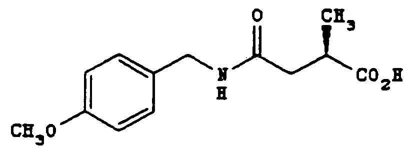

A large Fisher-Porter bottle was charged with the acid from the above reaction (5.00 g, 0.02 mol), rhodium (R,R)-DiPAMP (110 mg, 0.146 mmol) and 50 mL of degassed methanol. The starting acid was not completely soluble initially, but as the reaction progressed the solution became homogeneous. The solution was then hydrogenated at 40 psig for 16h at room temperature. After the hydrogen uptake ceased, the vessel was opened and the solution concentrated *in vacuo* to give a yellow solid. The crude product was then taken up in ethyl acetate and washed three times with sat. aq. NaHCO₃ solution. The combined aqueous extracts were acidified to pH=1 with 3 N HCl and then extracted three times with ethyl acetate. The combined ethyl acetate extracts were washed with brine, dried over anhyd. MgSO₄, filtered and concentrated to give the expected product as a white solid, 4.81g, 95%. This material was recrystallized from a mixture of methyl ethyl ketone/hexane to give 3.80g, 75% of pure product, [a]_{D} @ 25 °C=+11.6° (c=1.572, MeOH). ¹H nmr (CDCl₃) 300MHz 11.9(brs, 1H), 7.18(d, J=9.2Hz, 2H), 6.82(d, J=9.2Hz, 2H), 6.68(brt, J=5.6Hz, 1H), 4.33(d, J=5.6Hz, 2H), 3.77(s, 3H), 2.92(ddq, J=7.9, 5.4, 7.3Hz, 1H), 2.60(dd, J=5.4, 15.0Hz, 1H), 2.30(dd, J=7.9, 15.0Hz, 1H),1.22(d, J=7.3Hz, 3H).

### Butanediamide, N'-[3-[[[(1,1-dimethylethyl)amino]carbonyl](3-nethylbutyl)amino]-2-hydroxy-1-(phenylmethyl)propyl]-N-4-methoxyphenylmethyl-2-methyl, [1S-[1R*(2R*),2S*]]-

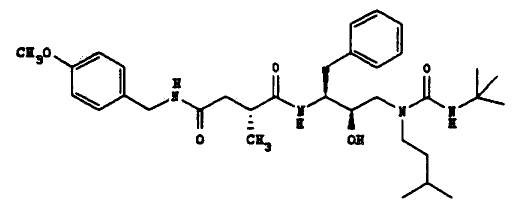

A 50 mL round bottomed flask was charged with 2(R)-methyl 4-N(4-methoxybenzyl)succinamide (588 mg, 2.35 mmol), N-hydroxybenzotriazole (511 mg, 3.34 mmol) and 6 mL of DMF. The solution was cooled to 0°C and treated with 1-(3-dimethylaminopropyl)-3-ethylcarbodiimide hydrochloride (502 mg, 2.62 mmol) for 20 m. A solution of (2R,3S)-3-amino-1-(3-methylbutyl)-1-[(1,1-dimethylethyl)amino]carbonyl)-4-phenyl-2-butanol (782 mg, 2.24 mmol) in 2 mL of DMF was added and the solution stirred at room temperature for a period of 24 h. The solution was concentrated *in vacuo* and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by radial chromatography on SiO₂ eluting with hexane/ethyl acetate to give 790 mg, 59% of pure product as a white foam.

### Butanediamide, N'-[3-[[[(1,1-dimethylethyl)amino]carbonyl](3-methylbutyl)amino]-2-hydroxy-1-(phenylmethyl)propyl]-N-phenylmethyl-2-methyl, [1S-[1R* (2R*) ,2S*]]-

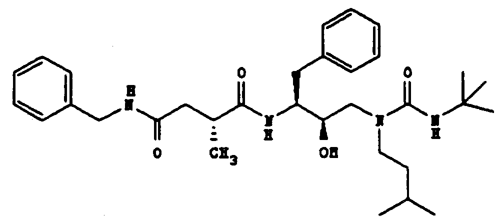

A 50 mL round bottomed flask was charged with 2(R)-methyl 4-N-(benzyl) succinamide (243 mg, 1.1 mmol), N-hydroxybenzotriazole (213 mg, 1.39 mmol) and 3 mL of DMF. The solution was cooled to 0° C and treated with 1-(3-dimethylaminopropyl)-3-ethylcarbodiimide hydrochloride (228 mg, 1.17 mmol) for 20 m. A solution of (2R,3S)-3-amino-1-(3-methylbutyl)-1-[(1,1-dimethylethyl)amino]carbonyl)-4-phenyl-2-butanol (327 mg, 0.95 mmol) in 2 mL of DMF was added and the solution stirred at room temperature for a period of 24 h. The solution was concentrated *in vacuo* and poured into 50 mL of 50% sat. aq. NaHCO₃, the aqueous phase was extracted with CH₂Cl₂. The organic phase was washed with 5% citric acid, NaHCO₃, brine, dried over anhyd. MgSO₄, filtered and concentrated to give an oil that was purified by flash chromatography on SiO₂ eluting with hexane/ethyl acetate to give 370 mg, 70% of pure product as a white foam.

### Example 29

Following the procedure generally as set forth in Example 28, the compounds shown in Table 14 were prepared.

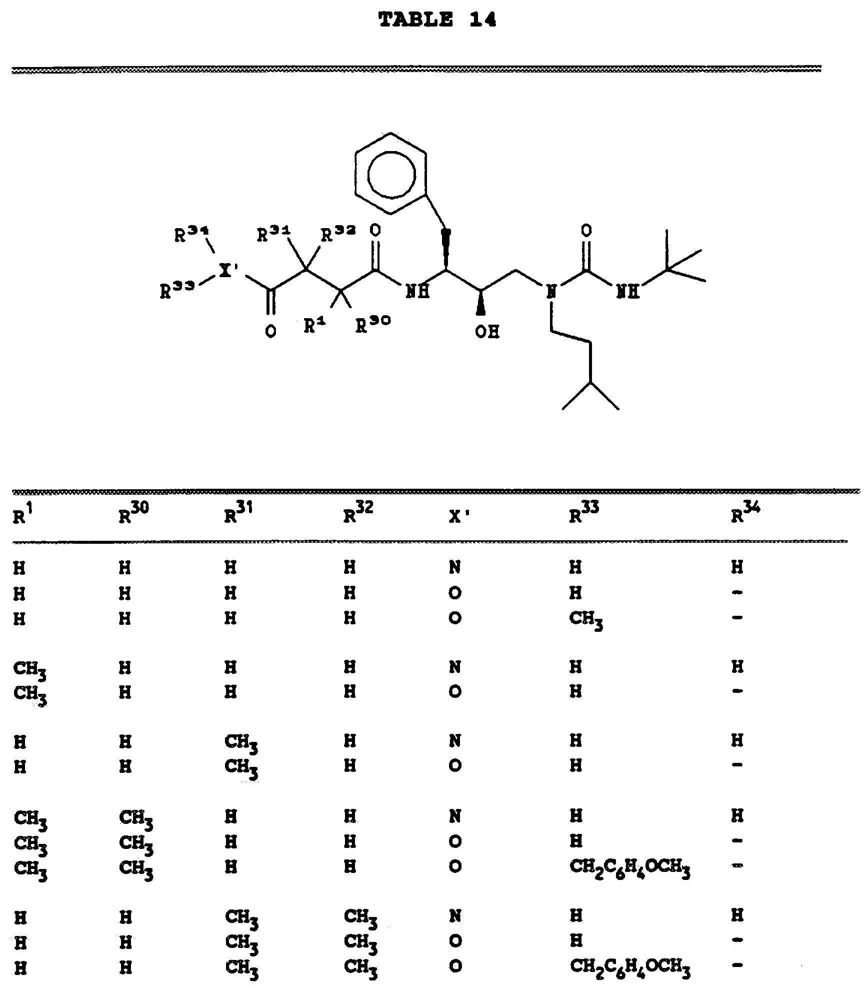

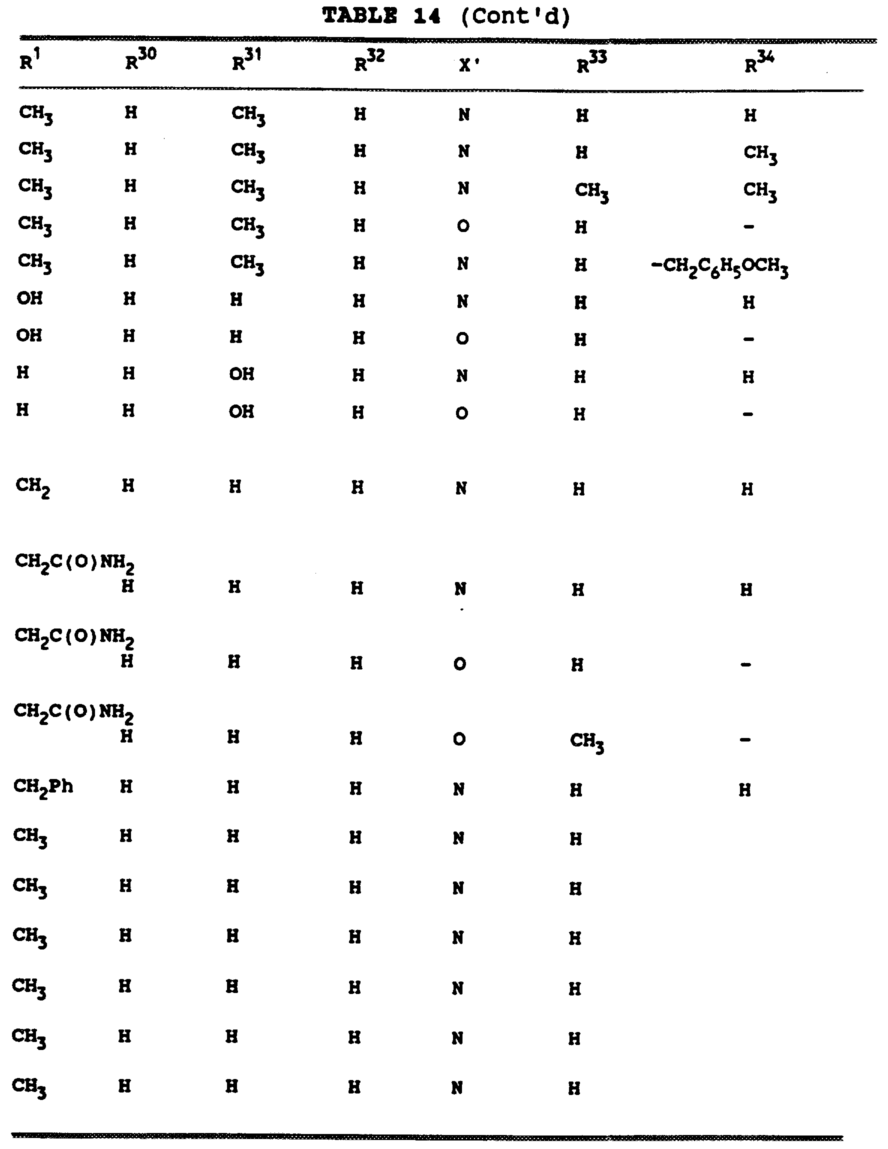

### Example 30

Following the procedure generally as set forth in Example 28, the compounds shown in Table 15 were prepared.

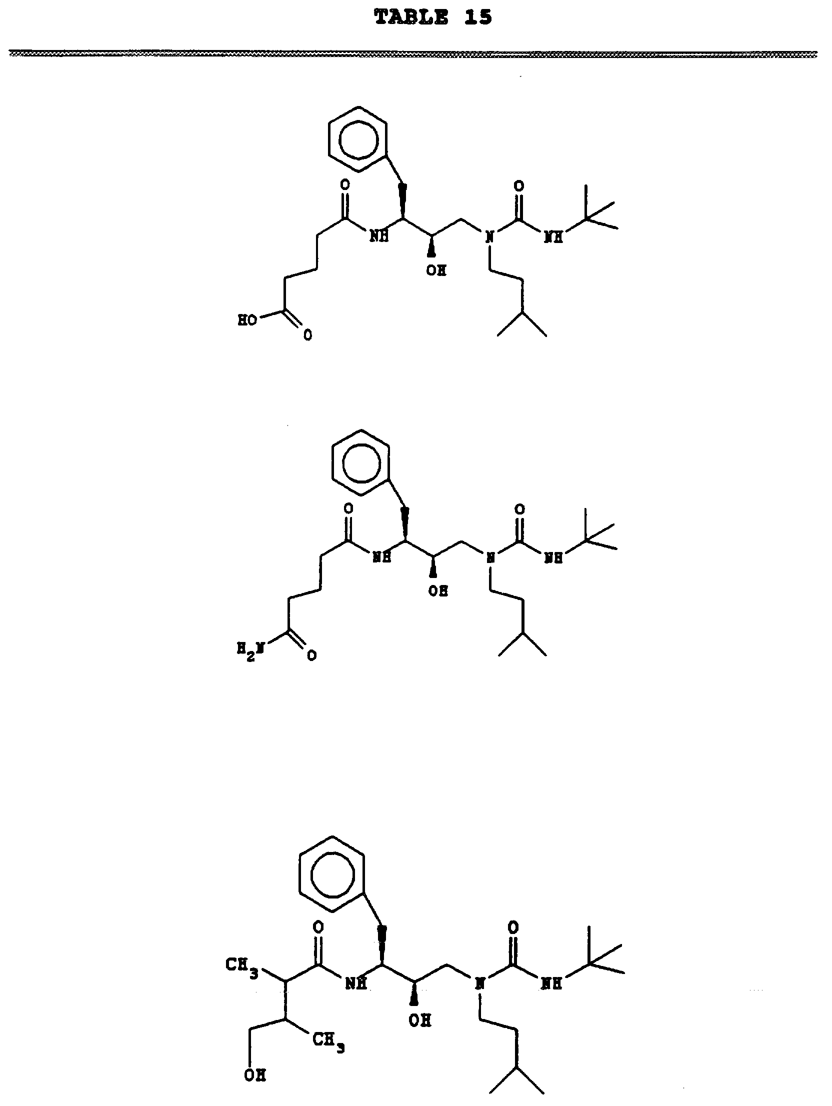

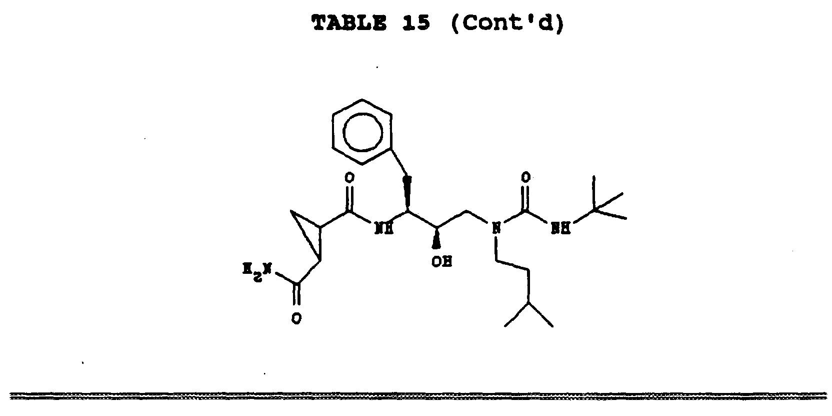

### Example 31

### Preparation of 3 (S)-[N-(2-quinolinylcarbonyl)-L-asparaginyl]amino-2(R)-hydroxy-4-phenylbutylamine, N-(3-methylbutyl).

### Part A:

Preparation of N-3(S)-(Benzyloxycarbonyl)-amino-2(R)-hydroxy-4-phenylbutylamine, N-(3-methylbutyl). A solution of 20g (67 mmol) of N-benzyloxycarbonyl-3(S)-amino-1,2-(S)-epoxy-4-phenylbutane in 140 mL of isopropyl alcohol was treated with 83g (952 mmol) of isoamylamine and refluxed for one hour. The solution was cooled, concentrated, hexane added and the resulting solid filtered to afford 22.4g of the desired product.

### Part B:

Preparation of N-3(S)-(Benzyloxycarbonyl)-amino-2(R)-hydroxy-4-phenylbutylamine, N-(3-methylbutyl)-N-(t-butyloxycarbonyl). To a solution of 22.4g (58.3 nmol) of product from Part A above, 6.48g (64.1 mmol) of triethylamine and 150 mg of N,N-dimethyl-4-aminopyridine in 200 mL of tetrahydrofuran at 0°C was added 12.7g (58.3 mmol) of di-t-butylpyrocarbonate in 10 mL of THF. After 3.5 hours at room temperature, the volatiles were removed, ethyl acetate added and washed with 5% citric acid, sat d NaHCO₃, dried and concentrated to afford 30g of crude product. Chromatography on silica gel using 20% ethyl acetate/hexane afforded 22.5g (79%) of the desired product.

### Part C:

Preparation of N-3(S)-[N-benzyloxycarbonyl-L-asparaginyl]amino-2(R)-hydroxy-4-phenylbutylamine, N-(3-methylbutyl)-N-(t-butyloxycarbonyl). A solution of 22.5g of product from Part B above in 200 mL of ethanol was hydrogenated over 5.9g of 10% palladium-on-carbon under 50 psig hydrogen for one hour. The catalyst was filtered and the solvent removed under reduced pressure to afford 15.7g of free amine. This was dissolved in 130 mL of DMF and 4.54g (44.9 mmol) of N-methylmorpholine an added to a mixture of 13.3g (49.9 mmol) N-benzyloxy-carbonyl-L-asparagine, 11.5g (74.9 mmol) of N-hydroxybenzotriazole and 10.5g (54.9 mmol) of EDCl in 120 mL of DMF at 0°C, which had been preactivated for one hour prior to the addition. The mixture was stirred for 2 hours at 0°C and then for 12 hours at room temperature. The reaction was poured into 1L of sat d aqueous sodium bicarbonate, the solid collected, dissolved in ethyl acetate, washed with water, sat d sodium bicarbonate, 5% citric acid and brine, dried and concentrated to afford 16.7g of the desired product.

### Part D:

Preparation of N-3(S)-[N-(2-quinolinylcarbonyl)-L-asparaginyl]amino-2(R)-hydroxy-4-phenylbutylamine, N-(3-methylbutyl)-N-(t-butyloxycarbonyl). A solution of 16.7g (28.0 mmol) of product from Part C in 250 mL of methanol was hydrogenated over 6.0g of 10% palladium-on-carbon and under 50 psig hydrogen for one hour. The catalyst was filtered and the solution concentrated to afford 10.0g of free amine. This was dissolved in 100 mL of methylene chloride, 4.35g (43 mmol) of N-methylmorpholine was added followed by 5.53g (20.5 mmol) of quinoline-2-carboxylic acid, N-hydroxysuccinimide ester. This was stirred at room temperature overnight, the solvent removed, ethyl acetate added and washed with 5% citric acid, sat d sodium bicarbonate, brine, dried and concentrated to afford 14g of crude product. Recrystallization from ethyl acetate and hexane afforded 10.5g (83%) of desired product.

### Part E:

Preparation of N-3(S)-[N-(2-quinolinylcarbonyl)-L-asparaginyl]amino-2(R)-hydroxy-4-phenylbutylamine, N-(3-methylbutyl). To 80 mL of 4N hydrochloric acid in dioxane was added 9.17g (14.8 mmol) of product from Part D above. After one hour, the product becomes gummy. The solvents were removed, diethyl ether added and removed and the residue dissolved in 20 mL of methanol. This solution was added to 400 mL of sat d aqueous sodium bicarbonate, the solids collected, washed with acetone and hexane and dried in vacuo over P₂O₅ to afford 4.75g of the desired product.

### Example 32A

### Preparation of Benzyl 2,2,3(R)-trimethylsuccinate

### Part A:

Preparation of Methyl (S)-lactate, 2-methoxy-2-propyl ether. To a mixture of methyll(s)-(-)-lactate (13.2g, 100 mmol) and, 2-methoxypropene (21.6g, 300 mmol) in CH₂Cl₂ (150 ml) was added POCl₃ (7 drops) at r.t. and the resulting mixture was stirred at this temperature for 16 hours. After the addition of Et₃N (10 drops), the solvents were removed in vacuo to give 20.0g of (98%) desired product.

### Part B:

Preparation of 2(S)-hydroxypropanal, 2-methoxy-2-propyl ether. To a solution of compound from Part A (20.0g) in CH₂Cl₂ (100 ml) was added DIBAL (65 ml of 1.5M solution in toluene, 97.5 mmol) dropwise at-78°C for 45 min., then stirring was continued at the temperature for another 45 min. To this cold solution was added MeOH (20 ml), saturated NaCl solution (10 ml) and allowed the reaction mixture to warm up to r.t. and diluted with ether (200 ml), MgSO₄ (150g) was added and stirred for another 2 h. The mixture was filtered and the solid was washed twice with ether. The combined filtrates were rotavaped to afford 11.2g (78%) of the desired aldehyde.

### Part C:

Preparation of 2(S)-hydroxy-cis-3-butene, 2-methoxy-2-propyl ether. To a suspension of ethyltriphenylphosphonium bromide (28g, 75.5 mmol) in THF (125 ml) was added KN (TMS)₂ (15.7g, 95%, 75 mmol) in portions at 0°C and stirred for 1 h at the temperature. This red reaction mixture was cooled to -78°C and to this was added a solution of aldehyde from Part B (11g, 75 mmol) in THF (25 ml). After the addition was completed, the resulting reaction mixture was allowed to warm up to r.t. and stirred for 16 h. To this mixture was added saturated NH₄Cl (7.5 ml) and filtered through a pad of celite with a thin layer of silica gel on the top. The solid was washed twice with ether. The combined filtrates were concentrated in vacuo to afford 11.5g of crude product. The purification of crude product by flash chromatography (silica gel, 10:1 Hexanes/EtoAc) affording 8.2g (69%) pure alkene.

### Part D:

Preparation of 2(S)-hydroxy-cis-3-butene. A mixture of alkene from Part C (8.2g) and 30% aqueous acetic acid (25 ml) was stirred at r.t. for 1 hour. To this mixture was added NaHCO₃ slowly to the pH ∼ 7, then extracted with ether (10 ml x 5). The combined ether solutions were dried (Na₂SO₄) and filtered. The filtrate was distilled to remove the ether to give 2.85g (64%) pure alcohol, m/e=87(M+H).

### Part E:

Preparation of 2,2,3( )-trimethyl-hex-(trans)-4-enoic acid. To a mixture of alcohol from Part D (2.5g, 29 mmol) and pyridine (2.5 ml) in CH₂Cl₂ (60 ml) was added isobutyryl chloride (3,1g, 29 mmol) slowly at 0°C. The resulting mixture was stirred at r.t. for 2 hours then washed with H₂O (30 ml x 2) and sat. NaCl (25 ml). The combined organic phases were dried (Na₂SO₄), concentrated to afford 4.2g (93%) ester 2(S)-hydroxy-cis-3-butenyl isobutyrate. This ester was dissolved in THF (10 ml) and was added to a 1.0M LDA soln. (13.5 ml of 2.0M LDA solution in THF and 13.5 ml of THF) slowly at -78°C. The resulting mixture was allowed to warm up to r.t. and stirred for 2 h and diluted with 5% NaOH (40 ml). The organic phase was separated, the aqueous phase was washed with Et₂O (10 ml). The aqueous solution was collected and acidified with 6N HCl to pH ∼ 3. The mixture was extracted with ether (30 ml x 3). The combined ether layers were washed with sat. NaCl (25 ml), dried (Na₂SO4) and concentrated to afford 2.5g (60%) of desired acid, m/e=157(M+H).

### Part F:

Preparation of benzyl 2,2,3(S)-trimethyltrans-4-hexenoate.A mixture of acid from Part E (2.5g, 16 mmol), BnBr (2.7g, 15.8 mmol), K₂CO₃ (2.2g, 16 mmol), NaI (2.4g) in acetone (20 ml) was heated at 75°C (oil bath) for 16 h. The acetone was stripped off and the residue was dissolved in H₂O (25 ml) and ether (35 ml). The ether layer was separated, dried (Na₂SO₄) and concentrated to afford 3.7g (95%) of benzyl ester, m/e=247(M+H).

### Part G:

Preparation of benzyl 2,2,3(R)-trimethylsuccinate. To a well-stirred mixture of KMₙO₄ (5.4g, 34, 2 mmol), H₂O (34 ml), CH₂Cl₂ (6 ml) and benzyltriethylammonium chloride (200 mg) was added a solution of ester from Part F (2.lg, 8.54 mmol) and acetic acid (6 ml) in CH₂Cl₂ (28 ml) slowly at 0°C. The resulting mixture was stirred at the temperature for 2 h then r.t. for 16 h. The mixture was cooled in an ice-water bath, to this was added 6N HCl (3 ml) and solid NaHSO₃ in portions until the red color disappeared. The clear solution was extracted with CH₂Cl₂ (30 ml x 3). The combined extracts were washed with sat. NaCl solution, dried (Na₂SO₄) and concentrated to give an oil. This oil was dissolved in Et₂O (50 ml) and to this was added sat. NaHCO₃ (50 ml). The aqueous layer was separated and acidified with 6N HCl to pH ∼ 3 then extracted with Et₂O (30 ml x 3). The combined extracts were washed with sat. NaCl solution (15 ml), dried (Na₂SO₄) and concentrated to afford 725 mg (34%) of desired acid, benzyl 2,2,3(R)-trimethylsuccinate, m/e=251(M+H).

### Example 32B

### Part A:

### Preparation of Butanediamide, N'-[3-[[[(1,1-dimethylethyl)amino]carbonyl](3-methylbutyl)amino]-2 -hydroxy-1-(phenylmethyl)propyl]-2,3,3-trimethyl-[1s-, [1R* (2S*),2S*]]-

To a well-stirred solution of acid benzyl 2,2,3(R)-trimethylsuccinate (225 mg, 0.9 mmol) in DMF (1.0 ml) was added HOBt (230 mg, 1.5 mmol). The clear reaction mixture was then cooled to 0°C, to this was added EDC (210 mg, 1.1 mmol) and stirred for 1 h at the temperature. To this cold mixture was added a powder of (350 mg, 1.0 mmol) and DMF (0.5 ml). The resulting reaction mixture was stirred for 2 h at 0°C and 16 h at r.t. After the removal of DMF (≤ 40°C), a solution of 60% sat. NaHCO₃ (10 ml) was added. This mixture was extracted with EtOAc (10 ml x 2). The extracts were combined and washed with sat. NaHCO₃ (10 ml x 2), 5% citric acid (10 ml x 2), H₂O (10 ml), sat. NaCl (10 ml) and dried (Na₂SO₄) then concentrated to afford 512 mg (98%) of desired product Butanoic Acid, 4-[[3-[[[(1,1-dimethylethyl)amino]carbonyl](3-methylbutyl)amio]-2-hydroxy-1-(phenylmethyl)propyl]amino]-2,2,3-trimethyl4-oxo, [1S-[1R*(3S*),2S*]]-benzyl ester as a white solid, m/e=582(M+H).

### Part B:

A mixture of benzyl ester 10 (480 mg, 0.825 mmol), 10% Pd/C (450 mg) in MeOH (25 ml) was hydrogenated (H₂, 50 psi) for 1/2 h at r.t. The mixture was filtered and the solid was washed with MeOH (10 ml). The collected filtrates were concentrated to afford a crude acid as a white solid. The crude acid was dissolved in Et₂O-EtOAc (10:1, 25 ml) and the solution was washed with sat. NaHCO₃ (25 ml) then 5% NaOH (10 ml). The combined aqueous layers were cooled to 0°C and acidified with concentrated HCl (Co₂) to pH ∼ 1 then extracted with Et₂O-EtOAC (10:1, 25 ml x 3). The combined extracts were washed with sat. NaCl (15 ml), dried (Na₂SO₄) and concentrated to afford 307 mg (75.7%) of pure acid Butanoic acid, 4-[[3-[[[(1,1-dimethylethyl)amino]carbonyl](3-methylbutyl)amino]-2-hydroxy-1-(phenylmethyl)propyl]amino]-2,2,3-trimethyl-4-oxo-,[1S-[1R*(3S*),2S*]]-, as a white solid, m/e=491(M+H).

### Part C:

### Butanoic acid, 4-[[3-[[[(1,1-dimethylethyl)amino]carbonyl](3-methylbutyl)amino]-2-hydroxy-1-(phenylmethyl)propyl]amino]-2,2,3-trimethyl-4-oxo-,[1S-[1R*(3S*),2S*]]-, as a white solid, m/e=491(M+H).

To a well-stirred solution of the acid 11 (245 mg, 0.5 mmol) in DMF (0.5 ml) was added HOBt (153 mg, 1.0 mmol) and EDC (143 mg, 0.75 mmol) at 0°C. After stirring at 0°C for 2 h, NH₄OH (0.63 ml of 28% NH₄OH, 5 mmol) was added and stirred at 0°C for 2 h, r.t. for 16 h. The removal of DMF (≤ 40°C) gave a white solid. The purification of the crude product by flash chromatography (silica gel, 5% MeOH/CH₂Cl₂) gave 172 mg (70%) of pure amide 12 as a white solid, m/e=491(M+H).

### Example 33

### Preparation of methyl 2,2-dimethyl-3-methyl succinate, (R) and (S) isomers.

### Part A:

Preparation of methyl 2,2-dimethyl-3-oxo-butanoate. A 250 ml RB flask equipped with magnetic stir bar and N₂ inlet was charged with 100 ml dry THF and 4.57g (180 mmol) of 95% NaH. The slurry was cooled to-20°C and 10g (87 mmol) methyl acetoacetate was added dropwise followed by 11.3 ml (181 mmol) CH₃I. The reaction was stirred at 0°C for 2 hours and let cool to room temperature overnight. The reaction was filtered to remove NaI and diluted with 125 ml Et₂O. The organic phase was washed with 1x100 l 5% brine, dried and concentrated in vacuo to a dark golden oil that was filtered through a 30g plug of silica gel with hexane. Concentration in vacuo yielded 10.05g of desired methyl ester, m/e= ? as a pale yellow oil, suitable for use without further purification.

### Part B:

Preparation of methyl 2,2-dimethyl-3-0-(trifluoromethanesulfonate)-but-3-enoate. A 250 ml RB flask equipped with magnetic stir bar and N₂ inlet was charged with 80 l by THF and 5.25 ml (37.5 mmol) diisopropylamine was added. The solution was cooled to -25°C (dry ice/ethylene glycol) and 15 ml (37.5 mmol) of 2.5 M nbuLi in hexanes was added. After 10 minutes a solution of 5g (35 mmol) 1 in 8 ml dry THF was added. The deep yellow solution was stirred at -20°C for 10 min. then 12.4g N-phenyl bis(trifluoromethanesulfonimide) (35 mmol) was added. The reaction was stirred @ -10°C for 2 hours, concentrated in vacuo and partioned between EA and sat. bicarb. The combined organic phase was washed with bicarb, brine and conc. to an amber oil that was filtered through 60g silica gel plug with 300 1 5% EA/H. Conc. in vacuo yielded 9.0g light yellow oil that was diluted with 65 ml EA and washed with 2x50 ml 5% aq K₂CO₃, 1x10 l brine, dried over Na₂SO₄ and conc. in vacuo to yield 7.5g (87%) vinyl triflate, (m/e=277(M+H) suitable for use without further purification.

### Part C:

Preparation of methyl 2,2-dimethyl-3-carboxyl-but-3-enoate. A 250 ml Fisher Porter bottle was charged with 7.5g (27 mmol) 2, 50 ml dry DMF, 360 mg (1.37 mmol) triphenyl phosphine and 155 mg (.69 mmol) Pd^{II}(OAc)₂. The reaction mixture was purged twice with N₂ then charged with 30 psi CO. Meanwhile a solution of 20 ml dry DMF and 7.56 ml (54 mmol) NEt₃ was cooled to 0°C to this was added 2.0g (43 mmol) of 99% formic acid. The mixture was swirled and added to the vented Fisher Porter tube. The reaction vessel was recharged to 40 psi of CO and stirred 6 hours @ room temperature. The reaction mixture was concentrated in vacuo and partionned between 100 l EA/75 ml 5% aq K₂CO₃. The aqueous phase was washed with 1x40 l additional EA and then acidified with conc. HCl/ice. The aqueous phase was extracted with 2x70 l EA and the organics were dried and conc. to yield 3.5g (75%) white crystals, mp 72-75°C, identified as the desired product (m/e=173(M+H).

### Part D:

Preparation of methyl 2,2-dimethyl-3-methylsuccinate, isomer #1. A steel hydrogenation vessel was charged with 510 mg (3.0 mmol) acrylic acid, 3, and 6 mg Ru (acac)₂ (R-BINAP) in 10 ml degassed MeOH. The reaction was hydrogenated at 50 psi/room temperature for 12 hours. The reaction was then filtered through celite and conc. to 500 mg clear oil which was shown to be a 93:7 mixture of isomer #1 and #2, respectively as determined by GC analysis using a 50 M β-cyclodextrin column: 150°C - 15 min. then ramp 2°C/min.; isomer #1, 17.85 min., isomer #2, 18-20 min.

### Part E:

Preparation of methyl 2,2-dimethyl-3-methylsuccinate, Isomer #2. A steel hydrogenation vessel was charged with 500 mg (2.9 mmol) acrylic acid, 3, and 6 mg Ru(OAc) (acac)(S-BINAP) in 10 ml degassed MeOH. The reaction was hydrogenated at 50 psi/room temperature for 10 hours. The reaction was filtered through celite and concentrated in vacuo to yield 490 mg of product as a 1:99 mixture of isomers #1 and #2, respectively, as determined by chiral GC as above.

### Example 34

### Preparation of 3-[[[(1,1-dimethylethyl)amino]carbonyl](3-methylbutyl)amino]-2(R) -hydroxy-1(S)-(phenylmethyl)propylamine, 1.

### Part A:

To a solution of 75.0g (0.226 mol) of N-benzyloxycarbonyl-L-phenylalanine chloromethyl ketone in a mixture of 807 mL of methanol and 807 mL of tetrahydrofuran at -2°C, was added 13.17g (0.348 mol, 1.54 equiv.) of solid sodium borohydride over one hundred minutes. The solvents were removed under reduced pressure at 40°C and the residue dissolved in ethyl acetate (approx. 1L). The solution was washed sequentially with 1M potassium hydrogen sulfate, saturated sodium bicarbonate and then saturated sodium chloride solution. After drying over anhydrous magnesium sulfate and filtering, the solution was removed under reduced pressure. To the resulting oil was added hexane (approx. 1L) and the mixture warmed to 60°C with swirling. After cooling to room temperature, the solids were collected and washed with 2L of hexane. The resulting solid was recrystallized from hot ethyl acetate and hexane to afford 32.3g (43% yield) of N-benzyloxycarbonyl-3(S)-amino-1-chloro-4-phenyl-2(S)-butanol, mp 150-151°C and M+Li⁺ = 340.

### Part B:

To a solution of 6.52g (0.116 mol, 1.2 equiv.) of potassium hydroxide in 968 mL of absolute ethanol at room temperature, was added 32.3g (0.097 mol) of N-CBZ-3(S)-amino-1-chloro-4-phenyl-2(S)-butanol. After stirring for fifteen minutes the solvent was removed under reduced pressure and the solids dissolved in methylene chloride. After washing with water, drying over magnesium sulfate, filtering and stripping, one obtains 27.9g of a white solid. Recrystallization from hot ethyl acetate and hexane afforded 22.3g (77% yield) of N-benzyloxycarbonyl-3(S)-amino-1,2(S)-epoxy-4-phenylbutane, mp 102-103°C and MH⁺ 298.

### Part C:

A solution of N-benzyloxycarbonyl 3(S)-amino-1,2-(S)-epoxy-4-phenylbutane (30.lg, 0.10 mol) and 165mL of isoamylamine in 150 mL of isopropyl alcohol was heated to reflux for 2.5 hours. The solution was cooled to room temperature, concentrated *in vacuo* and then recrystallized. The product was isolated by filtration and from ethylacetate/hexane to afford 31.7g (81%) of N[3(S)-benzyloxycarbonylamino-2(R)-hydroxy-4-phenylbutyl]N-isoamylamine.

### Part D:

A solution of N[3(S)-benzyloxycarbonylamino-2(R)-hydroxy-4-phenyl butyl], N-isoamylamine in 10 ml of tetrahydrofuran was treated with tert-butylisocyanate (267 mg, 2.70 mmol) at room temperature for 5 minutes. The solvent was removed *in vacuo* and replaced with ethyl acetate. The ethyl acetate solution was washed with 5% citric acid, water, and brine, dried over anhydrous MgSO₄, filtered and concentrated *in vacuo* to give 1.19g, 97% of N-benzyloxycarbonyl-3-[[[(1,1-dimethylethyl)amino]carbonyl](3-methylbutyl)amino]-2(R)-hydroxy-1(S)-(phenylmethyl)propylamine, MH⁺ m/z = 470.

### Part E:

A solution of (37.3g, 77 mmol) of product from Part D in 100 mL of methanol was hydrogenated over 10% palladium-on-carbon for 4 hours to afford 26.1g of the desired final product 1.

### Example 35

### Preparation of Butanediamide, N-(3-[[[(1,1-dimethylethyl)amino]carbonyl](3-methylbutyl)amino]-2 -hydroxy-1-(phenylmethyl)Dropyl]-, [1S-[1R*, 2S*]]-.

### Part A:

To a solution of 102mg (0.29 mmol) of 1 and 70 mg (0.89 mmol) of pyridine in 2 mL of methylene chloride was added 29 mg (0.29 mmol) of succinic anhydride. After 2 hours, ethyl acetate was added and then extracted with saturated NaHCO. The aqueous layer was acidified, reextracted with ethyl acetate, washed with saturated brine, dried over magnesium sulfate, filtered and concentrated *in vacuo* to afford 78 mg (60%) of butanoic acid, 4-[[3-[[[(1,1-dimethylethyl)amino]carbonyl](3-methylbutyl)amino)-2-hydroxy-1-(phenylmethyl)propyl]amino-4-oxo-, [1S-[1R*, 2S*]-.

### Part B:

This was activated with EDC and N-hydroxybenzotriazole in N,N-dimethylformamide and then reacted with ammonia to generate the desired final compound.

### Example 36

### Part A:

To a solution of 4.60g (24.7 mmol) of transdiethyl 1,2-cyclopropanedicarboxylatease in 100 mL of 50:50 v:v tetrahydrofuran/water was added 1.24g (29.6 mmol) of lithium hydroxide. After 17 hours, the tetrahydrofuran was removed *in vacuo,* the water layer washed with ethyl acetate, acidified with IN hydrochloric acid and reextracted with ethyl acetate. The organic layer was dried and stripped to afford 2.lg of crude product. After recrystallization from diethyl ether/hexane and then methylene chloride/hexane one obtains 1.1g (28%) of trans-monoethyl 1,2-cyclopropanedicarboxylate, m/e = 159 (M + H).

### Part B:

To a solution of 297 mg (1.87 mmol) of trans-monoethyl 1,2-cyclopropanedicarboxylate and 429 mg (2.8 mmol) N-hydroxybenzotriazole (HoBT) in 3 mL of anhydrous N,N-dimethylformamide (DMF) at 0°C was added 394 mg (2.0 mmol) of 1-(3-dimethylaminopropyl)-3-ethylcarbodiimide hydrochloride (EDC). After 30 min. a solution of 591 mg (1.7 mmol) of **1** in 2 mL DMF and 171 mg (1.69 mmol) of N-methylmorpholine (NMM) was added. After 2 hours at 0°C, the reaction was stirred at RT overnight, poured into water, extracted with ethyl acetate, washed with water, 5% aq. citric acid, sat'd NaHCO₃, sat'd brine, dried and stripped to afford 771 mg of crude product. This was chromatographed on silica gel using 5-20% methanol/methylene chloride to afford 670 mg (80%) of cyclopropane carboxylic acid, 2-[[[3-[[[(1,1-dimethylethyl)amino]carbonyl](3-methylbutyl)amino]-2-hydroxy-1-(phenylmethyl)propyl]amino]carbonyl]-, ethyl ester; m/e = 490 (M + H).

### Part C:

To a solution of 658 mg (1.32 mmol) of product from part B in 5 mL of 50:50 THF/water was added 66 mg (1.58 mmol) of lithium hydroxide. After 19 hours, the THF was removed *in vacuo,* the water washed with ethyl acetate, acidified and reextracted with ethyl acetate. The organic layer was dried and stripped to afford 328 mg (54%) of the corresponding acid, m/e = 462 (M + H).

### Part D:

To a solution of 304 mg (0.66 mmol) of product from part C, 151 mg (0.99 mmol) HoBT in 2.2 mL DMF at 0°C was added 139 mg (0.73 mmol) EDCl. After 30 min. at 0°C, 1.1 mL of conc. aqueous ammonia was added. After stirring at 0°C for 2 hours and RT for 20 hours, the reaction was poured into sat'd brine and extracted with ethyl acetate. After washing with sat'd NaHCO₃, sat'd brine, drying and stripping, one obtains 141 mg of crude product. This was chromatographed on silica gel with 1-5% methanol/methylene chloride to afford 40 mg (13%) of the desired final product, m/e = 561 (M + H).

### Example 37

### Preparation of trans-but-2-enediamide, N-[3-[[[(1,1-dimethylethyl)amino]carbonyl](3-methylbutyl)amino]-2 -hydroxy-1-(phenylmethyl)propyl]-, [1S-[1R*, 2S*].

### Part A:

To a solution of 137 mg (0.95 mmol) fumaric acid monoethyl ester in 1 mL of DMF at 0°C was added 183 mg (0.95 mmol) EDCl. After 15 minutes, a solution of 333 mg (0.95 mmol) of **1** in 1 mL DMF was added and the reaction stirred for 14 hours at RT. Ethyl acetate was added and extracted with sat'd brine, 0.2 n HCl, sat'd NaHCO₃, dried and stripped to afford 0.32g of crude product. Chromatography on silica gel using 0-50% ethyl acetate/hexane afforded 0.26g (58%) of but-2-enoic acid, 4-[[3-[[[(1,1-dimethylethyl)amino]carbonyl](3-methylbutyl)amino]-2-hydroxy-1-(phenylmethyl)propyl]amino]-4-oxo-, [1S-[1R*, 2S*]]-, ethyl ester, m/e = 476 (M + H).

### Part B:

To a solution of 26.6 mg (0.56 mmol) of product from part A in 3 mL of 50:50 THF/water was added 34 mg (0.82 mmol) of lithium hydroxide and the reaction stirred at RT for 1 hour. The THF was removed *in vacuo,* the aqueous layer acidified with 1N HCl and extracted with ethyl acetate. The organic layer was washed with brine, dried and stripped to afford 233 mg (93%) of trans-but-2-enoic acid, 4-[[3-[[[(1,1-dimethylethyl)amino]carbonyl](3-methylbutyl)amino]-2-hydroxy-1-(phenylmethyl)propyl]amino]-4-oxo-, [1S-[1R*, 2S*]-, m/e = 448 (M + H).

### Part C:

To a solution of 225 mg (0.50 mmol) of the product from part B in 1 mL of DMF was added 95 mg (0.50 mmol) EDCl. After 15 minutes at RT, 0.50 mL of conc. aqueous ammonia was added and the reaction stirred for 15 hours. Ethyl acetate was added and washed with 0.2N HCl, brine, dried and stripped to afford 170 mg of crude product. After chromatography on silica gel using 0-40% methanol/methylene chloride, one obtains 50 mg (22%) of trans-but-3-enediamide, N-[3-[[[(1,1-dimethylethyl)amino]carbonyl](3-methylbutyl)amino)-2-hydroxy-l-(phenylmethyl)propyl]-, [1S-[1R*, 2S*]-, m/e = 447 (M + H).

### Example 38

### Preparation of butanediamide. N-[3-[[[(1,1-dimethylethyl)amino]carbonyl](3-methylbutyl)amino]-2 -hydroxy-1-(phenylmethyl)propyl-2-methyl-, [1S-[1R*(2S*), 2S*]-.

### Part A:

To a suspension of 24.7g (0.22 mol) of itaconic anhydride in 100 mL of anhydrous toluene at reflux under a nitrogen atmosphere was added dropwise over 30 minutes 23.9g (0.22 mol) of benzyl alcohol. The insoluble material dissolved to provide a homogeneous solution which was refluxed for 1.5 hours. The solution was cooled to RT, then in an ice bath and the resulting white precipitate collected by filtration to afford 24.8g (51%) of 4-benzyl itaconate.

### Part B:

To a solution of 2.13g (9.5 mmol) of the product from part A in 12 mL of methylene chloride at 0°C was added 4.02g (29.1 mmol) of para-methoxybenzyl alcohol, 605 mg (4.95 mmol) of N,N-dimethyl 4-aminopyridine, 128 mg of N,N-dimethyl 4-aminopyridine hydrochloride salt and then 2.02g (4.7 mmol) dicyclohexylcarbodiimide (DCC). After stirring at 0°C for 1 hour and then RT for 2 hours, the precipitate was collected and discarded. The filtrate was washed with 0.5 N HCl, sat'd NaHCO₃, dried and stripped to afford 4.76g of crude product. This was chromatographed on silica gel using 0-50% ethyl acetate/hexane to afford 1.24g of pure 4'-methoxybenzyl-4-benzylitaconate , MH⁺ m/z = .

### Part C:

A solution of 1.24g (3.65 mmol) of product from part B and 20 mg of [(R,R)-Dipamp)cyclooctadienylrhodium] tetrafluoroborate in 30 mL of methanol was thoroughly degassed, flushed with nitrogen and then hydrogen and then stirred under 50 psig of hydrogen for 15 hours. The solution was filtered and stripped, dissolved in methylene chloride and washed with sat'd NaHCO₃, dried and stripped to afford 0.99g of a brown oil. This was then dissolved in 40 mL of methylene chloride, 3 mL of trifluoroacetic acid added and the solution stirred at RT for 3.5 hours. Water was added and separated and the organic layer extracted with sat'd NaHCO₃. The aqueous layer was acidified and reextracted with ethyl acetate, separated and the organic layer washed with brine, dried and stripped to afford 320 mg (50%) of 2(R)-methyl-4-benzylsuccinic acid.

### Part D:

To a solution of 320 mg (1.44 mmol) of product from part C and 314 mg (2.05 mmol) HoBT in DMF at 0°C was added 303 mg (1.58 mmol) of EDCl. After stirring for 30 minutes, a solution of 467 mg (1.34 mmol) of **1** in 4 mL of DMF was added. After stirring for 1 hour at 0°C and 14 hours at RT, ethyl acetate was added and washed with sat'd NaHCO₃, 5% aqueous citric acid, dried and stripped to afford 0.97g of crude product. This was chromatographed on silica gel using 0-10% ethyl acetate/hexane to afford 420 mg of pure butanoic acid, 4-[[3-[[[(1,1-dimethylethyl)amino]carbonyl](3-methylbutyl)amino]-2-hydroxy-1-(phenylmethyl)propyl]amino]-3-methyl-4-oxo-, [1S-[1R*(3S*), 2S*]-, benzyl ester.

### Part E:

A solution of 150 mg (0.27 mmol) of product from part D in 15 mL of methanol was hydrogenated over 10% palladium on carbon under 50 psig hydrogen for 17 hours. The reaction was filtered and stripped to afford 125 mg (100%) of butanoic acid, 4-[[3-[[[(1,1-dimethylethyl)amino]carbonyl](3-methylbutyl)amino]-2-hydroxy-1-(phenylmethyl)propyl]amino]-3-methyl-4-oxo-, [1S-[1R*(3S*), 2S*]-.

### Part F:

To a solution of 125 mg (0.27 mmol) of product from part E and 65 mg (0.42 mmol) of HoBT in 5 mL of DMF at 0°C was added 59 mg (0.31 mmol) of EDCl. After 30 min. at 0°C, 1 mL of conc. aqueous ammonia was added. After stirring at 0°C for 2 hours and RT fro 15 hours, ethyl acetate was added and washed with sat'd NaHCO₃, 5% aqueous citric acid, dried and stripped to afford 90 mg of crude product. This was recrystallized from ethyl acetate/hexane to afford 40 mg (32%) of pure butanediamide, N-[3-[[[(1,1-dimethylethyl)amino]carbonyl](3-methylbutyl)amino]-2-hydroxy-l-(phenylmethyl)propyl]-2-methyl, [1S-[1R* (2S*), 2S*]-.

### Example 39

### Preparation of butanediamide, N-[3-[[[(1,1-dimethylethyl)amino]carbonyl](3-methylbutyl)amino]-2 -hydroxy-1-(phenylmethyl)propyl]-2-methyl, [1S-[1R* (2R*), 2S*]-.

### Part A:

A solution of 1.41g (4.1 mmol) of 4'-methoxybenzyl 4-benzylitaconate and 25 mg of [(S,S-Dipamp)cyclooctadienylrhodium]tetrafluoroborate in 20 mL of methanol was thoroughly degassed, flushed with nitrogen and then hydrogen and then stirred under 40 psig hydrogen for 72 hours. The solution was filtered and concentrated to provide 1.34g of a brown oil. This was dissolved in 40 mL of methylene chloride and 3 mL of trifluoroacetic acid was added. AFter stirring for 4 hours, water was added, separated and the organic layer extracted with sat'd NaHCO₃. The aqueous layer was separated, reacidified, extracted with ethyl acetate which was separated, washed with brine, dried and stripped to afford 440 mg of 2(S)-methyl-4-benzylsuccinic acid.

### Part B:

To a solution of 440 mg (1.98 mmol) of the product from part A and 437 mg (2.86 mmol) of HoBT in 9 mL of DMF at 0°C was added 427 mg (2.23 mmol) of EDCl. After 30 minutes at 0°C, a solution of 653 mg (1.87 mmol) of 1 in 3 mL DMF was added. After 1 hour at 0°C and 15 hours at RT, ethyl acetate was added, extracted with sat'd NaHCO₃, 5% aqueous citric acid, dried and concentrated to afford 0.98g of crude product. Chromatography on silica gel using 0-10% ethyl acetate afforded 610 mg (59%) of pure butanoic acid, 4-[[3-[[[(1,1-dimethylethyl)-amino]carbonyl](3-methylbutyl)amino]-2-hydroxy-1-(phenylmethyl)propyl]amino]-3-methyl-4-oxo-, (1S-[1R*(3R*), 2S*], benzyl ester.

### Part C:

A solution of 310 mg (0.56 mmol) of the product from part B in 20 mL of methanol was hydrogenated over 20 mg of 10% palladium on carbon under 50 psig hydrogen for 19 hours. The solution was filtered and concentrated to afford 220 mg (85%) of butanoic acid, 4-[[3-[[[(1,1-dimethylethyl)-amino]carbonyl](3-methylbutyl)amino]-2-hydroxy-1-(phenylmethyl)propyl]amino]-3-methyl-4-oxo-, [1S-[1R* (3R*) , 2S*].

### Part D:

To a solution of 190 mg (0.41 mmol) of the product from part C and 90 mg (0.58 mmol) HoBT in 5 mL of DMF at 0°C, was added 88 mg (0.46 mmol) of EDCl. After 30 minutes at 0°C, 2 mL of conc. aqueous ammonia was added. After 1 hour at 0°C and 15 hours at RT, ethyl acetate was added, washed with sat'd NaHCO₃, 5% aqueous citric acid, dried and concentrated to afford crude product. Recrystallization from ethyl acetate/hexane afforded 20 mg (11%) of butanediamide, N-[3-[[[(1,1-dimethylethyl)amino]carbonyl](3-methylbutyl)amino]-2-hydroxy-1-(phenylmethyl)propyl]-2-methyl, [1S-[1R*(2R*), 2S*]-.

### Example 40

### Preparation of butanediamide, N-[3-[[[(1,1-dimethylethyl)amino]carbonyl](3-methylbutyl)amino]-2 -hydroxy-1-(phenylmethyl)propyl]-3-methyl-, [1S-[1R*(3S*), 2S*]-.

### Part A:

In a similar manner to the procedure used above, p-methoxybenzyl alcohol was reacted with itaconic anhydride in refluxing toluene to provide 4-(p-methoxybenzyl)itaconate.

### Part B:

To a solution of 3.30g (13.2 mmol) of the product from part A in 17 mL of toluene, was added 2.08g (13.7 mmol) of 1,8-diazabicyclo[5.40]undec-7-enc and then 2.35g (13.7 mmol) of benzyl bromide. After 2 hours, the solution was filtered and the filtrate washed with sat'd NaHCO₃, 3N HCl, brine, dried and concentrated to afford 3.12g of an oil. After chromatography on silica gel using 0-5% ethyl acetate/hexane one obtains 2.19g (49%) of benzyl 4-(4-methoxybenzyl)itaconate.

### Part C:

A solution of 1.22g (3.6 mmol) of product from part B and 150 mg of [((R,R-Dipamp)) cyclooctadienylrhodium] tetrafluoroborate in 15 mL of methanol was thoroughly degassed, flushed with nitrogen and then hydrogen and hydrogenated under 50 psig for 16 hours. The solution was filtered and concentrated to afford 1.2g of a brown oil. This was dissolved in 5 mL of methylene chloride and 5 mL of toluene and 3 mL of trifluoroacetic acid was added. After 4 hours, the solvents were removed *in vacuo,* the residue dissolved in methylene chloride, which was then extracted with sat'd NaHCO₃. After separation, the aqueous layer was acidified, reextracted with methylene chloride which was then dried and concentrated to afford 470 mg (60%) of 3(R)-methyl-4-benzylsuccinic acid.

### Part D:

To a solution of 470 mg (2.11 mmol) of product from part C and 463 mg (3.03 mg) of HoBT in 5 mL of DMF at 0°C was added 451 mg (2.35 mmol) of EDCl. After 30 min. at 0°C, a solution of 728 mg (2.08 mmol) of **1** in 3 mL of DMF was added. After stirring at 0°C for 1 hour and 15 hours at RT, ethyl acetate was added and extracted with sat'd NaHCO₃, 5% aqueous citric acid, brine, dried and concentrated to give 930 mg of crude product chromatography on silica gel using 0-10% ethyl acetate/hexane one obtains 570 mg (50%) of butanoic acid, 4-[[3-[[[(1,1-dimethylethyl)amino]carbonyl](3-methylbutyl)amino]-2-hydroxy-1-(phenylmethyl)propyl]amino]-2-methyl-4-oxo-, [1S-[1R*(2S*), 2S*]-, benzyl ester.

### Part E:

The product was hydrogenated in methanol using 10% palladium on carbon under 40 psig of hydrogen to afford butanoic acid, 4-[[3-[[[(1,1-dimethylethyl)amino]carbonyl]-(3-methylbutyl)amino]-2-hydroxy-1-(phenylmethyl)propyl]amino]-2-methyl-4-oxo-, [1S-[1R*(2S*), 2S*]-.

### Part F:

To a solution of 427 mg (0.92 mmol) of product from part E and 210 mg (1.37 mmol) in 3 mL of DMF at 0°C was added 196 mg (1.02 mmol) of EDCl. After 30 min. at 0°C, 2 mL of conc. aqueous ammonia was added. After 1 hour at 0°C and 15 hours at RT, ethyl acetate was added and then extracted with sat'd NaHCO₃, brine, dried and concentrated to afford crude product. Recrystallization from ethyl acetate/hexane afforded 50 mg (12%) of butanediamide, N-[3-[[[(1,1-dimethylethyl)amino]carbonyl](3-methylbutyl)amino]-2-hydroxy-l-(phenylmethyl)propyl]-3-methyl-, [1S-[1R*(3S*), 2S*]-.

### Example 41

### Preparation of butanediamide, N-[3-[[[(1,1-dimethylethyl)amino]carbonyl](3-methylbutyl)amino]-2 -hydroxy-1-(phenylmethyl)propyl]-3-methyl-, [1S-[1R*(3R*), 2S*]-.

This was prepared in an identical manner to the previous example except that the asymmetric hydrogenation step was done in the presence of [((S,S-dipamp)cyclooctadienyl)rhodium]-tetrafluoroborate as catalyst.

### Example 42

### Preparation of butanediamide, N-[3-[[[(1,1-dimethylethyl)amino]carbonyl](3-methylbutyl)amino]-2 -hydroxy-1-(phenylmethyl)propyl]-, [1S-[1R* (2S*, 3R*). 2S*]], and [1S-[1R*(2R*, 3S*), 2S*]].

### Part A:

To a solution of 863 mg (5.91 mmol) of meso-2,3-dimethylsuccinic acid in 7 mL of DMF at RT was added 1.13g (5.91mmol) of EDCl. After 15 minutes, a solution of 2.07g (5.91 mmol) of **1** and 1.4 mL of pyridine in 7 mL of anhydrous methylene chloride was added. After 11 hours, ethyl acetate was added and washed with 0.2N HCl, brine, dried and concentrated to afford 2.73g (97%) of a 1:1 mixture of diastereomeric acids.

### Part B:

To a solution of 1.45g (3.04 mmol) of the 1:1 mixture from part A and 613 mg (4.51 mmol) of HoBT in 10 mL of DMF at 0°C was added 635 mg (3.31 mmol) of EDCl. After 30 minutes at 0°C, 5 mL of conc. aqueous ammonia was added. After 1 hour at 0°C and 14 hours at RT, ethyl acetate was added, washed with 0.2N HCl, sat'd NaHCO₃, brine, dried and concentrated to afford 0.64g (44%) of a 1:1 mixture of amides.

These were separated on a Whatman 10 micron partisil column using 8%-14% isopropanol/-methylene chloride. The first isomer to elute was identified as butanediamide, N-[3-[[[(1,1-dimethylethyl)amino]carbonyl](3-methylbutyl)amino]-2-hydroxy-1-(phenylmethyl)propyl]-, [1S-[1R*(2R*, 3S*), 2S*], m/e/ = 477 (M + H).

The second isomer to elute was identified as butanediamide, N-[3-[[[(1,1-dimethylethyl)amino]-carbonyl](3-methylbutyl)amino]-2-hydroxy-1-(phenylmethyl)propyl]-, [1S-[1R*(2S*, 3R*), 2S*], m/e = 477 (M + H).

### Example 43

### Preparation of pentanediamide, N-[3-[[[(1,1-dimethylethyl)amino]carbonyl](3-methylbutyl)amino]-2 -hydroxy-1-(phenylmethyl)propyl-3,3-dimethyl-, [1S-[1R*, 2S*].

### Part A:

To a solution of 232 mg (0.66 mmol) of **1** and 98 mg (1.2 mmol) of pyridine in 2 mL of methylene chloride was added 95 mg (0.66 mmol) of 3,3-dimethylglutaric anhydride at RT. After 15 hours, ethyl acetate was added, washed with IN HCl, brine, dried and concentrated to afford 261 mg of crude product. Chromatography on silica gel using 5-20% methanol/methylene chloride afforded 108 mg of acid, m/e = 492 (M + H).

### Part B:

To a solution of 92 mg (0.19 mmol) of product from part A and 38 mg (0.28 mmol) HoBT in 0.5 mL DMF at 0°C was added 36 mg (0.19 mmol) of EDCl. After 30 minutes at 0°C, 0.25 mL of conc. aqueous ammonia was added. After 1 hour at 0°C and 16 hours at RT, ethyl acetate was added, washed with 0.2N HCl, sat'd NaHCO₃, brine, dried and concentrated to afford 72 mg of crude product. This was passed through a one-inch column of basic alumina with 10% methanol/methylene chloride to afford 53 mg of desired product, m/e = 491 (M + H).

### Example 44

### Preparation of butanediamide, N-[3-[[[(1,1-dimethylethyl)amino]carbonyl](3-methylbutyl)amino]-2 -hydroxy-1-(phenylmethyl)propyl]-2,3-dimethyl-[1S -[1R*(2R*, 3S*), 2S*]](Isomer #1) and Preparation of butanediamide, N-[3-[[[(1,1-dimethylethyl)amino]carbonyl](3-methylbutyl)amino]-2 -hydroxy-1-(phenylmethyl)propyl]-2,3-dimethyl-[1S -[1R*(2R*, 3S*), 2S*]] (Isomer #2).

### Part A:

To a solution of 1.47g (4.20 mmol) of 1 and 1.4 mL of pyridine in 9 mL of methylene chloride at RT was added 538 mg (4.20 mmol) of 2,2-dimethylsuccinic anhydride. After 15 hours, ethyl acetate was added and washed with 0.2N HCl, brine, dried and concentrated to afford 1.87g of crude product (approx. 3:1 mixture of isomer).

### Part B:

To a solution of 1.85g (3.9 mmol) of crude product from part A and 887 mg (5.8 mmol) of HoBT in 10 mL of DMF at 0°C was added 809 mg (4.2 mmol) EDCl. After 30 minutes at 0°C, 6 mL of conc. aqueous ammonia was added. After 1 hour at 0°C and 15 hours at RT, ethyl acetate was added, washed with 0.2N HCl, sat'd NaHCO₃, brine, dried and concentrated to afford 923 mg of crude product. The two isomers were separated on a Whatman Partisil 5 column using 8-14% isopropanol/methylene chloride. The major isomer was identified as Isomer #1, m/e = 477 (M + H).

The minor isomer was identified as Isomer #2, m/e = 477 (M + H).

### Example 45

This example illustrates the procedure utilized to prepare compounds wherein the stereochemistry about the hydroxyl group is (S).

### Part A:

A solution of 3(S)-(1,1-dimethylethoxycarbonyl)amino-1,2-(R)-epoxy-4-phenylbutane (1.00g, 3.80 mmol) and isobutylamine (5.55g, 76 mmol, 20 equiv.) in 10 mL of isopropyl alcohol was warmed to 60°C for 1 hour. The solution was cooled to room temperature and concentrated *in vacuo* and the residue recrystallized from hexane/methylene chloride to give 0.93g, 73% of [2(S), 3(S)]-N-[[[3-[(1,1-dimethylethyl)carbamoyl]amino]]-2-hydroxy-4-phenylbutyl]N-[(3-methylbutyl)]amine, mp 91.3 - 93.0°C.

### Part B:

The product from Part A (46.3mg, 0.14 mmol) was dissolved in a mixture of 5 mL of tetrahydrofuran and 2 mL of methylene chloride and treated with tert-butylisocyanate (136.4mg, 1.376 mmol) *via* syringe. The solution was stirred at room temperature for 0.5 hour and then the solvent was removed *in vacuo.* The product, TLC on SiO₂, 1:1 hexane: Ethyl acetate had Rf = 0.74 and was used directly in the next step without further purification.

### Part C:

The crude product from Part B was taken up in 10 mL of 4N hydrochloric acid in dioxane and stirred at room temperature for 0.25 hours. The solvent and excess hydrochloric acid was removed *in vacuo* whereupon the product crystallized. The solid was isolated by filtration washed with acetone and dried *in vacuo* to 3-[[(1,1-dimethylethyl)amino]carbonyl](2-methylpropyl)amino-2(S)-hydroxy-1(S)-(phenylmethyl)propylamine hydrochloride.

### Part D:

A solution of N-Cbz-L-asparagine (225.5mg, 0.847 mmol) and N-hydroxybenzotriazole (182.9mg, 1.21 mmol) was dissolved in 2 mL of dimethylformamide and cooled to 0°C and then treated with EDC (170.2mg, 0.898 mmol) for 10 minutes. This mixture was then treated with 3-[[(1,1-dimethylethyl)amino]carbonyl](2-methylpropyl)amino-2(s)-hydroxy-1(s)-(phenylmethyl)propylamine hydrochloride. (300.0mg, 0.807 mmol) followed by N-methylmorpholine (90.0mg, 0.888 mmol) *via* syringe. The solution was stirred at room temperature for 16 hours and then poured into 20 mL of rapidly stirring 60% saturated aqueous sodium bicarbonate solution whereupon a white precipitate formed. The solid was isolated by filtration, washed with saturated aqueous sodium bicarbonate solution, water, 5% aqueous citric acid solution, water and then dried *in vacuo* to give 319mg, 68% of butanediamide, N'-[3-[[[(1,1-dimethylethyl)amino]carboyl](2-methylpropyl)amino]-2(S)-hydroxy-1(S)-(phenylmethyl)propyl]-2(S)-[(benzyloxycarbonyl)amino] mp 139-141°C, MH⁺ m/z = 584.

### EXAMPLE 46

The compounds of the present invention are effective HIV protease inhibitors. Utilizing an enzyme assay as described below, the compounds set forth in the examples herein disclosed inhibited the HIV enzyme. The preferred compounds of the present invention and their calculated IC₅₀ (inhibiting concentration 50%, i.e., the concentration at which the inhibitor compound reduces enzyme activity by 50%) values are shown in Table 16. The enzyme method is described below. The substrate is 2-aminobenzoyl-Ile-Nle-Phe(p-NO₂)-Gln-ArgNH₂. The positive control is MVT-101 (Miller, M. et al, Science, 246, 1149 (1989)] The assay conditions are as follows:
- Assay buffer:: 20 mM sodium phosphate, pH 6.4
20% glycerol
1 mM EDTA
1 mM DTT
0.1% CHAPS

The above described substrate is dissolved in DMSO, then diluted 10 fold in assay buffer. Final substrate concentration in the assay is 80 µM.

HIV protease is diluted in the assay buffer to a final enzyme concentration of 12.3 nanomolar, based on a molecular weight of 10,780.

The final concentration of DMSO is 14% and the final concentration of glycerol is 18%. The test compound is dissolved in DMSO and diluted in DMSO to 10x the test concentration; 10µl of the enzyme preparation is added, the materials mixed and then the mixture is incubated at ambient temperature for 15 minutes. The enzyme reaction is initiated by the addition of 40µl of substrate. The increase in fluorescence is monitored at 4 time points (0, 8, 16 and 24 minutes) at ambient temperature. Each assay is carried out in duplicate wells.

**TABLE 16**

| | Compound | IC₅₀ (nanomolar) |
|---|---|---|
| 1. | 3-Thia-4,7,11-triazadodecan-12-amide, N,5-bis(1,1-dimethylethyl)-9-hydroxy-11-(3-methylbutyl)-6-oxo-8-(phenylmethyl)-1-phenyl-, 2,2-dioxide-,5S-,(5R*,8R*,9S*)] | 22nM |
| 2. | 3-Thia-4,7,11-triazadodecan-12-amide, N-(1,1-dimethylethyl)-5-(1-methylethyl)-9-hydroxy-11-(3-methylbutyl)-6-oxo-8-(phenylmethyl)-1-phenyl-, 2,2-dioxide-,[5S-,(5R*,8R*,9S*)] | 29nM |
| 3. | 3-Thia-4,7,11-triazadodecan-12-amide, N-(1,1-dimethylethyl)-5-(2-amino-2-oxo-ethyl)-9-hydroxy-11-(3-methylbutyl)-6-oxo-8-(phenylmethyl)-1-phenyl-, 2,2-dioxide-,[5S-,(5R*,8R*,9S*)] | 24nM |

### Example 47

The effectiveness of the compounds listed in Table 16 were determined in the above-described enzyme assay and in a CEM cell assay.

The HIV inhibition assay method of acutely infected cells is an automated tetrazolium based colorimetric assay essentially that reported by Pauwles et al, J. Virol. Methods 20, 309-321 (1988). Assays were performed in 96-well tissue culture plates. CEM cells, a CD4⁺ cell line, were grown in RPMI-1640 medium (Gibco) supplemented with a 10% fetal calf serum and were then treated with polybrene (2µg/ml). An 80 µl volume of medium containing 1 x 10⁴ cells was dispensed into each well of the tissue culture plate. To each well was added a 100µl volume of test compound dissolved in tissue culture medium (or medium without test compound as a control) to achieve the desired final concentration and the cells were incubated at 37°C for 1 hour. A frozen culture of HIV-1 was diluted in culture medium to a concentration of 5 x 10⁴ TCID₅₀ per ml (TCID₅₀ = the dose of virus that infects 50% of cells in tissue culture), and a 20µL volume of the virus sample (containing 1000 TCID₅₀ of virus) was added to wells containing test compound and to wells containing only medium (infected control cells). Several wells received culture medium without virus (uninfected control cells). Likewise, the intrinsic toxicity of the test compound was determined by adding medium without virus to several wells containing test compound. In summary, the tissue culture plates contained the following experiments:

| | Cells | Drug | Virus |
|---|---|---|---|
| 1. | + | - | - |
| 2. | + | + | - |
| 3. | + | - | + |
| 4. | + | + | + |

In experiments 2 and 4 the final concentrations of test compounds were 1, 10, 100 and 500 µg/ml. Either azidothymidine (AZT) or dideoxyinosine (ddI) was included as a positive drug control. Test compounds were dissolved in DMSO and diluted into tissue culture medium so that the final DMSO concentration did not exceed 1.5% in any case. DMSO was added to all control wells at an appropriate concentration.

Following the addition of virus, cells were incubated at 37°C in a humidified, 5% CO₂ atmosphere for 7 days. Test compounds could be added on days 0, 2 and 5 if desired. On day 7, post-infection, the cells in each well were resuspended and a 100µl sample of each cell suspension was removed for assay. A 20µL volume of a 5 mg/ml solution of 3-(4,5-dimethylthiazol-2-yl)-2,5-diphenyltetrazolium bromide (MTT) was added to each 100µL cell suspension, and the cells were incubated for 4 hours at 27°C in a 5% CO₂ environment. During this incubation, MTT is metabolically reduced by living cells resulting in the production in the cell of a colored formazan product. To each sample was added 100µl of 10% sodium dodecylsulfate in 0.01 N HCl to lyse the cells, and samples were incubated overnight. The absorbance at 590 nm was determined for each sample using a Molecular Devices microplate reader. Absorbance values for each set of wells is compared to assess viral control infection, uninfected control cell response as well as test compound by cytotoxicity and antiviral efficacy.

**TABLE 17**

| Compound | | IC₅₀ | EC₅₀ |
|---|---|---|---|
| 1. | 3-Thia-4,7,11-triazadodecan-12-amide, N,5-bis(1,1-dimethylethyl)-9-hydroxy-11-(3-methylbutyl)-6-oxo-8-(phenylmethyl)-1-phenyl-, 2,2-dioxide-,[5S-,(5R*,8R*,9S*)] | 22nM | 510nM |
| 2. | 3-Thia-4,7,11-triazadodecan-12-amide, N-(1,1-dimethylethyl)-5-(1-methylethyl)-9-hydroxy-11-(3-methylbutyl)-6-oxo-8-(phenylmethyl)-1-phenyl-, 2,2-dioxide-,[5S-,(5R*,8R*,9S*)] | 29nM | 440nM |

The compounds of the present invention are effective antiviral compounds and, in particular, are effective retroviral inhibitors as shown above. Thus, the subject compounds are effective HIV protease inhibitors. It is contemplated that the subject compounds will also inhibit other viruses such as HIV, human T-cell leukemia virus, respiratory syncytial virus, hepadnavirus, cytomegalovirus and picornavirus.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, palmoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other examples include salts with alkali metals or alkaline earth metals, such as sodium, potassium, calcium or magnesium or with organic bases.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as in normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds. Variations and changes which are obvious to one skilled in the art are intended to be within the scope and nature of the invention which are defined in the appended claims.

The preceding examples can be repeated with similar success by substituting the generically or specifically described reactants and/or operating conditions of this invention for those used in the preceding examples.

## Claims

1. A compounds represented by the formula: 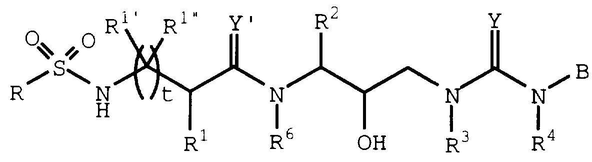 wherein
R represents alkyl, alkenyl, hydroxyalkyl, cycloalkyl, cycloalkylalkyl, heterocycloalkyl, heterocycloalkylalkyl, aryl, aralkyl or heteroaralkyl radicals;
t represents 0 or 1;
R¹ represents -CH₂SO₂NH₂, alkyl or cycloalkyl radicals or amino acid side chains selected from asparagine, S-methyl cysteine, or the corresponding sulfoxide or sulfone derivatives thereof, glycine, alloisoleucine, leucine, tert-leucine, phenylalanine, ornithine, alanine, threonine, allo-threonine, isoleucine, histidine, norleucine, valine, glutamine, serine, aspartic acid or beta-cyano alanine side chains;
R^{1'} and R^{1"} each independently represent hydrogen or radicals as defined for R¹;
R represents alkyl, aryl, cycloalkyl, cycloalkylalkyl or aralkyl radicals optionally substituted with a group selected from -OR⁹, -SR⁹, or halogen radicals, wherein R⁹ represents hydrogen or alkyl radicals;
R³ represents alkyl, alkenyl, hydroxyalkyl, cycloalkyl, cycloalkylalkyl, heterocycloalkyl, heterocycloalkylalkyl, aryl, aralkyl, or heteroaralkyl radicals;
Y and Y' each independently represent O or S;
R⁶ represents hydrogen or radicals as defined for R³;
B represents R⁵ or radicals represented by the formula: 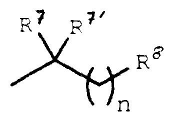 wherein n represents an integer of from 0 to 6; R⁷ and R^{7'} each independently represent radicals as defined for R³ or amino acid side chains selected from the group consisting of valine, isoleucine, glycine, alanine, allo-isoleucine, asparagine, leucine, glutamine, and t-butylglycine; or R⁷ and R^{7'} together with the carbon atom to which they are attached form a cycloalkyl radical; and R⁸ represents cyano, hydroxyl, alkyl, alkoxy, cycloalkyl, aryl, aralkyl, heterocycloalkyl or heteroaryl radicals or radicals represented by the formulas C(O)R¹⁶, CO₂R¹⁶, SO₂R¹⁶, SR¹⁶, CONR¹⁶R¹⁷, OR¹⁶, CF₃ or NR¹⁶R¹⁷, wherein R¹⁶ and R¹⁷ each independently represent hydrogen or radicals as defined for R³; or R¹⁶ and R¹⁷ together with a nitrogen to which they are attached represent heterocycloalkyl or heteroaryl radicals; and
R⁴ and R⁵ each independently represent radicals as defined for R³; or R⁴ and R⁵ together with the nitrogen atom to which they are bonded represent heterocycloalkyl or heteroaryl radicals; and
wherein alkyl, alone or in combination, is a straight-chain or branched-chain alkyl radical containing from 1 to 10 carbon atoms; alkenyl is a propenyl radical; cycloalkyl, alone or in combination, is a cyclic alkyl radical of from 3 to 8 carbon atoms; aryl, alone or in combination, is an unsubstituted phenyl radical, unsubstituted naphthyl radical, or phenyl or naphthyl radical substituted with one or more substituents selected from alkyl, alkoxy, halogen, hydroxy or amino; aralkyl, alone or in combination, is an alkyl radical in which one hydrogen atom is replaced by an aryl radical; heterocycloalkyl, alone or in combination, is a monocyclic, bicyclic, or tricyclic, saturated or partially unsaturated heterocycle radical selected from the group comprising pyrrolidinyl, piperidinyl, piperazinyl, morpholinyl, thiamorpholinyl, tetrahydroquinolyl, 1,2,3,4-tetrahydro-2-quinolyl, 1,2,3,4-tetraisoquinolyl and 1,2,3,4-tetrahydro-1-oxo-isoquinolyl; and heteroaryl, alone or in combination, is a monocyclic, bicyclic or tricyclic, aromatic or partially aromatic heterocycle radical selected from pyrrolyl,imidazolyl, pyrazolyl,pyridyl, pyrazinyl,pyrimidinyl, furyl, thienyl, triazolyl, oxazolyl, thiazolyl, indolyl, quinolyl, isoquinolyl, quinoxalinyl, beta-carbolinyl,2-benzofurancarbonyl and benzimidazolyl; wherein each heterocycloalkyl or heteroaryl radical contains one or more heteroatoms selected from the group consisting of nitrogen, oxygen and sulfur; and wherein each heterocycloalkyl or heteroaryl radical is optionally substituted on a secondary nitrogen atom with alkyl, aralkoxycarbonyl, alkanoyl, phenyl or phenylalkyl radicals; on a tertiary nitrogen atom with oxido; and on one or more substitutable carbon atoms with halogen, alkyl, alkoxy or oxo radicals.

2. Compound of Claim 1 represented by the formula: 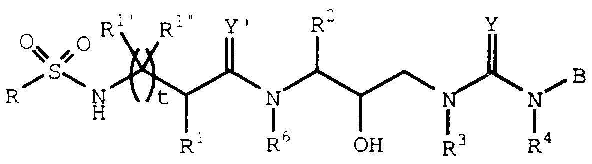 wherein
R represents alkyl, alkenyl, hydroxyalkyl, cycloalkyl, cycloalkyalkyl, heterocycloalkyl, heterocycloalkylalkyl, aryl, aralkyl or heteroaralkyl radicals;
t represents 0 or 1;
R¹ represents -CH₂SO₂NH₂, alkyl or cycloalkyl radicals, or amino acid side chains selected from the group of asparagine, S-methyl cysteine or the sulfoxide (SO) or sulfone (SO₂) derivatives thereof, histidine, norleucine, glutamine, glycine, allo-isoleucine, alanine, threonine, isoleucine, leucine, tert-leucine, phenylalanine, ornithine, allo-threonine, serine, aspartic acid, beta-cyano alanine or valine side chains;
R^{1'} and R^{1"} each independently represent hydrogen or radicals as defined for R¹;
R represents alkyl, aryl, cycloalkyl, cycloalkylalkyl, or aralkyl radicals, which radicals are optionally substituted with a group selected from halogen radicals or -OR⁹ or SR⁹, wherein R⁹ represents hydrogen or alkyl radicals;
R³ represents alkyl, alkenyl, hydroxyalkyl, cycloalkyl, cycloalkylalkyl, heterocycloalkyl, heterocycloalkylalkyl, aryl, aralkyl, heteroaryl or heteroaralkyl radicals;
R⁴ represents hydrogen or radicals as defined by R³; B represents radicals represented by the formula: 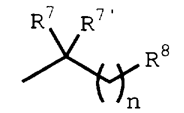 wherein
n represents an integer of from 0 to 6; R⁷ and R^{7'} each independently represent radicals as defined for R³ or amino acid side chains selected from the group consisting of valine, isoleucine, glycine, alanine, allo-isoleucine, asparagine, leucine, glutamine, and t-butylglycine; or R⁷ and R^{7'} together with the carbon atom to which they are attached form a cycloalkyl radical; and R⁸ represents cyano, hydroxyl, alkyl, cycloalkyl, aryl, aralkyl, heterocycloalkyl or heteroaryl radicals or radicals represented by the formulas C(O)R¹⁶, CO₂R¹⁶, SO₂R¹⁶, SR¹⁶, CONR¹⁶R¹⁷, OR¹⁶, CF₃ or NR¹⁶R¹⁷, wherein R¹⁶ and R¹⁷ each independently represent hydrogen or radicals as defined for R³; or R¹⁶ and R¹⁷ together with a nitrogen to which they are attached represent heterocycloalkyl or heteroaryl radicals;
R⁶ represents hydrogen or radicals as defined for R³;
t represents O or 1; and
Y represents O or S.

3. Compound of Claim 2 wherein R¹ represents methyl, ethyl or N-butyl radicals.

4. Compound of Claim 2 wherein R¹ represents alkyl radicals or amino acid side chains selected from the group consisting of asparagine, valine, threonine, allo-threonine, isoleucine, S-methyl cysteine and the sulfone and sulfoxide derivatives thereof, alanine, and allo-isoleucine.

5. Compound of Claim 2 wherein R¹ represents methyl, t-butyl, isopropyl or sec-butyl radicals, or amino acid side chains selected from the group consisting of asparagine, valine, S-methyl cysteine, alto-isoleucine, iso-leucine, threonine and allo-threonine side chains.

6. Compound of Claim 2 wherein R¹ represents methyl or t-butyl radicals.

7. Compound of Claim 2 wherein R¹ represents a t-butyl radical.

8. Compound of Claim 2 wherein R¹ represents amino acid side chains selected from the group consisting of asparagine, valine, alanine and isoleucine side chains.

9. Compound of Claim 2 wherein R¹ represents amino acid side chains selected from the group consisting of asparagine, iso-leucine and valine side chains.

10. Compound of Claim 2 wherein R¹ represents an asparagine side chain.

11. Compound of Claim 2 wherein R¹ represents a t-butyl radical or an asparagine side chain.

12. Compound of Claim 2 wherein R¹ represents a methyl radical when t is 1.

13. Compound of Claim 2 wherein t is 0.

14. Compound of Claim 2 wherein t is 1.

15. Compound of Claim 2 wherein R⁴ represents hydrogen; R³ represents alkyl, alkenyl, hydroxyalkyl, cycloalkyl, cycloalkyl-alkyl, heterocycloalkyl, heterocycloalkylalkyl, aryl, aralkyl or heteroaralkyl radicals; and R⁷ and R^{7'} each independently represent alkyl or aralkyl radicals, or together with the carbon atom to which they are attached form a cycloalkyl radical having from 3 to about 6 carbon atoms.

16. Compound of Claim 2 wherein R⁴ represents hydrogen; R³ represents alkyl, alkenyl, hydroxyalkyl, cycloalkyl, cycloalkyl-alkyl, heterocycloalkyl, heterocycloalkylalkyl, aryl, aralkyl or heteroaralkyl radicals; and R⁷ and R^{7'} each independently represent methyl or ethyl radicals; or R⁷ and R^{7'} together with the carbon atom to which they are attached represent cyclopropyl, cyclobutyl, cyclopentyl or cyclohexyl radicals.

17. Compound of Claim 2 wherein R⁴ represents hydrogen; R³ represents alkyl, alkenyl, hydroxyalkyl, cycloalkyl, cycloalkyl-alkyl, heterocycloalkyl, heterocycloalkylalkyl, aryl, aralkyl or heteroaralkyl radicals; and R⁷ and R^{7'} are both methyl.

18. Compound of Claim 2 wherein R⁷ and R^{7'} together with the carbon atom to which they are attached represent cyclopropyl, cyclobutyl, cyclopentyl or cyclohexyl radicals.

19. Compound of Claim 16 wherein R³ represents benzyl, para-fluorobenzyl, para-methoxybenzyl, paramethylbenzyl, or 2-naphthylmethyl radicals; and R⁴ represents t-butyl.

20. Compound of Claim 17 where n is O and R⁸ represents alkylcarbonyl, aryl, aroyl, aralkanoyl, cyano or alkoxycarbonyl.

21. Compounds of Claim 20 where R⁸ represents methylcarbonyl, phenyl or cyano.

22. Compound of Claim 20 where R⁸ represents methoxycarbonyl, ethoxycarbonyl, isopropoxycarbonyl, t-butoxycarbonyl or benzyloxycarbonyl.

23. Compound of Claim 20 where R⁸ represents -COOH.

24. Compound of Claim 17 where n is 1 or 2; and R⁸ represents alkoxycarbonyl, hydroxycarbonyl, arylsulfonyl, alkylsulfonyl, alkylthio, hydroxyl, alkoxy, aryloxy, aryl, heteroaryl or N,N-dialkylcarbamoyl.

25. Compound of Claim 17 where n is 1 or 2; and R⁸ represents N,N-dialkylamino or N-heterocycloamine.

26. Compound of Claim 24 where n is 1 and R⁸ represents methoxycarbonyl or hydroxycarbonyl.

27. Compound of Claim 24 where n is 1 and R⁸ represents methylsulfonyl, methylthio or phenylsulfonyl.

28. Compound of Claim 24 where n is 1 and R⁸ is hydroxy or methoxy.

29. Compound of Claim 24 where n is 1 and R⁸ is phenyl or 4-pyridyl or 4-pyridyl N-oxide.

30. Compound of Claim 24 where n is 1 and R⁸ is N,N-dimethylcarbamoyl.

31. Compound of Claim 25 where R⁸ represents N,N-dimethylamino, 1-piperidinyl, 4-morpholinyl, 4-(N-methyl)piperazinyl, or 1-pyrrolidinyl.

32. Compound of Claim 31 where n is 1 and R⁸ 5 represents 4-morpholinyl.

33. Compound of Claim 31 where n is 2 and R⁸ represents 4-morpholinyl, N,N-dimethylamino or 4-(N-methyl)piperazinyl.

34. Compound of Claim 18 where n is 0 and R⁸ represents methoxycarbonyl, ethoxycarbonyl, isopropoxycarbonyl, t-butoxycarbonyl and methylcarbonyl.

35. Compound of Claim 18 where n is 0 and R⁸ represents methylcarbonyl.

36. Compound of Claim 2 wherein R³ represents heteroaralkyl radicals.

37. Compound of Claim 2 wherein R³ is a p-fluorobenzyl radical.

38. Compound of Claim 2 wherein R³ is a 4-pyridylmethyl radical.

39. Compound of Claim 2 wherein R³ represents an i-amyl radical.

40. Compound of Claim 2 wherein R³ represents an isobutyl radical.

41. Compound of Claim 2 wherein R¹ and R^{1'} are both hydrogen and R^{1"} represents an alkyl radical having from 1 to about 4 carbon atoms.

42. Compound of Claim 2 wherein R¹ and R^{1"} are both hydrogen; and R^{1"} represents -CH₂SO₂NH₂, alkyl or cycloalkyl radicals or amino acid side chains selected from the group consisting of asparagine, S-methyl cystine and the sulfone and sulfoxide derivatives thereof, histidine, norleucine, glutamine, glycine, allo-isoleucine, alanine, threonine, isoleucine, leucine, tert-leucine, phenylalanine, ornithine, allo-threonine and valine side chains.

43. Compound of Claim 1 represented by the formula: 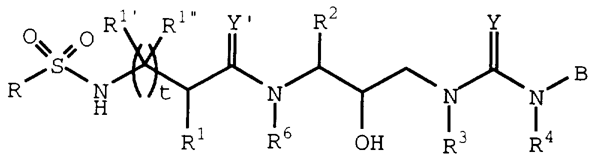 wherein
R represents radicals as defined for R³;
t represents 0 or 1;
R¹ represents -CH₂SO₂NH₂, alkyl or cycloalkyl radicals or amino acid side chains selected from asparagine, S-methyl cysteine, or the corresponding sulfoxide or sulfone derivatives thereof, glycine, allo-isoleucine, alanine, leucine, tert-leucine, phenylalanine, ornithine, threonine, allo-threonine, isoleucine, histidine, norleucine, valine, glutamine, serine, aspartic acid or beta-cyano alanine side chains;
R^{1'} and R^{1"} each independently represent hydrogen or radicals as defined for R¹;
R represents alkyl, aryl, cycloalkyl, cycloalkylalkyl or aralkyl radicals optionally substituted with a group selected from -OR⁹, -SR⁹, or halogen radicals, wherein R⁹ represents hydrogen or alkyl radicals;
R³ represents alkyl, alkenyl, hydroxyalkyl, cycloalkyl, cycloalkylalkyl, heterocycloalkyl, heterocycloalkylalkyl, aryl, heteroaryl, aralkyl, or heteroaralkyl radicals;
R⁴ and R⁵ each independently represent hydrogen or radicals as defined for R³; or R⁴ and R⁵ together with the nitrogen atom to which they are bonded represent heterocycloalkyl or heteroaryl radicals; and
Y and Y' each independently represent O or S.

44. Compound of Claim 43 wherein t is 0.

45. Compound of Claim 43 wherein R¹ represents hydrogen or alkyl radicals.

46. Compound of Claim 43 wherein R¹ represents alkyl radicals having from 1 to about 4 carbon atoms.

47. Compound of Claim 43 wherein R¹ represents methyl, ethyl, isopropyl or t-butyl radicals.

48. Compound of Claim 43 wherein R^{1'} and R^{1"} each independently represent hydrogen or alkyl radicals.

49. Compound of Claim 43 wherein R^{1'} and R^{1"} each independently represent hydrogen or methyl radicals.

50. Compound of Claim 43 wherein R^{1'} is hydrogen and R^{1"} is an alkyl radical.

51. Compound of Claims 2 or 43 wherein R represents alkyl, aryl or aralkyl radicals.

52. Compound of Claims 2 or 43 wherein R is selected from methyl, benzyl or phenethyl radicals.

53. Compound of Claims 2 or 43 wherein R represents alkyl, cycloalkylalkyl or aralkyl radicals, which radicals are optionally substituted with halogen radicals or radicals represented by the formula -OR⁹ or -SR⁹, where R⁹ represents alkyl radicals.

54. Compound of Claims 2 or 43 wherein R represents alkyl, cycloalkylalkyl or aralkyl radicals.

55. Compound of Claims 2 or 43 wherein R represents aralkyl radicals.

56. Compound of Claims 2 or 43 wherein R represents CH₃SCH₂CH₂-, iso-butyl, n-butyl, benzyl, 2-naphthylmethyl or cyclohexylmethyl radicals.

57. Compound of Claims 2 or 43 wherein R represents an n-butyl or iso-butyl radicals.

58. Compound of Claims 2 or 43 wherein R represents benzyl or 2-naphthylmethyl radicals.

59. Compound of Claims 2 or 43 wherein R represents a cyclohexylmethyl radical.

60. Compound of Claims 2 or 43 wherein R³, represents alkyl, alkenyl, hydroxyalkyl, cycloalkyl, cycloalkylalkyl, heterocycloalkyl, heterocycloalkylalkyl, aryl, aralkyl or heteroaralkyl radicals.

61. Compound of Claim 60 wherein R⁴ represents hydrogen.

62. Compound of Claim 60 wherein R³ represents alkyl or alkenyl radicals.

63. Compound cf Claim 61 wherein R³ represents alkyl or hydroxyalkyl radicals.

64. Compound of Claim 61 wherein R³ represents alkyl, cycloalkyl or cycloalkylalkyl radicals.

65. Compound of Claim 61 wherein R³ represents alkyl, heterocycloalkyl or heterocycloalkylalkyl radicals.

66. Compound of Claim 61 wherein R³ represents alkyl, aryl or aralkyl radicals.

67. Compound of Claim 61 wherein R³, R⁴ and R⁵ each independently represent alkyl, cycloalkyl, cycloalkylalkyl, heterocycloalkyl, heterocycloalkylalkyl, aryl, aralkyl, or heteroaralkyl radicals.

68. Compound of Claim 43 wherein R³ represents alkyl radicals having from about 2 to about 5 carbon atoms.

69. Compound of Claim 43 wherein R³ represents i-butyl, neo-pentyl, i-amyl, or n-butyl radicals.

70. Compound of Claim 43 wherein R⁵ represents hydrogen.

71. Compound of Claim 43 wherein R³ represents benzyl, para-fluorobenzyl, para-methoxybenzyl, para-methylbenzyl, or 2-naphthylmethyl radicals.

72. Compound of Claim 43 wherein R⁵ represents hydrogen, alkyl or cycloalkyl radicals.

73. Compound of Claim 43 wherein R⁴ and R⁵ each independently represent ethyl or t-butyl radicals; or R⁴ and R⁵ together with the nitrogen atom to which they are attached represent pyrrolidinyl, piperidinyl, morpholinyl or piperazinyl radicals.

74. Compound of Claim 43 wherein R⁴ and R⁵ are both ethyl radicals.

75. Compound of Claim 43 wherein R⁴ and R⁵ independently represent aikyl, cyclopropyl, cyclobutyl, cyclopentyl or cyclohexyl radicals.

76. A compound of any of Claims 1 to 75 wherein the stereochemistry about the hydroxy group is designated as (R).

77. A pharmaceutical composition comprising a compound of any of claims 1 to 76 and a pharmaceutically acceptable carrier.

78. Compound according to any of claims 1 to 76 for use as an inhibitor of retroviral protease.

79. Compound of claim 78 for use as an inhibitor of retroviral protease which is HIV protease.

80. Compound of any of claims 1 to 76 for use as a medicament.

81. Use of a compound of any of claims 1 to 76 for the manufacture of a medicament for the treatment of a retroviral infection.

82. Use according to claim 81 wherein the retroviral infection is an HIV infection.

83. Use according to claim 81 for the manufacture of a medicament for the treatment of AIDS.

## Patentansprüche

1. Verbindungen der Formel: 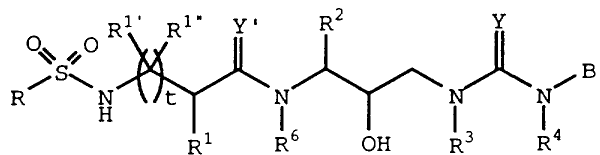 worin R bedeutet: Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste; t Null oder 1 ist; R¹ darstellt: -CH₂SO₂NH₂, Alkyl- oder Cycloalkylreste oder Aminosäure-Seitenketten, ausgewählt aus Asparagin-, S-Methylcystein-Seitenketten, oder den entsprechenden Sulfoxid- oder Sulfon-Derivaten hievon, Glycin-, Alloisoleucin-, Leucin-, tert.Leucin-, Phenylalanin-, Ornithin-, Alanin-, Threonin-, Allothreonin-, Isoleucin-, Histidin-, Norleucin-, Valin-, Glutamin-, Serin-, Aspartamsäure- oder β-Cyanoalanin-Seitenketten; R^{1'} und R^{1"} jeweils unabhängig Wasserstoff oder Reste, wie für R¹ definiert, bedeuten; R darstellt: Alkyl-, Aryl-, Cycloalkyl-, Cycloalkylalkyl- oder Aralkylreste, gegebenenfalls substituiert mit einer Gruppe, ausgewählt aus -OR⁹, -SR⁹, oder Halogenresten, worin R⁹ Wasserstoff oder Alkylreste bedeutet; R³ darstellt: Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste; Y und Y' jeweils unabhängig O oder S darstellen; R⁶ Wasserstoff oder Reste, wie für R³ definiert, bedeutet; B darstellt: R⁵ oder Reste der Formel: 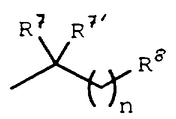 worin n eine ganze Zahl von Null bis 6 ist; R⁷ und R^{7'} jeweils unabhängig bedeuten: Reste, wie für R³ definiert, oder Aminosäure-Seitenketten, ausgewählt aus der Gruppe bestehend aus Valin, Isoleucin, Glycin, Alanin, Alloisoleucin, Asparagin, Leucin, Glutamin und tert.Butylglycin; oder R⁷ und R^{7'} zusammen mit dem Kohlenstoffatom, an das sie gebunden sind, einen Cycloalkylrest bilden; und R⁸ darstellt: Cyano-, Hydroxyl-, Alkyl-, Alkoxy-, Cycloalkyl-, Aryl-, Aralkyl-, Heterocycloalkyl- oder Heteroarylreste oder Reste der Formeln C(O)R¹⁶, CO₂R¹⁶, SO₂R¹⁶, SR¹⁶, CONR¹⁶R¹⁷, OR¹⁶, CF₃ oder NR¹⁶R¹⁷, worin R¹⁶ und R¹⁷ jeweils unabhängig Wasserstoff oder Reste, wie für R³ definiert, bedeuten; oder R¹⁶ und R¹⁷ zusammen mit einem Stickstoff, an den sie gebunden sind, Heterocycloalkyl- oder Heteroarylreste bedeuten; und R⁴ und R⁵ jeweils unabhängig Reste, wie für R³ definiert, darstellen; oder R⁴ und R⁵ zusammen mit dem Stickstoffatom, an das sie gebunden sind, Heterocycloalkyl- oder Heteroarylreste bedeuten; und worin Alkyl, allein oder in Kombination, ein geradkettiger oder verzweigtkettiger Alkylrest mit 1 bis 10 Kohlenstoffatomen ist; Alkenyl einen Propenylrest bedeutet; Cycloalkyl, allein oder in Kombination, einen cyclischen Alkylrest mit 3 bis 8 Kohlenstoffatomen darstellt; Aryl, allein oder in Kombination, bedeutet: einen unsubstituierten Phenylrest, unsubstituierten Naphthylrest oder einen Phenyl- oder Naphthylrest substituiert mit einem oder mehreren Substituenten, ausgewählt aus Alkyl, Alkoxy, Halogen, Hydroxy oder Amino; Aralkyl, allein oder in Kombination, einen Alkylrest, in dem ein Wasserstoffatom durch einen Arylrest ersetzt ist, darstellt; Heterocycloalkyl, allein oder in Kombination, bedeutet: einen monocyclischen, bicyclischen oder tricyclischen, gesättigten oder teilweise ungesättigten Heterocyclusrest, ausgewählt aus der Gruppe umfassend Pyrrolidinyl, Piperidinyl, Piperazinyl, Morpholinyl, Thiamorpholinyl, Tetrahydrochinolyl, 1,2,3,4-Tetrahydro-2-chinolyl, 1,2,3,4-Tetraisochinolyl und 1,2,3,4-Tetrahydro-l-oxoisochinolyl; und Heteroaryl, allein oder in Kombination, darstellt: einen monocyclischen, bicyclischen oder tricyclischen, aromatischen oder teilweise aromatischen Heterocyclusrest, ausgewählt aus Pyrrolyl, Imidazolyl, Pyrazolyl, Pyridyl, Pyrazinyl, Pyrimidinyl, Furyl, Thienyl, Triazolyl, Oxazolyl, Thiazolyl, Indolyl, Chinolyl, Isochinolyl, Chinoxalinyl, β-Carbolinyl, 2-Benzofurancarbonyl und Benzimidazolyl; worin jeder Heterocycloalkyl- oder Heteroarylrest eines oder mehrere Heteroatome enthält, ausgewählt aus der Gruppe bestehend aus Stickstoff, Sauerstoff und Schwefel; und worin jeder Heterocycloalkyl- oder Heteroarylrest gegebenenfalls substituiert ist: an einem sekundären Stickstoffatom mit Alkyl-, Aralkoxycarbonyl-, Alkanoyl-, Phenyl- oder Phenylalkylresten, an einem tertiären Stickstoffatom mit Oxido, und an einem oder mehreren substituierbaren Kohlenstoffatomen mit Halogen-, Alkyl-, Alkoxy- oder Oxoresten.

2. Verbindung nach Anspruch 1 der Formel: 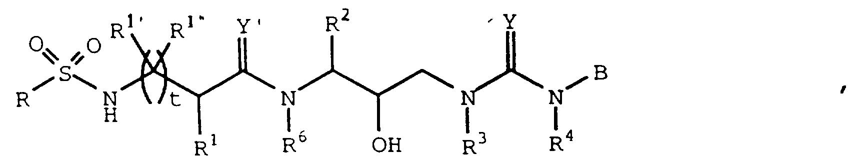 worin R bedeutet: Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste; t Null oder 1 ist; R¹ darstellt: -CH₂SO₂NH₂, Alkyl- oder Cycloalkylreste oder Aminosäure-Seitenketten, ausgewählt aus der Gruppe von Asparagin-, S-Methylcystein-Seitenketten oder den entsprechenden Sulfoxid-(SO-) oder Sulfon- (SO₂-) Derivaten hievon, Histidin-, Norleucin-, Glutamin-, Glycin-, Alloisoleucin-, Alanin-, Threonin-, Isoleucin-, Leucin-, tert.Leucin-, Phenylalanin-, Ornithin-, Allothreonin-, Serin-, Aspartamsäure-, β-Cyanoalanin-oder Valin-Seitenketten; R^{1'} und R^{1"} jeweils unabhängig Wasserstoff oder Reste, wie für R¹ definiert, bedeuten; R darstellt: Alkyl-, Aryl-, Cycloalkyl-, Cycloalkylalkyl- oder Aralkylreste, welche Reste gegebenenfalls substituiert sind mit einer Gruppe, ausgewählt aus Halogenresten oder -OR⁹ oder -SR⁹, worin R⁹ Wasserstoff oder Alkylreste bedeutet; R³ darstellt: Alkyl-, Alkenyl-, Hydroxyalkyl-, Cycloalkyl-, Cycloalkylalkyl-, Heterocycloalkyl-, Heterocycloalkylalkyl-, Aryl-, Aralkyl-, Heteroaryl- oder Heteroaralkylreste; R⁴ Wasserstoff oder Reste, wie für R³ definiert, bedeutet; B darstellt: Reste der Formel: 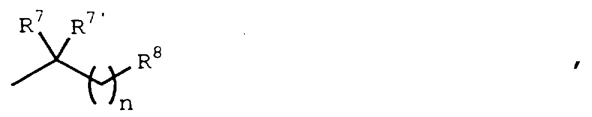 worin n eine ganze Zahl von Null bis 6 ist; R⁷ und R^{7'} jeweils unabhängig bedeuten: Reste, wie für R³ definiert, oder Aminosäure-Seitenketten, ausgewählt aus der Gruppe bestehend aus Valin, Isoleucin, Glycin, Alanin, Alloisoleucin, Asparagin, Leucin, Glutamin und tert.Butylglycin; oder R⁷ und R^{7'} zusammen mit dem Kohlenstoffatom, an das sie gebunden sind, einen Cycloalkylrest bilden; und R⁸ darstellt: Cyano-, Hydroxyl-, Alkyl-, Cycloalkyl-, Aryl-, Aralkyl-, Heterocycloalkyl- oder Heteroarylreste oder Reste der Formeln C(O)R¹⁶, CO₂R¹⁶, SO₂R¹⁶, SR¹⁶, CONR¹⁶R¹⁷, OR¹⁶, CF₃ oder NR¹⁶R¹⁷, worin R¹⁶ und R¹⁷ jeweils unabhängig Wasserstoff oder Reste, wie für R³ definiert, bedeuten; oder R¹⁶ und R¹⁷ zusammen mit einem Stickstoff, an den sie gebunden sind, Heterocycloalkyl- oder Heteroarylreste bedeuten; R⁶ Wasserstoff oder Reste, wie für R³ definiert, darstellt; t Null oder 1 ist; und Y die Bedeutung O oder S hat.

3. Verbindung nach Anspruch 2, worin R¹ Methyl-, Ethyl- oder N-Butylreste bedeutet.

4. Verbindung nach Anspruch 2, worin R¹ darstellt: Alkylreste oder Aminosäure-Seitenketten, ausgewählt aus der Gruppe bestehend aus Asparagin, Valin, Threonin, Allothreonin, Isoleucin, S-Methylcystein und den Sulfon- und Sulfoxid-Derivaten hievon, Alanin und Alloisoleucin.

5. Verbindung nach Anspruch 2, worin R¹ bedeutet: Methyl-, tert.Butyl-, Isopropyl- oder sek.Butylreste, oder Aminosäure-Seitenketten, ausgewählt aus der Gruppe bestehend aus Asparagin-, Valin-, S-Methylcystein-, Alloisoleucin-, Isoleucin-, Threonin- und Allothreonin-Seitenketten.

6. Verbindung nach Anspruch 2, worin R¹ Methyl- oder tert.Butylreste darstellt.

7. Verbindung nach Anspruch 2, worin R¹ einen tert.Butylrest bedeutet.

8. Verbindung nach Anspruch 2, worin R¹ darstellt: Aminosäure-Seitenketten, ausgewählt aus der Gruppe bestehend aus Asparagin-, Valin-, Alanin- und Isoleucin-Seitenketten.

9. Verbindung nach Anspruch 2, worin R¹ bedeutet: Aminosäure-Seitenketten, ausgewählt aus der Gruppe bestehend aus Asparagin, Isoleucin- und Valin-Seitenketten.

10. Verbindung nach Anspruch 2, worin R¹ eine Asparagin-Seitenkette darstellt.

11. Verbindung nach Anspruch 2, worin R¹ ein tert.Butylrest oder eine Asparagin-Seitenkette ist.

12. Verbindung nach Anspruch 2, worin R¹ einen Methylrest bedeutet, wenn t 1 ist.

13. Verbindung nach Anspruch 2, worin t Null ist.

14. Verbindung nach Anspruch 2, worin t 1 ist.

15. Verbindung nach Anspruch 2, worin R⁴ Wasserstoff bedeutet; R³ Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste darstellt; und R⁷ und R^{7'} jeweils unabhängig Alkyl- oder Aralkylreste bedeuten, oder zusammen mit dem Kohlenstoffatom, an das sie gebunden sind, einen Cycloalkylrest mit 3 bis etwa 6 Kohlenstoffatomen bilden.

16. Verbindung nach Anspruch 2, worin R⁴ Wasserstoff bedeutet; R³ Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste darstellt; und R⁷ und R^{7'} jeweils unabhängig Methyl- oder Ethylreste bedeuten, oder R⁷ und R^{7'} zusammen mit dem Kohlenstoffatom, an das sie gebunden sind, Cyclopropyl-, Cyclobutyl-, Cyclopentyl- oder Cyclohexylreste bedeuten.

17. Verbindung nach Anspruch 2, worin R⁴ Wasserstoff bedeutet; R³ Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste darstellt; und R⁷ und R^{7'} beide Methyl sind.

18. Verbindung nach Anspruch 2, worin R⁷ und R^{7'} zusammen mit dem Kohlenstoffatom, an das sie gebunden sind, Cyclopropyl-, Cyclobutyl-, Cyclopentyl- oder Cyclohexylreste bedeuten.

19. Verbindung nach Anspruch 16, worin R³ Benzyl-, p-Fluorbenzyl-, p-Methoxybenzyl-, p-Methylbenzyl- oder 2-Naphthylmethylreste darstellt; und R⁴ tert.Butyl ist.

20. Verbindung nach Anspruch 17, worin n Null ist; und R⁸ Alkylcarbonyl, Aryl, Aroyl, Aralkanoyl, Cyano oder Alkoxycarbonyl bedeutet.

21. Verbindung nach Anspruch 20, worin R⁸ Methylcarbonyl, Phenyl oder Cyano darstellt.

22. Verbindung nach Anspruch 20, worin R⁸ Methoxycarbonyl, Ethoxycarbonyl, Isopropoxycarbonyl, tert.Butoxycarbonyl oder Benzyloxycarbonyl bedeutet.

23. Verbindung nach Anspruch 20, worin R⁸ die Bedeutung -COOH hat.

24. Verbindung nach Anspruch 17, worin n 1 oder 2 ist; und R⁸ Alkoxycarbonyl, Hydroxycarbonyl, Arylsulfonyl, Alkylsulfonyl, Alkylthio, Hydroxyl, Alkoxy, Aryloxy, Aryl, Heteroaryl oder N,N-Dialkylcarbamoyl darstellt.

25. Verbindung nach Anspruch 17, worin n 1 oder 2 ist; und R⁸ N,N-Dialkylamino oder N-Heterocycloamin bedeutet.

26. Verbindung nach Anspruch 24, worin n 1 ist; und R⁸ Methoxycarbonyl oder Hydroxycarbonyl darstellt.

27. Verbindung nach Anspruch 24, worin n 1 ist; und R⁸ Methylsulfonyl, Methylthio oder Phenylsulfonyl bedeutet.

28. Verbindung nach Anspruch 24, worin n 1 ist; und R⁸ Hydroxy oder Methoxy darstellt.

29. Verbindung nach Anspruch 24, worin n 1 ist; und R⁸ Phenyl oder 4-Pyridyl oder 4-Pyridyl-N-oxid bedeutet.

30. Verbindung nach Anspruch 24, worin n 1 ist; und R⁸ N,N-Dimethylcarbamoyl darstellt.

31. Verbindung nach Anspruch 25, worin R⁸ N,N-Dimethylamino, 1-Piperidinyl, 4-Morpholinyl, 4-(N-Methyl)-piperazinyl oder 1-Pyrrolidinyl bedeutet.

32. Verbindung nach Anspruch 31, worin n 1 ist; und R⁸ 4-Morpholinyl darstellt.

33. Verbindung nach Anspruch 31, worin n 2 ist; und R⁸ 4-Morpholinyl, N,N-Dimethylamino oder 4-(N-Methyl)-piperazinyl bedeutet.

34. Verbindung nach Anspruch 18, worin n Null ist; und R⁸ Methoxycarbonyl, Ethoxycarbonyl, Isopropoxycarbonyl, tert.Butoxycarbonyl und Methylcarbonyl darstellt.

35. Verbindung nach Anspruch 18, worin n Null ist; und R⁸ Methylcarbonyl bedeutet.

36. Verbindung nach Anspruch 2, worin R³ Heteroaralkylreste darstellt.

37. Verbindung nach Anspruch 2, worin R³ ein p-Fluorbenzylrest ist.

38. Verbindung nach Anspruch 2, worin R³ ein 4-Pyridylmethylrest ist.

39. Verbindung nach Anspruch 2, worin R³ ein Isoamylrest ist.

40. Verbindung nach Anspruch 2, worin R³ ein Isobutylrest ist.

41. Verbindung nach Anspruch 2, worin R¹ und R^{1'} beide Wasserstoff bedeuten; und R^{1"} einen Alkylrest mit 1 bis etwa 4 Kohlenstoffatomen bedeutet.

42. Verbindung nach Anspruch 2, worin R¹ und R^{1'} beide Wasserstoff bedeuten; und R^{1"} darstellt: -CH₂SO₂NH₂, Alkyl- oder Cycloalkylreste oder Aminosäure-Seitenketten, ausgewählt aus der Gruppe bestehend aus Asparagin-, S-Methylcystein-Seitenketten und den Sulfon- und Sulfoxid-Derivaten hievon, Histidin-, Norleucin-, Glutamin-, Glycin-, Alloisoleucin-, Alanin-, Threonin-, Isoleucin-, Leucin-, tert.Leucin-, Phenylalanin-, Ornithin-, Allothreonin- und Valin-Seitenketten.

43. Verbindung nach Anspruch 1 der Formel: 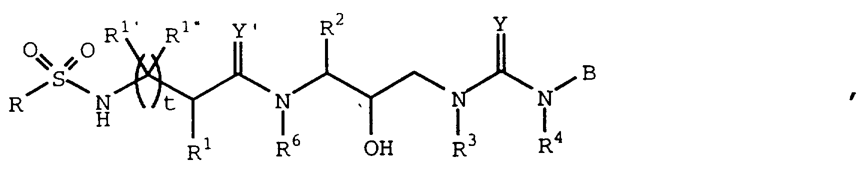 worin R Reste, wie für R³ definiert, bedeutet; t Null oder 1 ist; R¹ darstellt: -CH₂SO₂NH₂, Alkyl- oder Cycloalkylreste oder Aminosäure-Seitenketten, ausgewählt aus Asparagin-, S-Methylcystein-Seitenketten, oder den entsprechenden Sulfoxid- oder Sulfon-Derivaten hievon, Glycin-, Alloisoleucin-, Alanin-, Leucin-, tert.Leucin-, Phenylalanin-, Ornithin-, Threonin-, Allothreonin-, Isoleucin-, Histidin-, Norleucin-, Valin-, Glutamin-, Serin-, Aspartamsäure- oder β-Cyanoalanin-Seitenketten; R^{1'} und R^{1"} jeweils unabhängig Wasserstoff oder Reste, wie für R¹ definiert, bedeuten; R darstellt: Alkyl-, Aryl-, Cycloalkyl-, Cycloalkylalkyl- oder Aralkylreste, gegebenenfalls substituiert mit einer Gruppe, ausgewählt aus -OR⁹, -SR⁹, oder Halogenresten, worin R⁹ Wasserstoff oder Alkylreste bedeutet; R³ darstellt: Alkyl-, Alkenyl-, Hydroxyalkyl-, Cycloalkyl-, Cycloalkylalkyl-, Heterocycloalkyl-, Heterocycloalkylalkyl-, Aryl-, Heteroaryl-, Aralkyl- oder Heteroaralkylreste; R⁴ und R⁵ jeweils unabhängig Wasserstoff oder Reste, wie für R³ definiert, darstellen; oder R⁴ und R⁵ zusammen mit dem Stickstoffatom, an das sie gebunden sind, Heterocycloalkyl- oder Heteroarylreste bedeuten; und Y und Y' jeweils unabhängig O oder S sind.

44. Verbindung nach Anspruch 43, worin t Null ist.

45. Verbindung nach Anspruch 43, worin R¹ Wasserstoff oder Alkylreste darstellt.

46. Verbindung nach Anspruch 43, worin R¹ Alkylreste mit 1 bis etwa 4 Kohlenstoffatomen bedeutet.

47. Verbindung nach Anspruch 43, worin R¹ Methyl-, Ethyl-, Isopropyl- oder tert.Butylreste darstellt.

48. Verbindung nach Anspruch 43, worin R^{1'} und R^{1"} jeweils unabhängig Wasserstoff oder Alkylreste bedeuten.

49. Verbindung nach Anspruch 43, worin R^{1'} und R^{1"} jeweils unabhängig Wasserstoff oder Methylreste darstellen.

50. Verbindung nach Anspruch 43, worin R^{1'} Wasserstoff bedeutet; und R^{1"} ein Alkylrest ist.

51. Verbindung nach Anspruch 2 oder 43, worin R Alkyl-, Aryl-oder Aralkylreste darstellt.

52. Verbindung nach Anspruch 2 oder 43, worin R ausgewählt ist aus Methyl-, Benzyl- oder Phenethylresten.

53. Verbindung nach Anspruch 2 oder 43, worin R Alkyl-, Cycloalkylalkyl- oder Aralkylreste bedeutet, welche Reste gegebenenfalls substituiert sind mit Halogenresten oder Resten der Formel -OR⁹ oder -SR⁹, worin R⁹ Alkylreste darstellt.

54. Verbindung nach Anspruch 2 oder 43, worin R Alkyl-, Cycloalkylalkyl- oder Aralkylreste bedeutet.

55. Verbindung nach Anspruch 2 oder 43, worin R Aralkylreste darstellt.

56. Verbindung nach Anspruch 2 oder 43, worin R CH₃SCH₂CH₂-, Isobutyl-, n-Butyl-, Benzyl-, 2-Naphthylmethyl- oder Cyclohexylmethylreste bedeutet.

57. Verbindung nach Anspruch 2 oder 43, worin R n-Butyl- oder Isobutylreste darstellt.

58. Verbindung nach Anspruch 2 oder 43, worin R Benzyl- oder 2-Naphthylmethylreste bedeutet.

59. Verbindung nach Anspruch 2 oder 43, worin R einen Cyclohexylmethylrest darstellt.

60. Verbindung nach Anspruch 2 oder 43, worin R³ Alkyl-, Alkenyl-, Hydroxyalkyl-, Cycloalkyl-, Cycloalkylalkyl-, Heterocycloalkyl-, Heterocycloalkylalkyl-, Aryl-, Aralkyl- oder Heteroaralkylreste bedeutet.

61. Verbindung nach Anspruch 60, worin R⁴ Wasserstoff ist.

62. Verbindung nach Anspruch 60, worin R³ Alkyl- oder Alkenylreste darstellt.

63. Verbindung nach Anspruch 61, worin R³ Alkyl- oder Hydroxyalkylreste darstellt.

64. Verbindung nach Anspruch 61, worin R³ Alkyl-, Cycloalkyl-oder Cycloalkylalkylreste bedeutet.

65. Verbindung nach Anspruch 61, worin R³ Alkyl-, Heterocycloalkyl- oder Heterocycloalkylalkylreste darstellt.

66. Verbindung nach Anspruch 61, worin R³ Alkyl-, Aryl- oder Aralkylreste bedeutet.

67. Verbindung nach Anspruch 61, worin R³, R⁴ und R⁵ jeweils unabhängig Alkyl-, Cycloalkyl-, Cycloalkylalkyl-, Heterocycloalkyl-, Heterocycloalkylalkyl-, Aryl-, Aralkyl- oder Heteroaralkylreste darstellen.

68. Verbindung nach Anspruch 43, worin R³ Alkylreste mit etwa 2 bis etwa 5 Kohlenstoffatomen bedeutet.

69. Verbindung nach Anspruch 43, worin R³ Isobutyl-, Neopentyl-, Isoamyl- oder n-Butylreste darstellt.

70. Verbindung nach Anspruch 43, worin R⁵ Wasserstoff ist.

71. Verbindung nach Anspruch 43, worin R³ Benzyl-, p-Fluorbenzyl-, p-Methoxybenzyl-, p-Methylbenzyl- oder 2-Naphthylmethylreste bedeutet.

72. Verbindung nach Anspruch 43, worin R⁵ Wasserstoff, Alkyl-oder Cycloalkylreste darstellt.

73. Verbindung nach Anspruch 43, worin R⁴ und R⁵ jeweils unabhängig Ethyl- oder tert.Butylreste sind; oder R⁴ und R⁵ zusammen mit dem Stickstoffatom, an das sie gebunden sind, Pyrrolidinyl-, Piperidinyl-, Morpholinyl- oder Piperazinylreste bedeuten.

74. Verbindung nach Anspruch 43, worin R⁴ und R⁵ beide Ethylreste darstellen.

75. Verbindung nach Anspruch 43, worin R⁴ und R⁵ unabhängig Alkyl-, Cyclopropyl-, Cyclobutyl-, Cyclopentyl- oder Cyclohexylreste bedeuten.

76. Verbindung nach einem der Ansprüche 1 bis 75, worin die Stereochemie in bezug auf die Hydroxy-Gruppe als (R) bezeichnet ist.

77. Pharmazeutische Zusammensetzung, welche eine Verbindung nach einem der Ansprüche 1 bis 76 und einen pharmazeutisch annehmbaren Träger umfaßt.

78. Verbindung nach einem der Ansprüche 1 bis 76 zur Verwendung als Inhibitor von retroviraler Protease.

79. Verbindung nach Anspruch 78 zur Verwendung als Inhibitor von retroviraler Protease, die HIV-Protease ist.

80. Verbindung nach einem der Ansprüche 1 bis 76 zur Verwendung als Medikament.

81. Verwendung einer Verbindung nach einem der Ansprüche 1 bis 76 bei der Herstellung eines Medikaments zur Behandlung einer Retrovirusinfektion.

82. Verwendung nach Anspruch 81, wobei die Retrovirusinfektion eine HIV-Infektion ist.

83. Verwendung nach Anspruch 81 bei der Herstellung eines Medikaments zur Behandlung von AIDS.

## Revendications

1. Composé représenté par la formule : 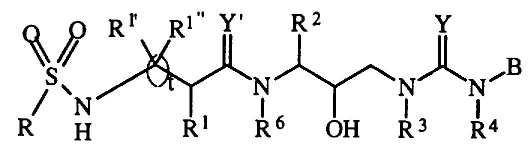 dans laquelle
R représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ;
t vaut 0 ou 1;
R¹ représente un radical -CH₂SO₂NH₂, alkyle ou cycloalkyle ou une chaîne latérale d'acide aminé choisi parmi l'asparagine, la S-méthyl cystéine, ou l'un de ses dérivés de type sulfone ou sulfoxyde, la glycine, l'allo-isoleucine, la leucine, la tert-leucine, la phénylalanine, l'ornithine, l'alanine, la thréonine, l'allo-thréonine, l'isoleucine, l'histidine, la norleucine, la valine, la glutamine, la sérine, l'acide aspartique et la béta-cyano alanine ;
R^{1'} et R^{1"} représentent, indépendamment l'un de l'autre, l'hydrogène ou un radical tel que défini pour R¹ ;
R représente un radical alkyle, aryle, cycloalkyle, cycloalkylalkyle ou aralkyle éventuellement substitué par un halogène ou par un groupe choisi parmi -OR⁹ et -SR⁹, dans lesquels R⁹ représente l'hydrogène ou un radical alkyle ;
R³ représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ;
Y et Y' représentent, indépendamment l'un de l'autre, O ou S ;
R⁶ représente l'hydrogène ou un radical tel que défini pour R³ ;
B représente R⁵ ou un radical représenté par la formule : 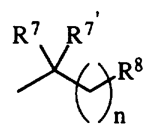 dans laquelle
n est un entier de 0 à 6 ; R⁷ et R^{7'}, indépendamment l'un de l'autre, représentent un radical tel que défini pour R³ ou une chaîne latérale d'acide aminé choisi parmi la valine, l'isoleucine, la glycine, l'alanine, l'allo-isoleucine, l'asparagine, la leucine, la glutamine et la t-butylglycine ; ou R⁷ et R^{7'} forment avec l'atome de carbone auxquels ils sont liés un radical cycloalkyle ; et R⁸ représente un radical cyano, hydroxy, alkyle, alcoxy, cycloalkyle, aryle, aralkyle, hétérocycloalkyle ou hétéroaryle, ou un radical représenté par la formule C(O)R¹⁶, CO₂R¹⁶, SO₂R¹⁶, SR¹⁶, CONR¹⁶R¹⁷, OR¹⁶, CF₃ ou NR¹⁶R¹⁷, dans lesquelles R¹⁶ et R¹⁷, indépendamment l'un de l'autre, représentent l'hydrogène ou un radical tel que défini pour R³ ; ou R¹⁶ et R¹⁷ forment avec l'atome d'azote auquel ils sont liés un radical hétérocycloalkyle ou hétéroaryle ; et
R⁴ et R⁵, indépendamment l'un de l'autre, représentent des radicaux tels que définis pour R³ ; ou R⁴ et R⁵ forment avec l'atome d'azote auxquels ils sont liés un radical hétérocycloalkyle ou hétéroaryle ; et
dans laquelle le radical alkyle, seul ou combiné, est un radical alkyle linéaire ou ramifié contenant 1 à 10 atomes de carbone ; le radical alcényle est le radical propényle ; le radical cycloalkyle, seul ou en combinaison, est un radical alkyle cyclique ayant 3 à 8 atomes de carbone ; le radical aryle, seul ou en combinaison, est un radical phényle non substitué, un radical naphtyle non substitué ou un radical phényle ou naphtyle substitué par un ou plusieurs substituants choisis parmi les radicaux alkyle, alcoxy, halogène, hydroxy et amino ; le radical aralkyle, seul ou en combinaison, est un radical alkyle dans lequel l'un des atomes d'hydrogène est remplacé par un radical aryle ; le radical hétérocycloalkyle, seul ou en combinaison, est un hétérocycle monocyclique, bicyclique ou tricyclique, saturé ou partiellement insaturé, choisi parmi les radicaux pyrrolidinyle, pipéridinyle, pipérazinyle, morpholinyle, thiamorpholinyle, tétrahydroquinolyle, 1,2,3,4-tétrahydro-2-quinolyle, 1,2,3,4-tétraisoquinolyle et 1,2,3,4-tétrahydroxy-1-oxoisoquinolyle; et le radical hétéroaryle, seul ou en combinaison, est un hétérocycle monocyclique, bicyclique ou tricyclique, aromatique ou partiellement aromatique, choisi parmi les radicaux pyrrolyle, imidazolyle, pyrazolyle, pyridyle, pyrazinyle, pyrimidinyle, furyle, thiényle, triazolyle, oxazolyle, thiazolyle, indolyle, quinolyle, isoquinolyle, quinoxalinyle, béta-carbolinyle, 2-benzofuranecarbonyle et benzimidazolyle ; dans lesquels chaque radical hétérocycloalkyle ou hétéroaryle contient un ou plusieurs hétéroatomes choisis parmi l'hydrogène, l'oxygène et le soufre ; et dans lesquels chacun des radicaux hétérocycloalkyle ou hétéroaryle est éventuellement substitué sur un atome d'azote secondaire par un radical alkyle, aralcoxycarbonyle, alcanoyle, phényle ou phénylalkyle, sur un atome d'azote tertiaire par un atome d'oxygène et sur un ou plusieurs atomes de carbone substituables par des radicaux halogène, alkyle, alcoxy ou oxo.

2. Composé selon la revendication 1 représenté par la formule : 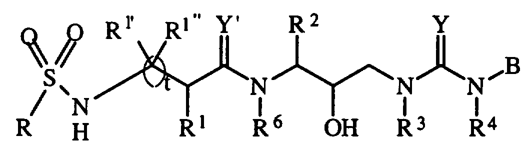 dans laquelle
R représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ;
t vaut 0 ou 1 ;
R¹ représente un radical -CH₂SO₂NH₂, alkyle ou cycloalkyle, ou une chaîne latérale d'acide aminé choisi parmi l'asparagine, la S-méthyl cystéine, ou l'un de ses dérivés de type sulfoxyde (SO) ou sulfone (SO₂), l'histidine, la norleucine, la glutamine, la glycine, l'allo-isoleucine, l'alanine, la thréonine, l'isoleucine, la leucine, la tert-leucine, la phénylalanine, l'ornithine, l'allo-thréonine, la sérine, l'acide aspartique, la béta-cyano alanine et la valine ;
R^{1'} et R^{1"} représentent, indépendamment l'un de l'autre, l'hydrogène ou un radical tel que défini pour R¹ ;
R représente un radical alkyle, aryle, cycloalkyle, cycloalkylalkyle ou aralkyle, lesquels radicaux sont éventuellement substitués par un radical choisi parmi les radicaux halogène ou -OR⁹ ou-SR⁹, dans lesquels R⁹ représente l'hydrogène ou un radical alkyle ;
R³ représente un radical alkyle, alcényle, hydroxyalkyle, cycloalkyle, cycloalkylalkyle, hétérocycloalkyle, hétérocycloalkylalkyle, aryle, aralkyle, hétéroaryle ou hétéroaralkyle ;
R⁴ représente l'hydrogène ou un radical tel que défini pour R³ ;
B représente un radical correspondant à la formule : 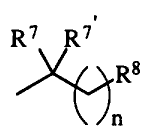 dans laquelle
n représente un entier de 0 à 6 ; R⁷ et R^{7'}, indépendamment l'un de l'autre, représentent un radical tel que défini pour R³ ou une chaîne latérale d'acide aminé choisi parmi la valine, l'isoleucine, la glycine, l'alanine, l'allo-isoleucine, l'asparagine, la leucine, la glutamine et la t-butylglycine ; ou R⁷ et R^{7'} forment avec l'atome de carbone auxquels ils sont attachés un radical cycloalkyle ; et R⁸ représente un radical cyano, hydroxy, alkyle, cycloalkyle, aryle, aralkyle, hétérocycloalkyle ou hétéroaryle, ou un radical représenté par l'une des formules C(O)R¹⁶, CO₂R¹⁶, SO₂R¹⁶, SR¹⁶, CONR¹⁶R¹⁷, OR¹⁶, CF₃ ou NR¹⁶R¹⁷, dans lesquels R¹⁶ et R¹⁷, indépendamment l'un de l'autre, représentent l'hydrogène ou un radical tel que défini pour R³ ; ou R¹⁶ et R¹⁷ forment avec l'atome d'azote auxquels ils sont attachés un radical hétérocycloalkyle ou hétéroaryle ;
R⁶ représente l'hydrogène ou un radical tel que défini pour R³ ; et
Y représente O ou S.

3. Composé selon la revendication 2, caractérisé en ce que R¹ représente un radical méthyle, éthyle ou N-butyle.

4. Composé selon la revendication 2, caractérisé en ce que R¹ représente un radical méthyle ou une chaîne latérale d'acide aminé choisi parmi l'asparagine, la valine, la thréonine, l'allo-thréonine, l'isoleucine, la S-méthyl cystéine ou l'un de ses dérivés de type sulfone ou sulfoxide, l'alanine ou l'allo-isoleucine.

5. Composé selon la revendication 2, caractérisé en ce que R¹ représente un radical méthyle, t-butyle, isopropyle ou sec-butyle, ou une chaîne latérale d'acide aminé choisi parmi l'asparagine, la valine, la S-méthyle cystéine, l'allo-isoleucine, l'isoleucine, la thréonine ou l'allo-thréonine.

6. Composé selon la revendication 2, caractérisé en ce que R¹ représente un radical méthyle ou t-butyle.

7. Composé selon la revendication 2, caractérisé en ce que R¹ représente un radical t-butyle.

8. Composé selon la revendication 2, caractérisé en ce que R¹ représente une chaîne latérale d'acide aminé choisi parmi l'asparagine, la valine, l'alanine ou l'isoleucine.

9. Composé selon la revendication 2, caractérisé en ce que R¹ représente une chaîne latérale d'acide aminé choisi parmi l'asparagine, l'isoleucine ou la valine.

10. Composé selon la revendication 2, caractérisé en ce que R¹ représente une chaîne latérale de l'asparagine.

11. Composé selon la revendication 2, caractérisé en ce que R¹ représente un radical t-butyle ou une chaîne latérale de l'asparagine.

12. Composé selon la revendication 2, caractérisé en ce que R¹ représente un radical méthyle lorsque t vaut 1.

13. Composé selon la revendication 2, caractérisé en ce t vaut 0.

14. Composé selon la revendication 2, caractérisé en ce que t vaut 1.

15. Composé selon la revendication 2, caractérisé en ce que R⁴ représente l'hydrogène ; R³ représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ; et R⁷ et R^{7'}, indépendamment l'un de l'autre, représentent un radical alkyle ou aralkyle, ou forment avec l'atome de carbone auquel ils sont attachés un radical cycloalkyle ayant 5 à 6 atomes de carbone.

16. Composé selon la revendication 2, caractérisé en ce que R⁴ représente l'hydrogène ; R³ représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ; et R⁷ et R^{7'}, indépendamment l'un de l'autre, représentent un radical méthyle ou éthyle ; ou R⁷ et R^{7'} forment avec l'atome de carbone auquel ils sont attachés un radical cyclopropyle, cyclobutyle, cyclopentyle ou cyclohexyle.

17. Composé selon la revendication 2, caractérisé en ce que R⁴ représente l'hydrogène ; R³ représente un radical alkyle, alcényle, hydroxyalkyle, cycloalkyle, cycloalkylalkyle, hétérocycloalkyle, hétérocycloalkylalkyle, aryle, aralkyle ou hétéroaralkyle ; et R⁷ et R^{7'} sont tous deux méthyle.

18. Composé selon la revendication 2, caractérisé en ce que R⁷ et R^{7'} forment avec l'atome de carbone auquel ils sont attachés un radical cyclopropyle, cyclobutyle, cyclopentyle ou cyclohexyle.

19. Composé selon la revendication 16, caractérisé en ce que R³ représente un radical benzyle, para-fluorobenzyle, para-méthoxybenzyle, para-méthylbenzyle, ou 2-naphtylméthyle ; et R⁴ représente t-butyle.

20. Composé selon la revendication 17, caractérisé en ce que n vaut 0 et R⁸ représente un radical alkylcarbonyle, aryle, aroyle, aralcanoyle, cyano ou alcoxycarbonyle.

21. Composé selon la revendication 20, caractérisé en ce que R⁸ représente un radical méthylcarbonyle, phényle ou cyano.

22. Composé selon la revendication 20, caractérisé en ce que R⁸ représente un radical méthoxycarbonyle, éthoxycarbonyle, isopropoxycarbonyle, t-butoxycarbonyle ou benzyloxycarbonyle.

23. Composé selon la revendication 20, caractérisé en ce que R⁸ représente -COOH.

24. Composé selon la revendication 17, caractérisé en ce que n vaut 1 ou 2 ; et R⁸ représente un radical alcoxycarbonyle, hydroxycarbonyle, arylsulfonyle, alkylsulfonyle, alkylthio, hydroxy, alcoxy, aryloxy, aryle, hétéroaryle ou N,N-dialkylcarbamoyle.

25. Composé selon la revendication 17, caractérisé en ce que n vaut 1 ou 2 ; et R⁸ représente un radical N,N-dialkylamino ou N-hétérocycloamine.

26. Composé selon la revendication 24, caractérisé en ce que n vaut 1 et R⁸ représente un radical méthoxycarbonyle ou hydroxycarbonyle.

27. Composé selon la revendication 24, caractérisé en ce que n vaut 1 et R⁸ représente un radical méthylsulfonyle, méthylthio ou phénylsulfonyle.

28. Composé selon la revendication 24, caractérisé en ce que n vaut 1 et R⁸ est hydroxy ou méthoxy.

29. Composé selon la revendication 24, caractérisé en ce que n vaut 1 et R⁸ représente un radical phényle, 4-pyridyle ou 4-pyridyl N-oxyde.

30. Composé selon la revendication 24, caractérisé en ce que n vaut 1 et R⁸ représente le radical N,N-diméthylcarbamoyle.

31. Composé selon la revendication 25, caractérisé en ce que R⁸ représente un radical N,N-diméthylamino, 1-pipérydinyle, 4-morpholinyle, 4-(N-méthyl)piperazinyle ou 1-pyrrolidinyle.

32. Composé selon la revendication 31, caractérisé en ce que n vaut 1 et R⁸ représente un radical 4-morpholinyle.

33. Composé selon la revendication 31, caractérisé en ce que n vaut 2 et R⁸ représente un radical 4-morpholinyle, N,N-diméthylamino ou 4-(N-méthyl)pipérazinyle.

34. Composé selon la revendication 18, caractérisé en ce que n vaut 0 et R⁸ représente un radical méthoxycarbonyle, éthoxycarbonyle, isopropoxycarbonyle, t-butoxycarbonyle ou méthylcarbonyle.

35. Composé selon la revendication 18, caractérisé en ce que n vaut 0 et R⁸ représente le radical méthylcarbonyle.

36. Composé selon la revendication 2, caractérisé en ce que R³ représente un radical hétéroaralkyle.

37. Composé selon la revendication 2, caractérisé en ce que R³ représente un radical p-fluorobenzyle.

38. Composé selon la revendication 2, caractérisé en ce que R³ représente le radical 4-pyridylméthyle.

39. Composé selon la revendication 2, caractérisé en ce que R³ représente un i-amyle radical.

40. Composé selon la revendication 2, caractérisé en ce que R³ représente un radical iso-butyle.

41. Composé selon la revendication 2, caractérisé en ce que R¹ et R^{1'} représentent tous deux l'hydrogène et R^{1"} représente un radical alkyle ayant 1 à 4 atomes de carbone.

42. Composé selon la revendication 2, caractérisé en ce que R1 et R^{1'} représentent tous deux l'hydrogène et R^{1"} représente un radical -CH₂SO₂NH₂, alkyle ou cycloalkyle ou une chaîne latérale d'acide aminé choisi parmi l'asparagine, la S-méthyl cystéine ou l'un de ses dérivés de type sulfone ou sulfoxyde, l'histidine, la norleucine, la glutamine, la glycine, l'allo-isoleucine, l'alanine, la thréonine, l'isoleucine, la leucine, la tert-leucine, la phénylalanine, l'ornithine, l'allo-thréonine et la valine.

43. Composé selon la revendication 1 représenté par la formule : 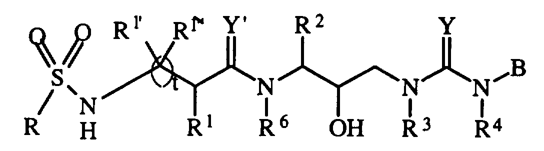 dans laquelle
R représente un radical tel que défini pour R³ ;
t vaut 0 ou 1 ;
R¹ représente un radical -CH₂SO₂NH₂, alkyle ou cycloalkyle ou une chaîne latérale d'acide aminé choisi parmi l'asparagine, la S-méthyl cystéine, ou l'un de ses dérivés de type sulfoxyde ou sulfone, la glycine, l'allo-isoleucine, l'alanine, la leucine, la tert-leucine, la phénylalanine, l'ornithine, la thréonine, l'allo-thréonine, l'isoleucine, l'histidine, la norleucine, la valine, la glutamine, la sérine, l'acide aspartique ou la béta-cyano alanine ;
R¹ et R^{1"}, indépendamment l'un de l'autre, représentent l'hydrogène ou l'un des radicaux définis pour R¹ ;
R représente un radical alkyle, aryle, cycloalkyle, cycloalkylalkyle ou aralkyle éventuellement substitué par un halogène ou par un groupement choisi parmi -OR⁹ et -SR⁹, dans lesquels R⁹ représente l'hydrogène ou un radical alkyle ;
R³ représente un radical alkyle, alcényle, hydroxyalkyle, cycloalkyle, cycloalkylalkyle, hétérocycloalkyle, hétérocycloalkylalkyle, aryle, hétéroaryle, aralkyle ou hétéroaralkyle ;
R⁴ et R⁵, indépendamment l'un de l'autre, représentent l'hydrogène ou un radical tel que défini pour R³ ; ou R⁴ et R⁵ forment ensemble avec l'atome d'azote auquel ils sont rattachés un radical hétérocycloalkyle ou hétéroaryle ; et
Y et Y', indépendamment l'un de l'autre, représentent O ou S.

44. Composé selon la revendication 43, caractérisé en ce que t vaut 0.

45. Composé selon la revendication 43, caractérisé en ce que R¹ représente l'hydrogène ou un radical alkyle.

46. Composé selon la revendication 43, caractérisé en ce que R¹ représente un radical alkyle ayant 1 à 4 atomes de carbone.

47. Composé selon la revendication 43, caractérisé en ce que R¹ représente un radical méthyle, éthyle, isopropyle ou t-butyle.

48. Composé selon la revendication 43, caractérisé en ce que R^{1'} et R^{1"}, indépendamment l'un de l'autre, représentent l'hydrogène ou un radical alkyle.

49. Composé selon la revendication 43, caractérisé en ce que R^{1'} et R^{1"}, indépendamment l'un de l'autre, représentent l'hydrogène ou un radical méthyle.

50. Composé selon la revendication 43, caractérisé en ce que R^{1'} est l'hydrogène et R^{1"} est un radical alkyle.

51. Composé selon la revendication 2 ou 43, caractérisé en ce que R représente un radical alkyle, aryle ou aralkyle.

52. Composé selon la revendication 2 ou 43, caractérisé en ce que R représente un radical méthyle, benzyle ou phénéthyle.

53. Composé selon la revendication 2 ou 43, caractérisé en ce que R représente un radical alkyle, cycloalkylalkyle ou aralkyle, éventuellement substitué par un halogène ou un radical représenté par la formule -OR⁹ ou -SR⁹, dans laquelle R⁹ représente un radical alkyle.

54. Composé selon la revendication 2 ou 43, caractérisé en ce que R représente un radical alkyle, cycloalkyle ou aralkyle.

55. Composé selon la revendication 2 ou 43, caractérisé en ce que R représente un radical aralkyle.

56. Composé selon la revendication 2 ou 43, caractérisé en ce que R représente un radical CH₃SCH₂CH₂-, iso-butyle, n-butyle, benzyle, 2-naphtylméthyle ou cyclohexylméthyle.

57. Composé selon la revendication 2 ou 43, caractérisé en ce que R représente un radical n-butyle ou iso-butyle.

58. Composé selon la revendication 2 ou 43, caractérisé en ce que R représente un radical benzyle ou 2-naphtylméthyle.

59. Composé selon la revendication 2 ou 43, caractérisé en ce que R représente un radical cyclohexylméthyle.

60. Composé selon la revendication 2 ou 43, caractérisé en ce que R3 représente un radical alkyle, alcényle, hydroxyalkyle, cycloalkyle, cycloalkylalkyle, hétérocycloalkyle, hétérocycloalkylalkyle, aryle, aralkyle ou hétéroaralkyle.

61. Composé selon la revendication 60, caractérisé en ce que R⁴ représente l'hydrogène.

62. Composé selon la revendication 60, caractérisé en ce que R³ représente un radical alkyle ou alcényle.

63. Composé selon la revendication 61, caractérisé en ce que R³ représente un radical alkyle ou hydroxyalkyle.

64. Composé selon la revendication 61, caractérisé en ce que R³ représente un radical alkyle, cycloalkyle ou cycloalkylalkyle.

65. Composé selon la revendication 61, caractérisé en ce que R³ représente un radical alkyle, hétérocycloalkyle ou hétérocycloalkylalkyle.

66. Composé selon la revendication 61, caractérisé en ce que R³ représente un radical alkyle, aryle ou aralkyle.

67. Composé selon la revendication 61, caractérisé en ce que R³, R⁴ et R⁵, indépendamment les uns des autres, représentent un radical alkyle, cycloalkyle, cycloalkylalkyle, hétérocycloalkyle, hétérocycloalkylalkyle, aryle, aralkyle ou hétéroaralkyle.

68. Composé selon la revendication 43, caractérisé en ce que R³ représente un radical alkyle ayant environ 2 à environ 5 atomes de carbone.

69. Composé selon la revendication 43, caractérisé en ce que R³ représente i-butyle, néo-pentyle, i-amyle ou n-butyle.

70. Composé selon la revendication 43, caractérisé en ce que R⁵ représente l'hydrogène.

71. Composé selon la revendication 43, caractérisé en ce que R³ représente un radical benzyle, parafluorobenzyle, paraméthoxybenzyle, para-méthylbenzyle ou 2-napthylméthyle.

72. Composé selon la revendication 43, caractérisé en ce que R⁵ représente 1' hydrogène ou un radical alkyle ou cycloalkyle.

73. Composé selon la revendication 43, caractérisé en ce que R⁴ et R⁵, indépendamment l'un de l'autre, représente un radical éthyle ou t-butyle ; ou R⁴ et R⁵ forment ensemble avec l'atome d'azote auquel ils sont attachés un radical pyrrolidinyle, pipéridinyle, morpholinyl ou pipérazinyle.

74. Composé selon la revendication 43, caractérisé en ce que R⁴ et R⁵ représentent tous deux un radical éthyle.

75. Composé selon la revendication 43, caractérisé en ce que R⁴ et R5, indépendamment l'un de l'autre, représentent un radical alkyle, cyclopropyle, cyclobutyle, cyclopentyle ou cyclohexyle.

76. Composé selon l'une quelconque des revendications 1 à 75, caractérisé en ce que la stéréochimie concernant le groupement hydroxy est désigné par (R).

77. Composition pharmaceutique comprenant un composé tel que défini dans l'une quelconque des revendications 1 à 76 et un support pharmaceutiquement acceptable.

78. Composé selon l'une quelconque des revendications 1 à 76, pour son utilisation à titre d'inhibiteur de protéase rétrovirale.

79. Composé selon la revendication 78, pour son utilisation en tant qu'inhibiteur d'une protéase rétrovirale qui est la protéase de VIH.

80. Composé selon l'une quelconque des revendications 1 à 76, pour son utilisation à titre de médicament.

81. Utilisation d'un composé tel que défini dans l'une quelconque des revendications 1 à 76 pour la fabrication d'un médicament destiné au traitement d'infections rétrovirales.

82. Utilisation selon la revendication 81, caractérisée en ce que l'infection rétrovirale est l'infection par VIH.

83. Utilisation selon la revendication 81, pour la préparation d'un médicament destiné au traitement du SIDA.
